# FASCICULE DE BREVET EUROPEEN

(11) **EP 0 659 747 B1**
(45) Date de publication et mention de la délivrance du brevet: **01.04.1998**
(21) Numéro de dépôt: 94402954.5
(22) Date de dépôt: 20.12.1994
(51) Int. Cl.: C07D 277/42, C07D 417/04, C07D 417/12, A61K 31/425

(54) **Dérivés amino ramifiés du thiazole, leurs procédés de préparation et les compositions pharmaceutiques qui les contiennent**
Verzweigte Aminothiazole-Derivate, Verfahren zu ihrer Herstellung und pharmazeutische Zusammenstellungen die sie enthalten
Branched aminothiazole derivatives process for their preparation and pharmaceutical compositions containing them

(30) Priorité: 21.12.1993 FR 9315386
(43) Date de publication de la demande: 28.06.1995
(73) Titulaire: SANOFI, 75008 Paris (FR)
(72) Inventeur: Roger, Pierre - Marie - Albert, F-78423 Montigny le Bretonneux (FR); Gully, Danièle - Anne - Jeanne, F-31600 Muret (FR); Courtemanche, Gilles - Victor - Carlos, F-95270 ST. Martin Du Tertre (FR); Gautier, Claudie - Suzanne - Yvette, F-75013 Paris (FR); Geslin, Michel - Jacques - Henri, F-31270 Villeneuve-Tolosane (FR); Wermuth, Camille-Georges, F-67100 Strasbourg (FR)
(74) Mandataire: Le Guen, Gérard

(56) Documents cités:
- EP-A- 0 283 390
- EP-A- 0 576 350
- WO-A-91/09857

## Description

La présente invention concerne des dérivés amino ramifiés du thiazole, leurs procédés de préparation et les compositions pharmaceutiques qui les contiennent.

Un grand nombre de dérivés du 2-aminothiazole est déjà connu. La demande de brevet EP-0 462 264 décrit des dérivés du 2-aminothiazole, dont l'amine tertiaire en position 2, comporte deux substituants ayant chacun au moins un hétéroatome dont un dérivé d'amine. Ces composés sont des antagonistes du PAF-acéther et trouvent leurs applications dans le traitement de l'asthme, de certains états allergiques ou inflammatoires, de maladies cardiovasculaires, de l'hypertension et de diverses pathologies rénales ou encore comme agents contraceptifs. La demande GB-2 022 285 décrit des composés possédant une activité régulatrice de la réponse immunitaire et ayant des propriétés anti-inflammatoires. Il s'agit de dérivés du thiazole substitués en position 2 par des groupes amines secondaires.

Certains dérivés hétérocycliques de 2-acylaminothiazole ont été décrits dans la demande de brevet EP-0 432 040. Ces composés sont des antagonistes de la cholecystokinine et de la gastrine. Des dérivés du 2-amino-4,5-diphényl thiazole ayant des propriétés antiinflammatoires sont aussi connus (demande de brevet JP-01 75 475). On connaît aussi des dérivés du 2-amino-4-(4-hydroxyphényl)thiazole utiles comme intermédiaires de synthèse pour la préparation des dérivés du 2,2-diaryl chroméno thiazole (demande de brevet EP-0 205 069). Des dérivés du 2-(N-méthyl-N-benzylamino) thiazole sont aussi décrits dans J. Chem. Soc. Perkin, Trans 1, (1984), 2, pp. 147-153 et dans J. Chem. Soc. Perkin, Trans 1, (1983), 2, pp. 341-347.

La demande de brevet EP-0 283 390, décrit parmi d'autres dérivés du thiazole, des dérivés du 2-[N-alkyl N-pyridylalkylamino] thiazole de formule :

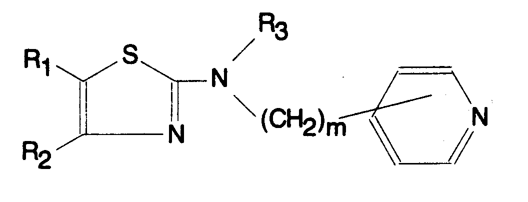

Ces dérivés, dont l'amine en position 2 est substituée par un radical pyridylalkyle non ramifié, sont doués de propriétés pharmacologiques intéressantes et possèdent notamment une activité stimulante de la transmission cholinergique centrale. Ils peuvent donc être utilisés comme agonistes des récepteurs muscariniques et trouvent leurs applications dans le traitement des troubles de la mémoire et des démences séniles.

Les composés de la présente invention se distinguent d'autres dérivés du 2-aminothiazole décrits dans la littérature, par leur structure originale et par leurs propriétés pharmacologiques nouvelles. Il s'agit de dérivés du 2-amino thiazole dont l'amine en position 2 est une amine tertiaire ayant un substituant alkyle ou aralkyle ramifié.

Cette structure particulière, confère aux produits de l'invention des propriétés pharmacologiques très intéressantes. En effet, les composés de l'invention déplacent à de très faibles concentrations - inférieures à 10 µM -, la liaison du ¹²⁵I-CRF aux récepteurs spécifiques présents sur membranes de cerveaux humains et/ou de souris. Les composés selon l'invention vont donc moduler les effets du CRF, peptide dont la séquence de 41 acides aminés, a été caractérisée par VALE et al. en 1981. Le CRF est le principal facteur endogène impliqué dans la régulation de l'axe hypothalamo-hypophyso-surrénalien (libération de l'hormone adrénocorticotrope : ACTH) et ses pathologies, ainsi que dans les syndromes dépressifs qui en découlent. Le CRF provoque également la sécrétion de β-endorphine, de β-lipotropine et de corticostérone. Sa localisation spécifique dans les zones limbiques du cerveau ainsi que dans le locus ceruleus, suggère que ce peptide joue un rôle important dans les réponses comportementales au stress.
De nombreuses expérimentations animales ont montré que l'administration centrale de CRF provoque des effets anxiogènes variés tels que la modification du comportement en général : par exemple néophobie, réduction de la réceptivité sexuelle, diminution de la consommation alimentaire et du sommeil lent chez le rat. L'injection intracérébroventriculaire de CRF augmente également l'excitation des neurones noradrénergiques qui est souvent associée chez l'animal, à un état d'anxiété. Chez le rat, l'administration centrale ou périphérique de CRF induit des modifications de la vidange gastrique, du transit intestinal, de l'excrétion fécale, de la sécrétion acide, ainsi que des effets tensionnels.
L'implication spécifique du CRF dans ces effets, a été mise en évidence par l'utilisation d'un antagoniste peptidique, l'alpha-hélical CRF(9-41) ou d'anticorps spécifiques (RIVIER J. et al., 1984) ; ce qui permet d'envisager pour ce peptide, un rôle dans l'installation de troubles endocriniens et comportementaux liés au stress.
L'administration répétée de CRF chez l'homme provoque des réactions qui ressemblent à celles décrites lors de la dépression, par exemple augmentation de l'émotivité et de l'activité du système nerveux sympathique, diminution de l'appétance sexuelle et apparition de troubles de l'appétit. De plus, l'utilisation chez l'homme d'un test de stimulation de l'axe HPA par le CRF est un méthode complémentaire de diagnostic (CHROUSOS G.P. et al., 1984), qui démontre bien l'implication du CRF dans de nombreuses pathologies telles que la dépression, l'anorexie nerveuse et le sevrage alcoolique.
Il est important de signaler également, trois conséquences possibles des états de stress chronique que sont l'immunodépression, les troubles de la fertilité, ainsi que l'installation du diabète.

Les composés de l'invention trouvent donc leur application dans le traitement des maladies liées au stress et plus généralement dans le traitement de toutes les pathologies impliquant le CRF, tels que par exemple, les désordres psychiatriques, I'anxiété, l'anorexie, les troubles de l'activité sexuelle et de la fertilité, l'immunodépression, les troubles gastrointestinaux et cardiovasculaires ou autres.

Les produits de l'invention possèdent aussi certaines caractéristiques physicochimiques intéressantes. Il s'agit notamment de produits solubles dans les solvants ou solutions couramment utilisés en thérapeutique pour l'administration orale ou parentérale de principes actifs.

La présente invention a plus particulièrement pour objet, les composés de formule I : 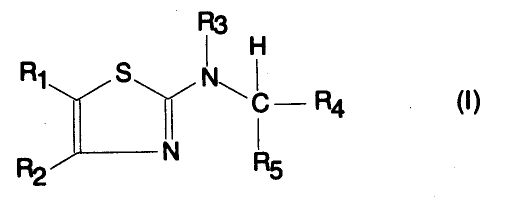 dans laquelle,
- R₁ représente un atome d'hydrogène ou un radical alkyle de 1 à 5 atomes de carbone,
- R₂ représente un radical de formule (A) : 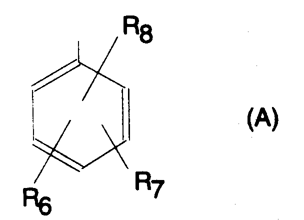 (dans laquelle R₆ représente un radical hydroxyalkyle de 1 à 5 atomes de carbone, et R₇ et R₈, identiques ou différents, représentent chacun un atome d'hydrogène, un atome d'halogène ou un radical hydroxyalkyle de 1 à 5 atomes de carbone, un trifluorométhyle, un radical alcoxy de 1 à 5 atomes de carbone ou un radical alkyle de 1 à 5 atomes de carbone),
   un radical de formule (B) : 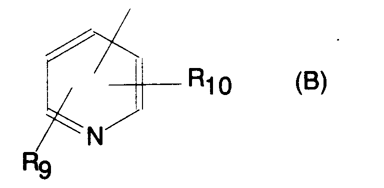 (dans laquelle R₉ et R₁₀ identiques ou différents, représentent chacun un atome d'hydrogène, un atome d'halogène, un radical alkyle de 1 à 5 atomes de carbone ou un radical alcoxy de 1 à 5 atomes de carbone),
   ou un radical de formule (C) : 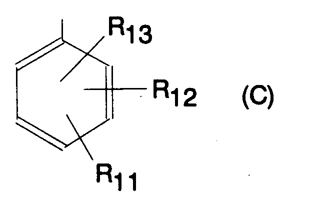 (dans laquelle R₁₁, R₁₂, R₁₃ identiques ou différents, représentent chacun un atome d'hydrogène, un atome d'halogène, un trifluorométhyle, un radical alcoxy de 1 à 5 atomes de carbone ou un radical alkyle de 1 à 5 atomes de carbone),
- R₃ représente un radical alkyle de 1 à 5 atomes de carbone, un radical hydroxyalkyle de 1 à 5 atomes de carbone, un radical tetrahydropyran-2-yloxyalkyle dont le radical alkyle comporte de 1 à 5 atomes de carbone, un radical alcoxyalkyle de 2 à 10 atomes de carbone ou un radical acyloxyalkyle de 3 à 11 atomes de carbone,
- R₄ représente un radical cycloalkyle de 3 à 6 atomes de carbone, un radical hydroxyalkyle de 1 à 5 atomes de carbone, un radical alcoxyalkyle de 2 à 10 atomes de carbone, un radical cycloalkyloxyalkyle de 4 à 11 atomes de carbone, un radical hydroxyalkyloxyalkyle de 2 à 10 atomes de carbone, un radical alcoxyalkyloxyalkyle de 3 à 12 atomes de carbone, un radical acyloxyalkyle de 3 à 11 atomes de carbone ou un radical alkylthioalkyle de 2 à 10 atomes de carbone,
   et,
- R₅ représente un radical cycloalkyle de 3 à 6 atomes de carbone, un radical phényle, un radical thiényle ou un radical pyridyle (éventuellement substitués par un ou plusieurs atomes d'halogène, par des radicaux alcoxy de 1 à 5 atomes de carbone, des radicaux alkyle de 1 à 5 atomes de carbone ou des radicaux trifluorométhyle), ou un radical de formule (D) : 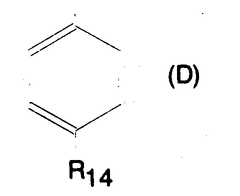 (dans lesquelles R₁₄ représente un radical carboxy, un radical carboxyalkyle de 2 à 6 atomes de carbone, un radical alcoxycarbonyle de 2 à 6 atomes de carbone, un radical acyloxyalkyle de 3 à 11 atomes de carbone, un radical alcoxyalkyle de 2 à 10 atomes de carbone, un radical aralcoxyalkyle de 8 à 16 atomes de carbone (éventuellement substitué sur le cycle aromatique par un ou plusieurs atomes d'halogène, des radicaux alcoxy de 1 à 3 atomes de carbone ou par des radicaux trifluorométhyle), un radical alkyle mono-halogéné de 1 à 5 atomes de carbone, un radical hydroxyalkyle linéaire ou ramifié de 1 à 5 atomes de carbone, un radical de formule (E) : 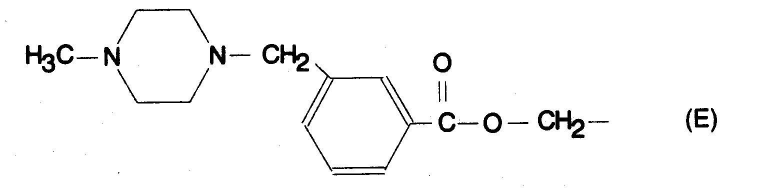 ou un radical sulfoxyalkyle de 1 à 5 atomes de carbone),
   un radical 3-hydroxyalkylpyrid-6-yle ou un radical 2-hydroxyalkylpyrid-5-yle (dont les radicaux alkyle comportent de 1 à 5 atomes de carbone),
   à condition toutefois que lorsque R₃ représente un radical alkyle de 1 à 5 atomes de carbone, R₄ représente un radical cycloalkyle et R₅ représente soit un radical cycloalkyle soit un radical phényle, un radical thiényle ou un radical pyridyle (éventuellement substitués par un ou plusieurs atomes d'halogène, par des radicaux alcoxy de 1 à 5 atomes de carbone, des radicaux alkyle de 1 à 5 atomes de carbone ou des radicaux trifluorométhyle), R₂ ne représente pas un radical de formule (C),
   leurs stéréoisomères et leurs sels d'addition.

Parmi les composés préférés de l'invention, on trouve les composés de formule I' : 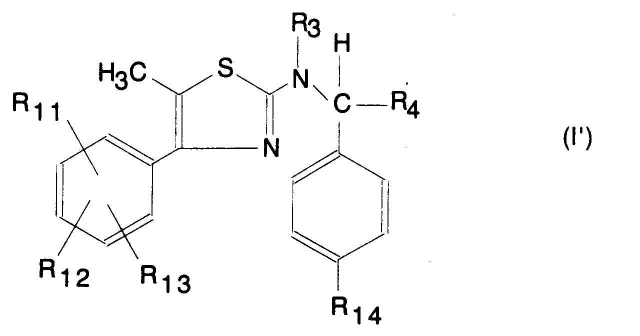 dans laquelle,
- R₁₁ représente un atome d'halogène et R₁₂ et R₁₃ ont la même signification que pour la formule I,
- R₃ représente un radical alkyle de 1 à 5 atomes de carbone,
- R₄ représente un radical cycloalkyle de 3 à 6 atomes de carbone, et
- R₁₄ a la même signification que pour la formule I. leurs stéréoisomères et leurs sels d'addition.

Un autre groupe de composés préférés de l'invention correspond à la formule I'' : 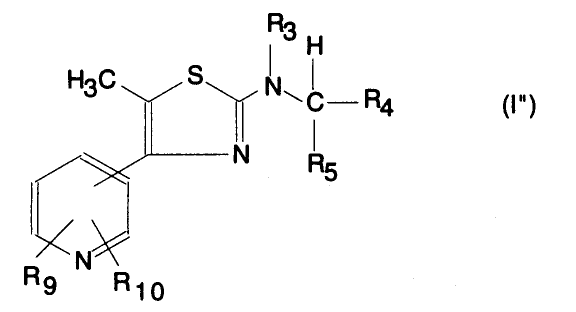 dans laquelle,
- R₃, R₄, R₅, R₉, et R₁₀ ont la signification donnée pour la formule I,
   leurs stéréoisomères et leurs sels d'addition.

Parmi les composés préférés de l'invention sont aussi les composés de formule I'''. 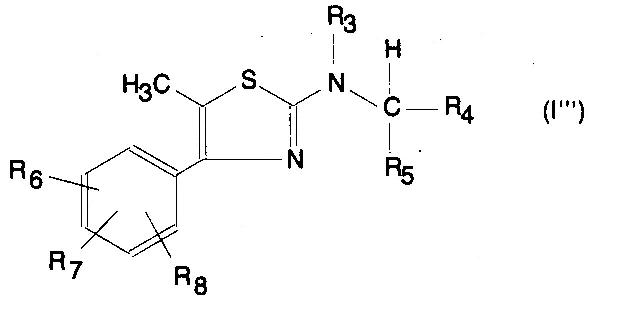 dans laquelle,
- R₇ représente un atome d'halogène ou un alcoxy en C₁-C₅, R₆ et R₈ ont la même signification que pour la formule I,
- R₃ représente un radical alkyle de 1 à 5 atomes de carbone,
   et,
- R₄ et R₅ ont la signification donnée pour la formule I,
   leurs stéréoisomères et leurs sels d'addition.

On préfère plus particulièrement les composés de formule I dans laquelle :
- R₁ représente un atome d'hydrogène ou un radical alkyle de 1 à 5 atomes de carbone,
- R₂ représente un radical de formule (A) ou un radical de formule (B),
- R₃ représente un radical alkyle de 1 à 5 atomes de carbone,
- R₄ représente un radical cycloalkyle de 3 à 6 atomes de carbone,
   et
- R₅ représente un radical cycloalkyle de 3 à 6 atomes de carbone, un radical phényle, un radical thiényle ou un radical pyridyle (éventuellement substitués par un ou plusieurs atomes d'halogène, par des radicaux alcoxy de 1 à 5 atomes de carbone, des radicaux alkyle de 1 à 5 atomes de carbone ou des radicaux trifluorométhyle),
   leurs stéréoisomères et leurs sels d'addition.

On préfère aussi plus particulièrement les composés de formule I dans laquelle :
- R₁ représente un atome d'hydrogène ou un radical alkyle de 1 à 5 atomes de carbone,
- R₂ représente un radical de formule (C),
- R₃ représente un radical alkyle de 1 à 5 atomes de carbone,
- R₄ représente un radical cycloalkyle de 3 à 6 atomes de carbone,
   et,
- R₅ représente un radical de formule (D), un radical 3-hydroxyalkylpyrid-6-yle ou un radical 2-hydroxyalkylpyrid-5-yle,
   leurs stéréoisomères et leurs sels d'addition.

Les composés de formule I dans laquelle,
- R₁ représente un atome d'hydrogène ou un radical alkyle de 1 à 5 atomes de carbone,
- R₂ représente un radical de formule (C),
- R₃ représente un radical alkyle de 1 à 5 atomes de carbone,
- R₄ un radical hydroxyalkyle de 1 à 5 atomes de carbone, un radical alcoxyalkyle de 2 à 10 atomes de carbone, un radical cycloalkyloxyalkyle de 4 à 11 atomes de carbone, un radical hydroxyalkyloxyalkyle de 2 à 10 atomes de carbone, un radical alcoxyalkyloxyalkyle de 3 à 12 atomes de carbone, un radical acyloxyalkyle de 3 à 11 atomes de carbone ou un radical alkylthioalkyle de 2 à 10 atomes de carbone, et,
- R₅ représente un radical cycloalkyle de 3 à 6 atomes de carbone, un radical phényle, un radical thiényle ou un radical pyridyle (éventuellement substitués par un ou plusieurs atomes d'halogène, par des radicaux alcoxy de 1 à 5 atomes de carbone, des radicaux alkyle de 1 à 5 atomes de carbone ou des radicaux
   trifluorométhyle), leurs stéréoisomères et leurs sels d'addition sont aussi des composés particulièrement préférés de l'invention.

On préfère aussi plus particulièrement les composés de formule I dans laquelle,
- R₁ représente un atome d'hydrogène ou un radical alkyle de 1 à 5 atomes de carbone,
- R₂ représente un radical de formule (C),
- R₃ représente un radical hydroxyalkyle de 1 à 5 atomes de carbone, un radical tetrahydropyran-2-yloxyalkyle dont le radical alkyle comporte de 1 à 5 atomes de carbone, un radical alcoxyalkyle de 2 à 10 atomes de carbone ou un radical acyloxyalkyle de 3 à 11 atomes de carbone,
- R₄ représente un radical cycloalkyle de 3 à 6 atomes de carbone,
   et,
- R₅ représente un radical cycloalkyle de 3 à 6 atomes de carbone, un radical phényle, un radical thiényle ou un radical pyridyle (éventuellement substitués par un ou plusieurs atomes d'halogène, par des radicaux alcoxy de 1 à 5 atomes de carbone, des radicaux alkyle de 1 à 5 atomes de carbone ou des radicaux trifluorométhyle),
   leurs stéréoisomères et leurs sels d'addition.

Par le terme radical alkyle, on entend des radicaux alkyle de 1 à 5 atomes de carbone, linéaires ou ramifiés. Il en est de même des radicaux alkyle substitués par d'autres radicaux tels qu'hydroxyalkyle etc.

Par le terme aralkyle on entend un radical de formule : 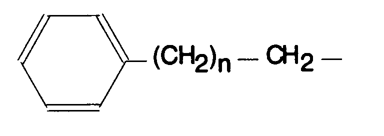 dans laquelle n peut prendre les valeurs allant de 0 à 4.

Parmi les composés préférés de l'invention on peut citer les composés suivants :
Le 4-(2,4-dichlorophényl)-5-méthyl-2-{N-[α-cyclopropyl-4-(méthoxycarbonyl) benzyl] N-propylamino} thiazole,
le 4-(2,4-dichlorophényl)-5-méthyl-2-{N-[α-cyclopropyl-4-(hydroxyméthyl)benzyl] N-propyl amino} thiazole,
le 4-(2,4-dichlorophényl)-5-méthyl-2-{N-[α-cyclopropyl-4-(éthoxycarbonyl)benzyl] N-propylamino} thiazole,
le 4-(2,4-dichlorophényl)-5-méthyl-2-{N-[α-(acétoxyméthyl)benzyl] N-propylamino} thiazole,
le 4-(2,4-dichlorophényl)-5-méthyl-2-{N-[α-cyclopropyl-4-(acétoxyméthyl)benzyl] N-propylamino} thiazole,
le 4-(2,4-dichlorophényl)-5-méthyl-2-{N-[α-cyclopropyl-4-(iodométhyl)benzyl] N-propylamino} thiazole,
le 4-(2,4-dichlorophényl)-5-méthyl-2-{N-[α-(méthoxyméthyl)benzyl]-N-propylamino} thiazole,
le 4-(2,4-dichlorophényl)-5-méthyl-2-{N-[α(-éthoxyméthyl)benzyl]-N-propylamino} thiazole,
le 4-(2,4-dichlorophényl)-5-méthyl-2-{N-[α-cyclopropyl-4-(1-hydroxyéth-1-yl)benzyl] N-propylamino} thiazole,
le 4-(2,4-dichlorophényI)-5-méthyl-2-{N-[α-hydroxyéthyloxyméthyl)benzyl] N-propylamino] thiazole,
le 4-(2,4-dichlorophényl)-5-méthyl-2-{N-[α-(méthoxyéthyloxyméthyl)benzyl] N-propylamino} thiazole,
le 4-(2,4,5-trichlorophényl)-5-méthyl-2{N-[α-(méthoxyméthyl)benzyl] N-propylamino}thiazole,
le 4-(2,4-dichlorophényl)-5-méthyl-2-{N-[α- cyclopropyl-4-(méthoxyéthyl)benzyl] N-propylamino} thiazole,
le 4-(2-méthyl-4-chlorophényl)-5-méthyl-2-{N-[α-(méthoxyméthyl)benzyl] N-propylamino} thiazole,
le 4-(2-chloro-4-méthylphényl)-5-méthyl-2-{N-[α-(méthoxyméthyl)benzyl] N-propylamino} thiazole,
le (R)-4-(2-chloro-4-méthyloxyphényl)-5-méthyl-2-{N-[α-(méthoxyméthyl)benzyl] N-propylamino} thiazole,
le (S)-4-(2-chloro-4-méthyloxyphényl)-5-méthyl-2-{N-[α-(méthoxyméthyl)benzyl] N-propylamino} thiazole,
le 4-(2,4-diméthoxyphényl)-5-méthyl-2-{N-[α-(méthoxyméthyl)benzyl] N-propylamino} thiazole,
le 4-(2-chloro-4-méthoxyphényl)-5-méthyl-2-{N-[α-(méthoxyméthyl)benzyl] N-propylamino} thiazole,
le 4-(2-méthoxy-4-chlorophényl)-5-méthyl-2-{N-[α-(méthoxyméthyl)benzyl] N-propylamino} thiazole,
le 4-(2-méthoxy-4-méthylphényl)-5-méthyl-2-{N-[α-(méthoxyméthyl)benzyl] N-propylamino} thiazole,
le 4-(2-méthyl-4-méthoxyphényl)-5-méthyl-2-{N-[α-(méthoxyméthyl)benzyl] N-propylamino} thiazole,
le 4-(2-chloro-4-méthoxyphényl)-5-méthyl-2-{N-[α-(méthoxyméthyl)cyclopropyl méthyl] N-propylamino} thiazole,
le 4-(2-chloro-4-méthoxyphényl)-5-méthyl-2-{N-[α-(méthylthiométhyl)benzyl] N-propylamino} thiazole,
le 4-(2-méthoxy-4-chlorophényl)-5-méthyl-2-{N-[α-(méthylthiométhyl)benzyl] N-propylamino} thiazole,
le 4-(2,5-dichloro-4-méthoxyphényl)-5-méthyl-2-{N-[α-(méthoxyméthyl)benzyl] N-propylamino} thiazole,
le 4-(2,5-dichloro-4-méthoxyphényl)-5-méthyl-2-{N-[α-(méthylthiométhyl)benzyl] N-propylamino} thiazole,
le 4-(2,4-dichlorophényl)-5-méthyl-2-{N-[α-(méthylthiométhyl)-4-fluorobenzyl] N-propylamino} thiazole,
le 4-(2-méthyl-4-méthoxy-5-chlorophényl)-5-méthyl-2-{N-[α-(méthoxyméthyl) benzyl] N-propylamino} thiazole,
le 4-(2,4-dichlorophényl)-5-méthyl-2-{N-[α-(méthoxyméthyl)(4-fluorobenzyl)] N-propylamino} thiazole,
le 4-(2,4,5-trichlorophényl)-5-méthyl-2-{N-[α-(méthoxyméthyl)(4-fluorobenzyl)] N-propylamino} thiazole,
le 4-(2-méthyl-4-méthoxy-5-chlorophényl)-5-méthyl-2-{N-[α-(méthoxyméthyl)(4-fluorobenzyl)] N-propylamino} thiazole,
le 4-(2-chloro-4-méthoxyphényl)-5-méthyl-2-{N-[α-(méthoxyméthyl) 4-fluorobenzyl] N-propylamino} thiazole,
le 4-(2,4-dichlorophényI)-5-méthyl-2-{N-[α-(méthylthiométhyl)benzyl] N-propylamino}thiazole
le 4-(2,4-dichlorophényl)-5-méthyl-2-[N-(1-cyclopropyl-2-méthoxy éth-1-yl) N-propylamino] thiazole
le 4-(2,4-dichlorophényl)-5-méthyl-2-{N-[α-(cyclopropylméthoxyméthyl) benzyl] N-propylamino} thiazole
le 4-(2,4-dichlorophényl)-5-méthyl-2-{N-[α-(méthoxyméthyl)benzyl] N-propylamino} thiazole,
le (R)-4-(2,4-dichlorophényl)-5-méthyl-2-{N-[α-(méthoxyméthyl)benzyl] N-propylamino} thiazole,
le (S)-4-(2,4-dichlorophényl)-5-méthyl-2-{N-[α-(méthoxyméthyl)benzyl] N-propylamino} thiazole,
le 4-(2,4-dichlorophényl)-5-méthyl-2-{N-[α-(méthoxyméthyl)thién-2-yl-méthyl]N-propylamino} thiazole,
le 4-(2-chloro-4-méthoxyphényl)-5-méthyl-2-{N-[α-(méthoxyméthyl)thién-2-yl-méthyl] N-propylamino} thiazole,
le 4-(2,4-dichlorophényl)-5-méthyl-2-{N-[(α-cyclopropyl)-4-hydroxyméthylpyrid-6-yl-méthyl] N-propylamino} thiazole.

Tous ces composés peuvent être sous forme de sels.

Les composés de l'invention sous forme libre présentent généralement des propriétés basiques, or il en existe certains ayant des propriétés acides et ceci en fonction des substituants.

Les sels des composés de formule I avec des acides ou des bases (lorsque cela est possible) pharmaceutiquement acceptables sont les sels préférés, mais ceux qui peuvent permettre d'isoler les composés de formule I, notamment de les purifier ou d'obtenir des isomères purs, sont aussi objet de l'invention.

Parmi les acides pharmaceutiquement acceptables pour la préparation des sels d'addition aux composés de formule I, on peut citer les acides chlorhydrique, phosphorique, fumarique, citrique, oxalique, sulfurique, ascorbique, tartrique, maléique, mandélique, méthanesulfonique, lactobionique, gluconique, glucarique, succinyle sulfonique, hydroxypropane sulfonique, etc.

Parmi les bases pharmaceutiquement acceptables pour la préparation des sels d'addition aux composés de formule I lorsque ceux-ci ont des propriétés acides, on peut citer l'hydroxyde de sodium, de potassium, d'ammonium etc.

La présente invention a également pour objet un procédé de préparation des composés de formule I, caractérisé en ce que l'on fait réagir un dérivé carbonylé alpha-halogéné, de préférence alpha-bromé, de formule II : 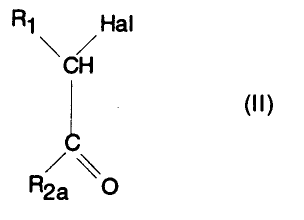 dans laquelle,
- R₁ a la même signification que pour la formule I,
- R₂ₐ représente un radical de formule (Aₐ) : 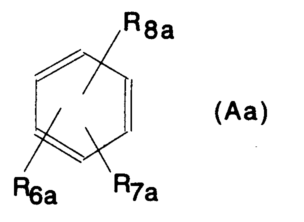 (dans laquelle R₆ₐ représente un atome de brome ou d'iode, et R₇ₐ et R₈ₐ, identiques ou différents, représentent chacun un atome d'hydrogène ou un atome d'halogène), un radical de formule (B) ou un radical de formule (C), les radicaux (B) et (C) ayant la même signification que pour la formule I,
   et,
- Hal représente un atome d'halogène, de préférence le brome
   soit
   avec une thiourée de formule IIIₐ : 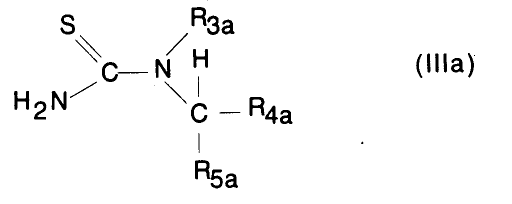 dans laquelle,
- R₃ₐ représente un radical alkyle de 1 à 5 atomes de carbone,
- R₄ₐ représente un radical cycloalkyle de 3 à 6 atomes de carbone, ou un radical de formule (F) :

   - Alk - O - ProtecA (F)

   (dans laquelle Alk représente un radical alkyle de 1 à 5 atomes de carbone et ProtecA, un groupe protecteur éliminable par hydrolyse acide tel qu'un radical tetrahydropyran-2-yle), un radical alcoxyalkyle de 2 à 10 atomes de carbone ou un radical alkylthioalkyle de 2 à 10 atomes de carbone,
- R₅ₐ représente un radical cycloalkyle de 3 à 6 atomes de carbone, un radical phényle, un radical thiényle ou un radical pyridyle (éventuellement substitués par un ou plusieurs atomes d'halogène, par des radicaux alcoxy de 1 à 5 atomes de carbone, des radicaux alkyle de 1 à 5 atomes de carbone ou par des radicaux trifluorométhyle), un radical 3-hydroxyalkylpyrid-6-yle, un radical 2-hydroxy alkylpyrid-5-yle ou un radical de formule (D) dans laquelle R₁₄ représente un radical hydroxyalkyle de 2 à 5 atomes de carbone,
   pour former des composés de formule Ia : 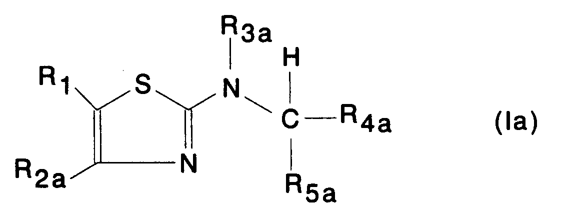 dans laquelle,
- R₁ a la même signification que pour la formule I,
- R₂ₐ a la signification donnée pour la formule II,
   et
- R₃ₐ, R₄ₐ et R₅ₐ ont la signification donnée pour la formule IIIa,
   soit
   avec une thiourée de formule III_{b} : 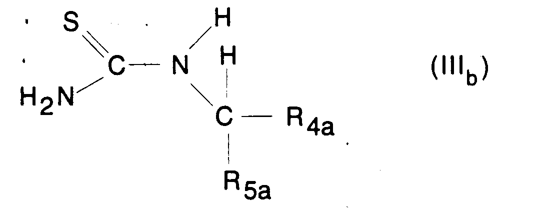 dans laquelle,
- R₄ₐ et R₅ₐ ont les mêmes significations que pour la formule IIIₐ. pour former les composés de formule IV : 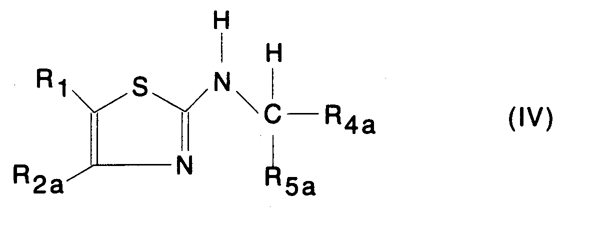 dans laquelle,
- R₁ a la signification donnée pour la formule I,
- R₂ₐ a la signification donnée pour la formule II,
   et - R₄ₐ et R₅ₐ, les significations données pour la formule IIIₐ,
   que l'on fait réagir avec un halogénure de formule V :

   Hal-R_{3b} (V)

   dans laquelle,
- Hal représente un atome d'halogène
   et,
- R_{3b} représente un radical alkyle de 1 à 5 atomes de carbone ou un radical de formule (F),
   pour former les composés de formule Ib : 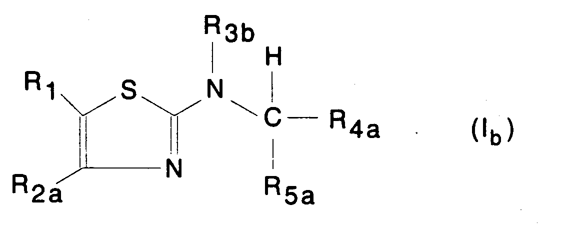 dans laquelle,
- R₁ a la signification donnée pour la formule I,
- R₂ₐ a la signification donnée pour la formule II,
- R₄ₐ et R₅ₐ, les significations données pour la formule IIIₐ
   et,
- R_{3b} a la signification donnée pour la formule V,
   et ensuite, on réalise une des étapes suivantes (a) à (d)

(a) l'on soumet les composés de formules Iₐ et I_{b} dans lesquelles R₂ₐ représente un radical de formule (Aₐ) :
   * soit à l'action du *tert*-butyllithium et d'anhydride carbonique et ensuite à une réduction pour obtenir les composés de formule I dans laquelle R₂ représente un radical de formule (A), dont le substituant R₆ et éventuellement les substituants R₇ et/ou R₈ représentent un radical hydroxyméthyle,
   * soit à l'action du *tert*-butyllithium et d'un aldéhyde aliphatique (C₂-C₅) pour obtenir les composés de formule I dans laquelle R₂ représente un radical de formule (A) dont le substituant R₆ et éventuellement les substituants R₇ et/ou R₈ représentent un radical hydroxyalkyle linéaire ou ramifié de 2 à 5 atomes de carbone,
(b) ou,
   * soit
      l'on soumet les composés de formule Iₐ et I_{b} dans lesquelles R₅ₐ représente un radical 4-bromophényle
      ou,
      à l'action d'un lithien tel que par exemple le *tert*-butyllithium dans un solvant approprié éthéré et d'anhydride carbonique pour obtenir les composés de formule I dans laquelle R₅ représente un radical 4-carboxyphényle, à partir desquels sont ensuite obtenus :
      - par estérification, les composés de formule I dans laquelle R₅ représente un radical 4-(alcoxycarbonyl) phényle,
      - par réduction, les composés de formule I dans laquelle R₅ représente un radical 4-(hydroxyméthyl) phényle,
      - par réduction et ensuite estérification, les composés de formule I dans laquelle R₅ représente un radical 4-(acyloxyméthyl) phényle,
      - par réduction et ensuite action d'un halogénure d'alkyle ou d'un halogénure d'aralkyle, les composés de formule I dans laquelle R₅ représente un radical 4-(alcoxyméthyl) phényle ou 4-(aralcoxyméthyl) phényle,
      - par réduction et ensuite par action de chlorhydrine sulfurique, les composés de formule I dans laquelle R₅ représente un radical 4-(sulfoxyméthyl)phényle,
      - par réduction, action du chlorure de 3-chlorométhylbenzoyle puis de la N-méthylpipérazine, les composés de formule I dans laquelle R₅ représente un radical de formule (D) et R₁₄ est un radical de formule (E),
      - par réduction puis action d'un halogène, les composés de formule I dans laquelle R₅ représente un radical 4-(halogénométhyl) phényle,
      ou,
      à l'action du *tert*-butyllithium et à l'action d'un aldéhyde aliphatique pour obtenir les composés de formule I dans laquelle R₅ représente un radical 4-(*sec*-hydroxyalkyl) phényle, d'une cétone aliphatique pour obtenir les composés de formule I dans laquelle R₅ représente un radical 4-(*tert*-hydroxyalkyl) phényle,
   * soit
      l'on soumet les composés de formule Iₐ et I_{b} dans lesquelles R₅ₐ représente un radical de formule (D) dans laquelle R₁₄ représente un radical hydroxyalkyle de 2 à 5 atomes de carbone :
      - à une estérification pour obtenir les composés de formule I dans laquelle R₅ représente un radical 4-(acyloxyalkyl) phényle,
      - à une alkylation pour obtenir les composés de formule I dans laquelle R₅ représente un radical 4-(alcoxyalkyl) phényle ou 4-(aralcoxyalkyl) phényle,
      - à l'action de chlorhydrine sulfurique pour obtenir les composés de formule I dans laquelle R₅ représente un radical 4-(sulfoxyalkyl)phényle,
      - à l'action d'un halogène pour obtenir les composés de formule I dans laquelle R₅ représente un radical 4-(halogénoalkyl) phényle,
(c) ou,
   l'on soumet les composés de formule Iₐ et I_{b} dans lesquelles R₄ₐ représente un radical de formule (F) à une déprotection, pour obtenir les composés de formule I dans laquelle R₄ représente un radical hydroxyalkyle, à partir desquels sont ensuite obtenus :
   - par estérification, les composés de formule I dans laquelle R₄ représente un radical acyloxyalkyle,
   - par alkylation, les composés de formule I dans laquelle R₄ représente un radical alcoxyalkyle ou cycloalkyloxyalkyle,
   - par alkylation avec des alcools aliphatiques halogénés protégés puis déprotection, les composés de formule I dans laquelle R₄ représente un radical hydroxyalkyloxyalkyle,
   - par alkylation avec des alcools aliphatiques halogénés protégés, puis déprotection et ensuite alkylation, les composés de formule I dans laquelle R₄ représente un radical alcoxyalkyloxyalkyle,
(d) ou,
   l'on soumet les produits de formule I_{b} dans laquelle R_{3b} représente un radical de formule (F) à une déprotection par hydrolyse acide, pour obtenir les composés de formule I, dans laquelle R₃ représente un radical hydroxyalkyle, à partir desquels sont obtenus ensuite par alkylation ou par estérification, les composés de formule I, dans laquelle R₃ représente respectivement un radical alcoxyalkyle, ou un radical acyloxyalkyle,
   et le cas échéant, les composés de formule I,
   sont ensuite éventuellement séparés en leurs stéréoisomères possibles et/ou salifiés, pour former les sels correspondants.

Les dérivés de formule II peuvent être obtenus à partir des cétones correspondantes non halogénées de formule R₂ₐ-CO-CH₂-R₁, soit par action du brome dans un solvant organique approprié, tel que l'acide acétique, le tétrachlorure de carbone ou l'éther éthylique, soit par action des tribromures d'ammonium quaternaires selon la méthode décrite dans Bull. Chem. Soc. Japan (1987), 60, pp. 1159-1160 et pp. 2667-2668, soit encore par action du bromure cuivrique dans un solvant organique, tel qu'un mélange de chloroforme et d'acétate d'éthyle (J. Org. Chem. (1964), 29, pp. 3451-3461).

Les cétones de formule R₂ₐ-CO-CH₂-R₁, sont en général des produits connus et disponibles dans le commerce. Ces composés peuvent être préparés par la réaction de Friedel et Crafts, entre un composé de formule R₂ₐH et un halogénure d'acyle de formule R₁CH₂COHal (dans laquelle Hal représente un atome d'halogène), de préférence un chlorure d'acyle de formule R₁CH₂COCl, en présence d'un acide de Lewis.

Les composés de formule II, dans laquelle R₂ₐ représente un radical de formule (C) substitué en positions 2 et 4 par un atome d'halogène, et R₁ représente un radical méthyle, peuvent être obtenus à partir des dérivés halogénés du benzène et notamment par les benzènes 1,3-dihalogénés et les alcoxyphényles, sur lesquels on fait réagir le bromure de 2-bromo propionyle en présence de chlorure d'aluminium.

Les composés de formule II, dans laquelle R₂ₐ représente un radical 2,6-dihalogénopyrid-3-yle, sont obtenus soit à partir des dérivés 2,6-dihalogénés correspondants de 3-formyl pyridine, sur lesquels on fait réagir des organomagnésiens aliphatiques, pour obtenir des alcools secondaires substitués en position 1 par des radicaux 2,6-dihalogénopyrid-3-yle, lesquels sont ensuite soumis à une oxydation pour obtenir les cétones correspondantes.

Ces derniers composés sont ensuite transformés selon les méthodes indiquées précédemment en bromocétones de formule II.

Les composés de formule II dans laquelle R₂ₐ représente un radical 2,4-dialkyl pyrid-5-yle sont obtenus à partir des dérivés de la 2,4-dialkyl 5-cyanopyridine sur lesquels on fait réagir des dérivés organomagnésiens aliphatiques. On obtient ainsi des (2,4-dialkyl pyrid-5-yl) alkyl cétones qui sont ensuite transformées en dérivés carbonylés alpha bromé de formule II.

Les composés de formule IIIₐ et III_{b} sont obtenus à partir des composés de formule VI : 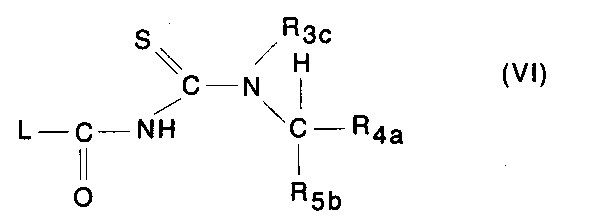 dans laquelle,
- R_{3c} a la même signification que pour la formule IIIₐ ou représente un atome d'hydrogène,
- R₄ₐ a la signification de R₄ₐ de la formule IIIₐ,
- R_{5b} a la signification de R_{5b} de la formule IIIₐ ou représente un radical 2-hydroxyalkylpyrid-5-yle, un radical 3-hydroxyalkylpyrid-6-yle ou un radical 4-hydroxyalkylphényle dans lesquels l'hydrogène de la fonction alcool a été remplacé par un groupe protecteur éliminable en milieu basique ou acide suivant le cas,
   et,
- L représente un radical phényle ou un radical *tert*-butyle,
   soit par un traitement basique en utilisant de préférence l'hydroxyde de sodium. soit par un traitement acide en utilisant de préférence l'acide chlorhydrique,

Quand L est un radical phényle, le traitement par un acide minéral est particulièrement utilisé lorsque R_{5b} est un radical pyridyle éventuellement substitué par un ou plusieurs atomes d'halogène, par des radicaux alcoxy, des radicaux alkyle ou des radicaux trifluorométhyle.

On procède à un traitement basique lorsque R₄ₐ est un groupement cycloalkyle, par exemple un cyclopropyle, ou lorsque R₄ₐ représente un radical de formule (F). On procède aussi à un traitement basique lorsque R_{5b} représente soit un radical 2-hydroxyalkylpyrid-5-yle, soit un radical 3-hydroxyalkylpyrid-6-yle soit un radical 4-hydroxyalkylphényle dont l'hydrogène de la fonction alcool a été remplacé par un groupe protecteur éliminable en milieu basique ou acide suivant le cas.

Quand L représente un radical *tert*-butyle, les dérivés de thiourées de formules IIIa et IIIb sont obtenus à partir des composés de formule VI par action d'un acide fort, par exemple de l'acide chlorhydrique concentré, à une température comprise entre 10°C et 100°C.

Lorsque R₄ₐ représente un radical de formule (F), les composés de formule IIIₐ et III_{b} sont obtenus à partir des composés de formule VI dans laquelle R_{4b} représente un radical hydroxyalkyle. On procède d'abord à la protection du radical hydroxyalkyle avec un groupe protecteur éliminable par une hydrolyse acide et ensuite on soumet les composés ainsi obtenus à un traitement basique pour obtenir les composés de formule IIIₐ et III_{b}.

Les composés de formule VI sont obtenus en faisant réagir l'isothiocyanate de benzoyle ou l'isothiocyanate de pivaloyle sur les amines de formule VII : 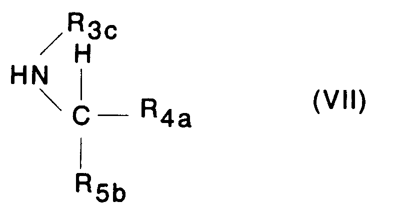 dans laquelle,
R_{3c}, R₄ₐ et R_{5b} ont la même signification que pour la formule VI,

Les amines de formule VII lorsqu'il s'agit d'amines secondaires, peuvent être préparées par des méthodes classiques.

Selon une première méthode, lorsque R_{3c} représente un radical alkyle de 1 à 5 atomes de carbone, on procède à l'alcoylation de l'amine primaire correspondante VIIₐ : 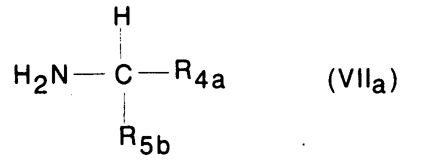 par un halogénure d'alkyle de 1 à 5 atomes de carbone, de préférence à chaud, en présence d'un sel alcalin dans un solvant organique polaire, par exemple le diméthylformamide.

Selon une autre méthode d'alcoylation les amines de formule VIIₐ sont soumises à l'action d'un halogénure d'acide ou d'un anhydride d'acide dans un solvant organique choisi parmi les hydrocarbures halogénés, tel que le chlorure de méthylène, en présence d'un accepteur de protons, de préférence la triéthylamine. L'amide issue de cette réaction est ensuite réduite par des hydrures (AlLiH₄ ou autres) dans des solvants organiques de type éther.

Les deux méthodes citées ci-dessus sont utilisées de manière préférentielle pour la préparation des composés de formule VII, sous forme d'énantiomères purs.

Une autre méthode de préparation des composés de formule VII consiste à condenser une amine primaire de formule R₃ₐNH₂ (dans laquelle R₃ₐ a la même signification que pour la formule IIIₐ) avec une cétone en milieu déshydratant, pour former l'imine correspondante laquelle ensuite est réduite de façon classique par un hydrure métallique, de préférence le borohydrure de sodium, ou par l'hydrogène en présence d'un catalyseur approprié. Lors de la réaction de l'amine primaire de formule R₃ₐNH₂ avec une cétone en milieu déshydratant, on utilise de manière préférentielle, soit le chlorure de titane IV (TiCl₄), soit une catalyse par l'acide paratoluène sulfonique.

Les amines de formule VII lorsque R_{3c} représente un radical alkyle de 1 à 5 atomes de carbone, sont préparées de manière préférentielle selon une méthode dont le principe est donné dans le schéma suivant :

### Etape A

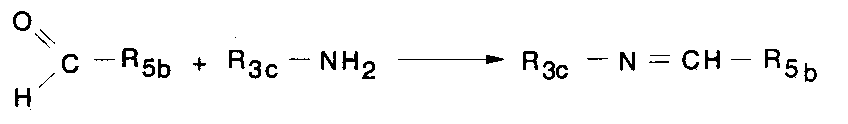

### Etape B

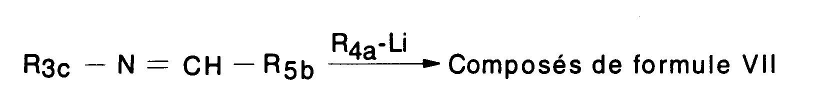

La condensation de l'aldéhyde avec l'amine primaire à l'Etape A est réalisée de préférence dans l'éthanol ou dans le toluène, à température ambiante et la réaction de l'imine avec un dérivé d'alkyllithium à l'Etape B, est réalisée dans l'éther éthylique ou le tétrahydrofurane à une température comprise entre 0°C et 15°C.

Les composés de la présente invention possèdent des propriétés pharmacologiques fort intéressantes. Les composés de l'invention déplacent notamment à des concentrations inférieures à 10 µM (0,01-10 µM) la liaison du ¹²⁵I-CRF aux récepteurs spécifiques présents sur membranes de cortex de rat, selon la méthode décrite par De Souza E.B. (J. Neurosci (1987), 7 (1), pp. 88-100). Cela est étonnant et inattendu, puisque des composés de structure proche de celle des composés de l'invention, mais dont l'amine en position 2 du noyau thiazole ne comporte pas de substituant ramifié, ne déplacent pas de manière significative la liaison ¹²⁵I-CRF.

En effet, le 2-[N-méthyl N-(pyrid-3-yl-méthyl) amino]-4-(2,4,6-triméthylphényl) thiazole, composé décrit dans l'exemple 112 de la demande de brevet EP-0 283 390, ne provoque qu'un déplacement d'environ 8 %, à la concentration 10⁻⁵ M.

Le facteur de libération de l'hormone corticotrope (C.R.F.) est un neuropeptide qui contrôle l'activité de l'axe hypothalamo-hypophyso-surrénalien. Ce facteur est responsable des réponses endocrines et comportementales liées au stress.

En effet, il a été démontré que le CRF peut moduler le comportement comme aussi certaines fonctions du système nerveux autonome (G.F. Koob, F.E. Bloom, Fed. Proc. (1985), 44, p. 259 ; M.R. Brown, L.A. Fisher, Fed. Proc. (1985), 44, p. 243). Plus particulièrement, le CRF induit la sécrétion de la corticotropine (ACTH), de β-endorphine et autres peptides dérivés de la pro-opiomélanocortine (A. Tazi et al, Régul. Peptides (1987) 18, p. 37 ; M.R. Brown et al, Regul. Peptides (1986) 16, p. 321 ; C.L. Williams et al., Am. J. Physiol.. (1987), G 582, p. 253).

Les composés de l'invention peuvent être donc utiles à la régulation de la sécrétion de ces substances endogènes. Ils trouvent plus spécialement leurs applications en tant que principes actifs des médicaments pour diminuer la réponse au stress (comportement, états émotionnels, troubles gastro-intestinaux et cardiovasculaires, désordres du système immunitaire) et plus généralement dans les pathologies impliquant le CRF, par exemple désordres psychiatriques, anxiété, anorexie nerveuse, troubles d'activité sexuelle et de fertilité, maladie d'Alzheimer ou autres.

Les produits de l'invention sont des produits très peu toxiques. Cette propriété permet d'envisager des doses journalières importantes.

L'invention s'étend aussi aux compositions pharmaceutiques contenant comme principe actif au moins un composé de formule générale I, ou l'un de ses sels avec un acide minéral ou organique pharmaceutiquement compatible, en association avec un ou plusieurs excipients inertes et appropriés.

Les compositions pharmaceutiques ainsi obtenues sont présentées avantageusement sous des formes diverses telles que par exemple comprimés, dragées, gélules, suppositoires, solutions injectables ou buvables.

Le principe actif peut également être présenté sous forme de complexe avec une cyclodextrine, par exemple α, β, γ-cyclodextrine, 2-hydroxypropyl-β-cyclodextrine, méthyl-β-cyclodextrine.

La posologie peut largement varier en fonction de l'âge, du poids du patient, de la nature et de la sévérité de l'affection ainsi que de la voie d'administration. D'une manière générale, la posologie unitaire s'échelonnera entre 0,5 mg et 200 mg et la posologie journalière utilisable en thérapeutique humaine, entre 0,5 mg et 800 mg.

La voie d'administration préférée est la voie orale ou parentérale.

Les exemples suivants illustrent l'invention.

Dans les différentes préparations sont décrites les méthodes de synthèse des différents intermédiaires permettant d'obtenir les composés de l'invention.

Les points de fusion ont été mesurés selon la technique Micro-Köfler.

Les spectres de résonance magnétique nucléaire du proton (RMN-¹H) des composés de formule I ont été enregistrés, selon le cas, à 200 MHz ou à 100 MHz.

Les composés de l'invention présentent une analyse centésimale conforme à la théorie.

### PREPARATIONS

### PREPARATION DES COMPOSES DE FORMULE II

### PREPARATION I

### 2-bromo-1-(2,4-dichlorophényl) propan-1-one(Composé 1)

A 7 g de 1-(2,4-dichlorophényl) propan-1-one en solution dans un mélange de 420 ml de chlorure de méthylène et de 140 ml de méthanol, ajouter 17,4 g de tribromure de *tetra*-butyl ammonium à température ambiante. Après 24 heures, évaporer à sec le milieu réactionnel, sous vide. Reprendre à l'eau, extraire avec de l'acétate d'éthyle, sécher la phase organique par du sulfate de sodium. Evaporer sous vide, puis purifier sur colonne de silice, en utilisant comme éluant un mélange de cyclohexane et d'acétate d'éthyle (20 : 1 V/V). Huile.
Rendement : 78 %

De la même manière, peut aussi être obtenue la 2-bromo-1-(2-chloro 4-méthoxyphényl) propan-1-one ***(Composé 2)***.

### PREPARATION II

### 2-bromo-1-(2,4,6-triméthylphényl) éthan-1-one(Composé 3)

Dissoudre 0,3 mole de 1-(2,4,6-triméthylphényl) éthan-1-one dans 200 ml d'acide acétique glacial et ajouter goutte à goutte 31,8 g de brome, en maintenant le milieu réactionnel à une température inférieure à 10°C. En fin d'addition, laisser revenir le milieu réactionnel à température ambiante et abandonner à cette température pendant 2 heures. Verser ensuite le milieu réactionnel sur 500 ml d'eau glacée et extraire la phase aqueuse avec de l'éther éthylique. Laver les extraits organiques avec une solution aqueuse saturée en bicarbonate de sodium, puis avec de l'eau salée et sécher sur du sulfate de magnésium anhydre.

Après évaporation du solvant, on obtient une huile qui peut être utilisée sans autre purification.

### Autres composés(Composés 4 à 12)

Les composés suivants ont été obtenus selon la méthode décrite pour la préparation de 2-bromo-1-(2,4,6-triméthylphényl) éthan-1-one, en utilisant comme matières premières, les cétones adéquates.
- ***Composé 4*** :: 2-bromo-1-(2,4-diméthylphényl) propan-1-one
- ***Composé 5*** :: 2-bromo-1-(4-chloro 2-méthylphényl) propan-1-one
- ***Composé 6*** :: 2-bromo-1-(2-chloro 4-méthylphényl) propan-1-one
- ***Composé 7*** :: 2-bromo-1-(2,4-diméthoxyphényl) propan-1-one
- ***Composé 8*** :: 2-bromo-1-(4-chlorophényl)propan-1-one
- ***Composé 9*** :: 2-bromo-1-(2,4-dichlorophényl) éthan-1-one
- ***Composé 10*** :: 2-bromo-1-(4-méthoxyphényl) propan-1-one
- ***Composé 11*** :: 2-bromo-1-(4-chloro-2-méthoxyphényl) propan-1-one
- ***Composé 12*** :: 2-bromo-1-(4-méthylphényl) propan-1-one

### PREPARATION III

### 2-bromo-1-(2,4,6-triméthoxyphényl) propan-1-one(Composé 13)

Porter à reflux une suspension de 45,3 g de bromure cuivrique dans 150 ml d'acétate d'éthyle et ajouter rapidement à cette température 25,1 g de 1-(2,4,6-triméthoxyphényl) propan-1-one en solution dans 150 ml de chloroforme. Il apparaît un abondant précipité jaune verdâtre.

Porter le milieu réactionnel à reflux pendant 2 heures et 30 minutes. Laisser ensuite revenir à température ambiante, filtrer les sels insolubles et laver avec de l'acétate d'éthyle.

Les phases organiques sont traitées avec du noir animal. Après élimination du solide par filtration, concentrer sous pression réduite pour obtenir une huile.

Purifier par chromatographie sur colonne de silice en utilisant comme éluant un mélange de cyclohexane et d'acétate d'éthyle (6 : 4 V/V). Huile.
Rendement : 60 %

### PREPARATION IV

### 2-bromo-1-(2,4-dibromophényl) propan-1-one(Composé 14)

A 25 g de 1,3-dibromobenzène dans 250 ml de sulfure de carbone, ajouter avec précaution à 0°C, 15 g de chlorure d'aluminium puis couler lentement 22,86 g de bromure de 2-bromopropionyle. Porter à reflux 8 heures puis évaporer sous vide le sulfure de carbone et verser le milieu réactionnel sur de la glace pilée. Extraire à deux reprises à l'heptane, sécher, évaporer à sec puis purifier sur colonne de silice en utilisant comme éluant un mélange de cyclohexane et d'acétate d'éthyle (10 : 1 V/V), pour obtenir le produit attendu.
Rendement : 76 %

Le procédé décrit ci-dessus peut être utilisé et adapté selon des méthodes connues pour préparer la 2-bromo 1-(2,4-dichlorophényl) propan-1-one ***(Composé 1))***.

De la même manière, a été préparé la 2-bromo 1-(2-chloro-4-iodo phényl) propan-1-one ***(Composé 15)*** en utilisant comme produit de départ le 1-chloro-3-iodobenzène au lieu du 1,3-dibromobenzène. La 2-bromo 1-(4-bromo-2-chlorophényl) propan-1-one ***(Composé 16)*** et la 2-bromo 1-(2-bromo-4-chloro phényl) propan-1-one ***(Composé 17)*** ont aussi été préparés selon le même procédé.

Les bromocétones de formule (II) ci-après ont été préparées en utilisant le procédé décrit ci-dessus en utilisant comme produit de départ les halogénures d'acyle et les dérivés aromatiques adéquats.
- **Composé 18** :: 2-bromo-1-(2,4,5-trichlorophényl) propan-1-one
- **Composé 19** :: 2-bromo-1-(2,3,4-trichlorophényl) propan-1-one
- **Composé 20** :: 2-bromo-1-(2-méthoxy-4-méthylphényl) propan-1-one
- **Composé 21** :: 2-bromo-1-(2-méthyl-4-méthoxyphényl) propan-1-one
- **Composé 22** :: 2-bromo-1-(2,5-dichloro-4-méthoxyphényl) propan-1-one
- **Composé 23** :: 2-bromo-1-(2-méthyl-4-méthoxy-5-chloro) propan-1-one
- **Composé 24** :: 2-bromo-1-(2,6-difluoro-4-méthoxyphényl) propan-1-one
- **Composé 25** :: 2-bromo-1-(2-chloro-4-méthoxyphényl) propan-1-one

### PREPARATION V

### 2-bromo- 1-(2,6-dichloropyrid-3-yl) propan-1-one(Composé 26)

### Etape A

Couler à -70°C dans une solution de 5,2 g de (2,6-dichloropyrid-3-yl) carbaldéhyde dans 50 ml d'éther éthylique, 50 ml d'une solution 3 M de bromure d'éthyl magnésium dans l'éther éthylique. Laisser la température monter à - 20°C puis hydrolyser. Extraire à l'aide d'éther éthylique puis purifier sur colonne de silice en utilisant comme éluant un mélange de chlorure de méthylène et de méthanol (99 : 1 V/V).

On obtient ainsi le 1-(2,6-dichloropyrid-3-yl) propan-1-ol.
Huile.
Rendement : 75 %

### Etape B

A une solution de 4,77 g de 1-(2,6-dichloropyrid-3-yl) propan-1-ol dans 200 ml de chlorure de méthylène, additionner 20 g de dioxyde de manganèse activé. Chauffer pendant 8 heures à reflux, filtrer à chaud et évaporer le filtrat à sec.

Purifier sur colonne de silice en utilisant comme éluant un mélange de cyclohexane et d'acétate d'éthyle (4 : 1 V/V), pour obtenir la 1-(2,6-dichloro pyrid-3-yl) propan-1-one.
Huile.
Rendement : 65 %

### Etape C

Procéder ensuite comme il est indiqué dans la Préparation I, en utilisant comme cétone le composé obtenu à l'étape précédente, pour obtenir la 2-bromo 1-(2,6-dichloropyrid-3-yl) propan-1-one. Huile.

### PREPARATION VI

### 2-bromo-1-(2,4-diméthylpyrid-5-yl) propan-1-one(Composé 27)

### Etape A

Solubiliser 23 g de 2-chloro-3-cyano-4,6-diméthylpyridine dans 185 ml d'acide chlorhydrique concentré et 240 ml d'eau. Ajouter 25,4 g d'étain métallique et porter à 100°C. Suivre la réaction par chromatographie sur couche mince (CCM). La réaction terminée, laisser revenir à température ambiante, filtrer et neutraliser le filtrat à l'aide d'une solution d'hydroxyde de sodium. Extraire au chlorure de méthylène, laver les phases organiques réunies avec une solution aqueuse saturée en bicarbonate de sodium et sécher sur sulfate de magnésium anhydre. Concentrer pour obtenir la 5-cyano-2,4-diméthylpyridine sous forme de précipité jaune. Rendement : 54 %

### Etape B

Mettre en suspension 8,91 g du composé obtenu à l'étape précédente dans 100 ml d'éther éthylique anhydre, et refroidir à -4°C. Additionner 25 ml d'une solution 3 M de bromure d'éthyl magnésium dans l'éther éthylique et remonter à température ambiante. Hydrolyser avec une solution aqueuse de chlorure d'ammonium, extraire à l'éther éthylique, évaporer et purifier le résidu sur colonne de silice en utilisant comme éluant le chlorure de méthylène. Evaporer à sec l'éluat pour obtenir la 1-(2,4-diméthylpyrid-5-yl) propan-1-one sous forme de poudre blanche. Rendement : 43 %

### Etape C

Procéder comme il est décrit dans la Préparation II en utilisant comme cétone la cétone obtenue à l'étape précédente pour obtenir la 2-bromo 1 -(2,4-diméthylpyrid-5-yl) propan-1-one.
Huile.
Rendement : 80 %

### PREPARATION DES COMPOSES DE FORMULE VII

### PREPARATION VII

### N-(α-cyclopropylbenzyl) N-propylamine(Composé 28)

A 10 g de cyclopropylphényl cétone dans 60 ml de toluène anhydre, ajouter du tamis moléculaire 4 Å et 100 mg d'acide paratoluène sulfonique, puis 6 g de propylamine. La formation d'imine est suivie par dosage en chromatographie phase gazeuse. Au bout de six jours de chauffage à 55°C, refroidir le mélange réactionnel, filtrer le tamis moléculaire et évaporer à sec sous vide. Reprendre le résidu dans 100 ml d'éthanol anhydre. Refroidir à 0°C, ajouter par petites portions, 2,65 g de borohydrure de sodium. Après une nuit d'agitation à température ambiante, évaporer à sec sous vide, reprendre dans de l'eau, hydrolyser avec l'acide chlorhydrique N pour amener le pH à 2, laver avec de l'acétate d'éthyle. Amener à pH 9 par addition d'hydroxyde de sodium 2 N puis extraire à plusieurs reprises au chlorure de méthylène.

La phase organique après séchage et évaporation donne une huile qui peut être utilisée directement.
Rendement : 60 %
Spectre de résonance magnétique nucléaire du proton (solvant CDCl₃) :
   0,15-1,70 ppm, m, 11H ; 2,40 ppm, t+d, 2H ; 2,80 ppm, d, 1H ; 7,30-7,40 ppm, m, 5H.

### Autres Composés(Composés 29 à 39)

Les amines indiquées dans le Tableau I sont obtenues selon le procédé décrit ci-dessus.

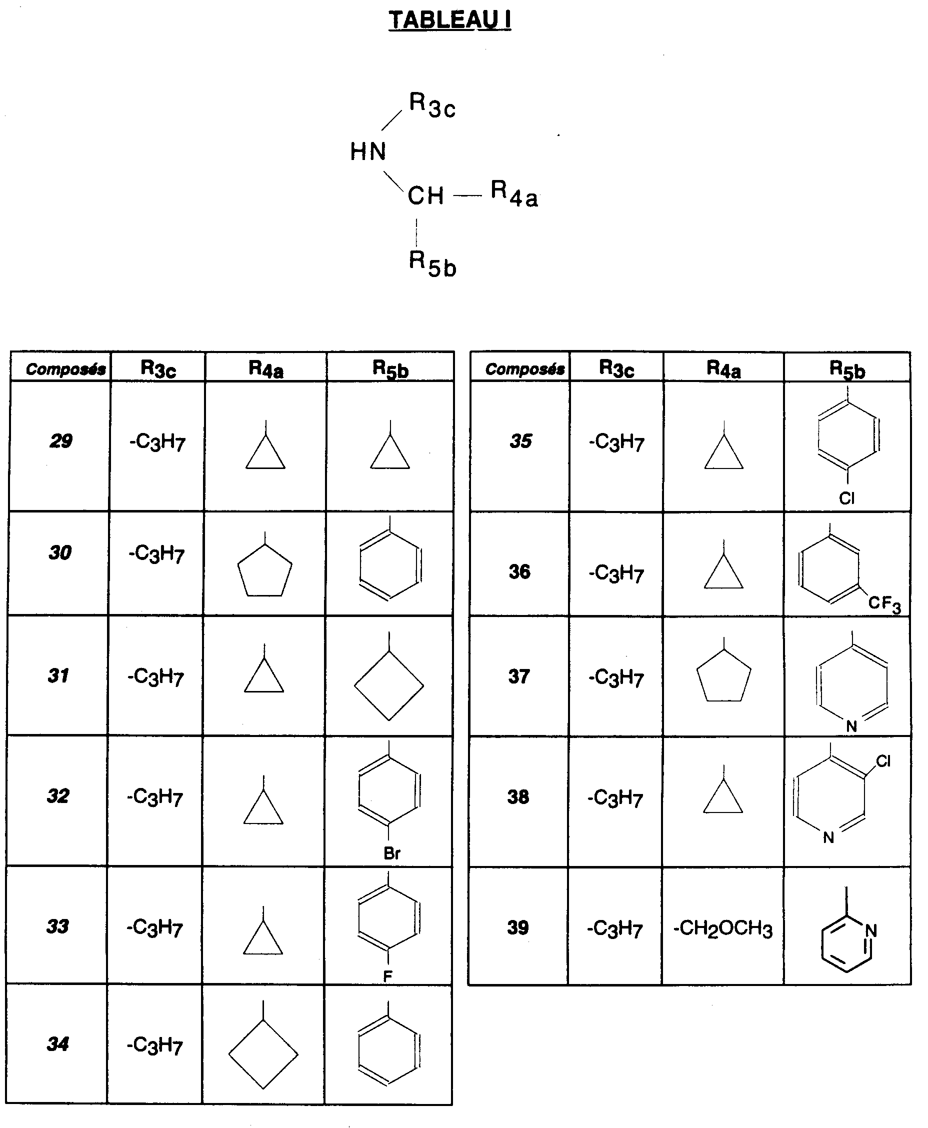

### PREPARATION VIII

### N-(1-cyclopropyl-2-méthoxyéthyl) N-propylamine(Composé 40)

### Etape A

A 1 g de magnésium couvert de 10 ml d'éther éthylique, additionner lentement en maintenant la température du mélange réactionnel entre 32°C et 35°C, une solution de 1,6 ml de bromure de cyclopropyle dans 30 ml d'éther éthylique.

Porter à reflux 1 heure puis, après retour à la température ambiante et filtration sur coton de verre, la solution de bromure de cyclopropyl magnésium obtenue est refroidie à 0°C et additionnée de 2 ml de méthoxyacétonitrile dilués dans 5 ml d'éther éthylique. Lors de l'addition, la température interne augmente jusqu'à 10°C.

Après 15 heures d'agitation à 20°C, le milieu réactionnel est refroidit à 0°C et versé lentement sur de l'eau glacée.

La suspension est agitée vigoureusement et additionnée de 12 ml d'acide sulfurique 30 %. Après décantation, la phase éthérée est lavée par une solution aqueuse de bicarbonate de sodium puis par de l'eau saturée en chlorure de sodium. Sécher sur sulfate de sodium anhydre et évaporer l'éther éthylique à froid, pour obtenir la cyclopropyl méthoxyméthyl cétone. Huile.
Rendement : 98 %

### Etape B

Sous agitation à 0°C, additionner lentement à une solution contenant 1,7 g de cétone obtenue au stade précédent et 6,16 ml de N-propylamine dans 40 ml de chlorure de méthylène, 15 ml d'une solution molaire de tétrachlorure de titane dans le chlorure de méthylène.

Après agitation pendant 15 heures à température ambiante, le milieu réactionnel est partiellement concentré, dilué par 40 ml de méthanol, refroidit à 0°C et additionné par portions de 1,14 g de borohydrure de sodium. Après agitation pendant 24 heures à température ambiante, le méthanol est évaporé et le résidu est repris par l'eau et le chlorure de méthylène. Eliminer le précipité blanc formé par filtration, extraire au chlorure de méthylène, laver à l'eau saturée en chlorure de sodium, sécher sur sulfate de sodium anhydre et évaporer à sec pour obtenir la N-(1-cyclopropyl-2-méthoxyéthyl) N-propylamine.
Gomme.
Rendement : 74 %

Les amines décrites dans le TABLEAU II sont obtenues selon le procédé décrit ci-dessus selon la préparation VIII (étape A et B ou B).

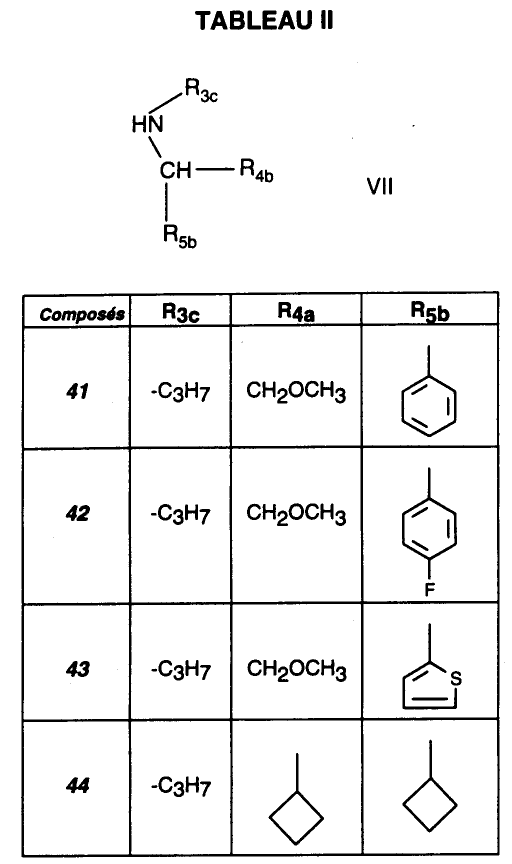

### PREPARATION IX

### N-(cyclopropylpyrid-4-yl méthyl) N-propylamine(Composé 45)

La N-(cyclopropylpyrid-4-yl méthyl) N-propylamine peut aussi être préparée de la façon suivante :

### Etape A

Dissoudre 1,07 g de pyrid-4-yl carbaldéhyde dans 10 ml d'éthanol absolu et ajouter lentement 0,8 g de N-propylamine. Après 30 minutes d'agitation, évaporer à sec pour obtenir 1,48 g d'huile.
Rendement : 99 %

### Etape B

Dissoudre l'imine obtenue à l'étape précédente dans 10 ml d'éther éthylique anhydre. Ajouter cette solution sous agitation à 0°C dans 30 ml d'une solution de cyclopropyl lithium (20 mmol) dans l'éther éthylique. Après deux heures d'agitation à température ambiante refroidir à 0°C et additionner goutte à goutte 3 ml de méthanol puis 10 ml d'une solution aqueuse de chlorure d'ammonium à 30 %. Extraire la phase éthérée par l'acide chlorhydrique N. Neutraliser la phase aqueuse acide par du bicarbonate de sodium puis extraire par l'acétate d'éthyle.

Sécher sur sulfate de sodium anhydre et évaporer à sec pour obtenir une huile incolore.
Rendement : 80 %
Spectre de résonance magnétique nucléaire du proton (solvant CDCl₃) :
   0,28-0,76 ppm, m, 4H ; 0,95 ppm, t, 3H ; 1,48 ppm, m, 2H ; 2,31-2,49 ppm, m, 2H ; 2,78 ppm, d, 1H ; 7,35 ppm, dd, 2H ; 8,54 ppm, dd, 2H.

### Autres composés(Composés 46 à 53)

Les amines indiquées dans le Tableau III, sont obtenues selon le procédé décrit ci-dessus.

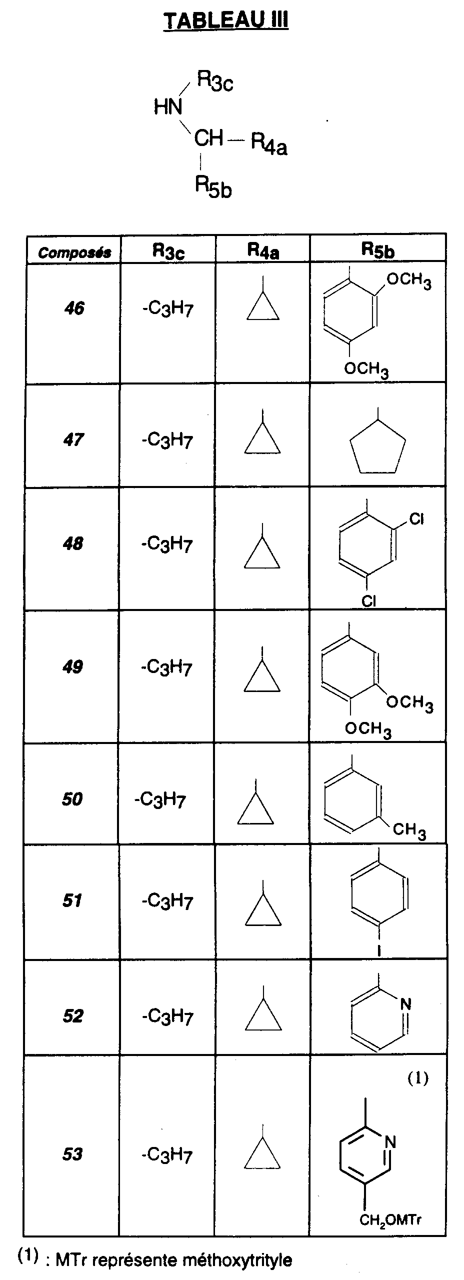

### PREPARATION X

### α-cyclopropyl benzylamine(Composé 54)

Mettre sous agitation à 50°C pendant 4 jours sous argon, 100 g de cyclopropyl phényl cétone dans 2000 ml de méthanol avec 500 g d'acétate d'ammonium préalablement séchés et 50 g de cyanoborohydrure de sodium en présence de tamis moléculaire 4 Å. Après refroidissement, filtrer le tamis moléculaire puis additionner de l'acide chlorhydrique pour amener le pH à 2. Evaporer le méthanol à sec sous vide et reprendre le résidu dans l'éther diéthylique. La phase aqueuse est lavée à l'éther éthylique puis alcalinisée par addition d'une solution d'hydroxyde de potassium concentrée pour que le pH soit supérieur à 10. Extraire à deux reprises par du chlorure de méthylène, laver avec une solution saturée en chlorure de sodium, sécher sur sulfate de magnésium anhydre puis concentrer sous vide pour obtenir l'α-cyclopropyl benzylamine.
Huile.
Rendement : 76 %

### PREPARATION XI

### 2-phényl-2-(N-propylamino) éthanol(Composé 55)

### Etape A

Additionner lentement sous agitation à 0°C à une suspension de 45,35 g de D,L-phénylglycine dans 450 ml de méthanol, 24 ml de chlorure de thionyle. Après 1 heure d'agitation à 25°C puis 30 minutes à 50°C, le milieu réactionnel est évaporé à sec. Le chlorhydrate ainsi obtenu est versé par portions sur 700 ml d'une solution aqueuse saturée en bicarbonate de sodium. La base formée est extraite par du chlorure de méthylène. L'extrait organique est lavé à l'eau saturée en chlorure de sodium, séché sur sulfate de sodium anhydre et évaporé à sec.

On obtient ainsi le phénylglycinate de méthyle.

### Etape B

Additionner sous agitation à 0°C à une solution de 44,5 g de l'ester obtenu à l'étape précédente dans 350 ml de diméthylformamide et de 50 ml de triéthylamine, 27 ml de chlorure de propionyle. Après agitation pendant 1 heure à température ambiante, verser le milieu réactionnel sur de la glace puis extraire à l'acétate d'éthyle. Laver l'extrait organique avec de l'eau saturée en chlorure de sodium, sécher sur sulfate de sodium anhydre et évaporer à sec pour obtenir le N-propionyl phénylglycinate de méthyle.

### Etape C

Ajouter lentement sous agitation à 20°C, à une suspension de 41 g d'hydrure d'aluminium et de lithium dans 500 ml de tetrahydrofurane anhydre, une solution de 60 g du composé obtenu à l'étape précédente dans 250 ml de tetrahydrofurane. Après 6 heures à reflux, refroidir le milieu réactionnel à 0°C et additionner sous agitation 200 ml d'hydroxyde de sodium à 15 %. Filtrer, concentrer le filtrat sous pression réduite, additionner d'eau et extraire par le chlorure de méthylène.

Mettre ensuite en suspension dans 500 ml d'acide chlorhydrique 2 N le précipité contenu dans le filtre puis, filtrer sur célite. Laver le filtrat acide par l'éther éthylique puis alcaliniser par l'hydroxyde de sodium à 30 %. Extraire le précipité formé par le chlorure de méthylène.

Réunir les extraits organiques, laver à l'eau saturée en chlorure de sodium, sécher sur sulfate de sodium anhydre et évaporer à sec pour obtenir le 2-phényl 2-(N-propylamino) éthanol.
Huile.
Rendement : 91 %
Spectre de résonance magnétique nucléaire du proton (solvant CDCl₃) :
   0,88 ppm, t, 3H ; 1,44 ppm, m, 2H ; 2,46 ppm, m, 2H ; 2,70 ppm, s, 1H ; 3,52-3,74 ppm, m, 3H ; 7,30 ppm, s, 5H.

En procédant selon la préparation XI ci-dessus et en utilisant la (R) phénylglycine et la (S) phénylglycine on obtient respectivement la
(R)-2-phényl-2-(N-propylamino)éthanol **(Composé 56)** et le
(S)-2-phényl-2-(N-propylamino)éthanol **(Composé 57)**.

### PREPARATION XII

### N-[cyclopropyl (2-tert-butyldiméthylsilyloxyméthylpyrid-5-yl)méthyl] N-propylamine (Composé 58)

### Etape A

Dissoudre 17 g de (2-*tert*-butyldiméthylsilyloxyméthylpyrid-5-yl) carboxylate de méthyle dans 40 ml d'éther éthylique. Additionner cette solution lentement à 0°C et sous argon, à une solution de 1,62 g d'hydrure de lithium et d'aluminium dans 240 ml d'éther éthylique. Laisser pendant 2 heures sous agitation à la même température. Hydrolyser ensuite en ajoutant 1,6 ml d'eau, puis 1,6 ml d'hydroxyde de sodium à 15 % et 4,8 ml d'eau. Filtrer et diluer le filtrat dans l'acétate d'éthyle. Laver la phase organique à l'eau. Sécher sur sulfate de sodium anhydre. Purifier sur colonne de silice en utilisant comme éluant un mélange de chlorure de méthylène et de méthanol (97 : 3 V/V).

On obtient ainsi le (2-*tert*-butyldiméthylsilyloxyméthylpyrid-5-yl) méthanol.
Huile.
Rendement : 53 %

### Etape B

Dissoudre 8,1 g du composé obtenu à l'étape précédente dans 250 ml de chlorure de méthylène, puis ajouter par portions, 8 g de dioxyde de manganèse activé. Chauffer à 40°C environ 4 heures puis purifier sur colonne de silice en utilisant comme éluant un mélange de chlorure de méthylène et de méthanol (99 : 1 V/V puis 97 : 3 V/V).

On obtient ainsi le (2-*tert*-butyldiméthylsilyloxyméthylpyrid-5-yl) carbaldéhyde.
Huile.
Rendement : 99 %
Etape C

Ajouter à 150 ml d'éther éthylique 70 ml d'une solution 0,57 N de cyclopropyl lithium et refroidir à -70°C. Additionner à la même température 8,4 g de l'aldéhyde obtenue à l'Etape B en solution dans l'éther éthylique. Laisser pendant 3 heures sous agitation puis additionner à -70°C 3,6 ml de méthanol, puis de l'eau.

Extraire à l'acétate d'éthyle puis purifier sur colonne de silice en utilisant comme éluant un mélange de chlorure de méthylène et de méthanol (97 : 3 V/V).

On obtient ainsi le cyclopropyl (2-*tert*-butyldiméthylsilyloxyméthyl pyrid-5-yl) méthanol.
Rendement : 55 %

### Etape D

Dissoudre 5,44 g du composé obtenu à l'Etape C dans 170 ml de chlorure de méthylène et additionner par portions, 14 g de dioxyde de manganèse activé. Porter à reflux pendant 4 heures. Purifier sur colonne de silice en utilisant comme éluant un mélange de chlorure de méthylène et de méthanol (99 : 1 V/V) pour obtenir la cyclopropyl (2-*tert*-butyldiméthylsilyloxyméthyl pyrid-5-yl) cétone.
Huile
Rendement : 88 %
Spectre de résonance magnétique nucléaire du proton (solvant CDCl₃) :
   0,03 ppm, s, 6H ; 0,85 ppm, s, 9H ; 0,80-1,20 ppm, m, 4H ; 2,55 ppm, m, 1H ; 4,83 ppm, s, 2H ; 7,56 ppm, d, 1H ; 8,23 ppm, dd, 1H ; 9,08 ppm, d, 1H.

### Etape E

Dissoudre 4,76 g de la cétone obtenue à l'étape précédente dans 100 ml de méthanol. Additionner à température ambiante 12,6 g d'acétate d'ammonium, 1,83 g de cyanoborohydrure de sodium et du tamis moléculaire 4 Å. Chauffer à 55°C pendant 48 heures puis éliminer par filtration le tamis moléculaire, laver avec du chlorure de méthylène et évaporer à sec. Reprendre à l'eau et extraire par le chlorure de méthylène.

Purifier sur colonne de silice en utilisant comme éluant un mélange de chlorure de méthylène et de méthanol (96 : 4 V/V) pour obtenir la cyclopropyl [2-(*tert*-butyl diméthylsilyloxyméthyl) pyrid-5-yl] méthylamine. Huile
Rendement : 54 %

### Etape F

Introduire 0,93 g de l'amine obtenue à l'étape précédente dans 40 ml de toluène contenant du tamis moléculaire (4 Å). Additionner 0,9 ml de propionaldéhyde. Chauffer jusqu'à 37°C et laisser revenir à température ambiante. Abandonner sous agitation pendant 2 heures, filtrer sous argon et laver avec du chlorure de méthylène. Evaporer les solvants et reprendre le résidu par du méthanol.

Ajouter 0,15 g de borohydrure de sodium à 0°C et laisser sous agitation pendant environ 1 heure et 30 minutes. Ajouter toujours à 0°C quelques gouttes d'eau et évaporer partiellement le solvant. Reprendre avec le chlorure de méthylène, laver à l'eau et sécher la phase organique sur sulfate de sodium anhydre.

Purifier sur colonne de silice en éluant avec un mélange de chlorure de méthylène et de méthanol (97 : 3 V/V).
Huile
Rendement : 91 %
Spectre de résonance magnétique nucléaire du proton (solvant CDCl₃) : 0,07 ppm, s, 6H ; 0,10-1,05 ppm, m, 17H ; 1,05-1,80 ppm, m, 2H ; 2,20-2,40 ppm, m, 2H ; 2,77 ppm d, 1H ; 4,78 ppm, s, 2H ; 7,42 ppm, d, 1H ; 7,69 ppm, dd, 1H ; 8,37 ppm, d, 1H.

### PREPARATION XII(BIS)

### N-[cyclopropyl-3-p-anisyldiphénylméthyloxyméthylpyrid-6-yl)méthyl] N-propylamine (Composé 53)

### Etape A

Dissoudre 22 g du (2-*tert*-butyldiméthylsilyloxyméthylpyrid-5-yl) méthanol dans 100 ml de pyridine anhydre. Additionner à 0°C sous argon 32 g de -p-anisylchlorodiphénylméthane. Laisser le mélange réactionnel pendant 3 heures sous agitation à température ambiante. Evaporer à sec, reprendre dans l'eau puis extraire à l'acétate d'éthyle. Sécher sur sulfate de sodium anhydre. Purifier sur colonne de silice en utilisant comme éluant du cyclohexane et de l'acétate d'éthyle (9 : 1 v/v).
On obtient ainsi le dérivé tritylé correspondant.
Rendement : 95 %.

### Etape B

Dissoudre 50 g du composé obtenu précédemment dans 800 ml de tétrahydrofurane. Additionner à température ambiante, goutte à goutte, 100 ml d'une solution de fluorure de tétrabutylammonium. Après 16 heures d'agitation à température ambiante, évaporer le solvant. Reprendre le résidu dans le chlorure de méthylène. Laver la phase organique à l'eau. Sécher sur sulfate de sodium puis évaporer à sec. Purifier sur colonne de silice en utilisant comme éluant du chlorure de méthylène et du méthanol (99 : 1 v/v puis 92 : 8 v/v). On obtient ainsi le[3-(p-anisyldiphénylméthyloxyméthyl)pyrid-6-yl)]méthanol.
Rendement : 85 %.

### Etape C

Dissoudre 27,8 g du composé précédent dans 500 ml de chlorure de méthylène. Additionner par portions 24 g de dioxyde de manganèse activé. Chauffer 8 heures à 50 °C. Purifier sur colonne de silice en utilisant comme éluant un mélange de chlorure de méthylène et de méthanol (92 : 8 v/v). On obtient ainsi le 3-(p-anisyldiphénylméthyloxyméthylpyrid-6-yl)carbaldéhyde.
Rendement : 70 %.
Ce composé conduit selon la préparation IX étapes A et B à l'amine attendue.

### PREPARATION XIII

### N-[α-cyclopropyl-4-(tert-butyldiméthylsilyloxyéthyl) benzyl] N-propylamine (Composé 59)

### Etape A

Introduire 5 g de 2-(4-cyanophényl) éthanol dans 50 ml de diméthylformamide. Ajouter 2,54 g d'imidazole, 5,63 g de chlorure de *tert*-butyldiméthylsilyle et une pointe de spatule de diméthylaminopyridine. Laisser 2 heures à température ambiante. Additionner le milieu réactionnel dans un mélange eau - glace. Extraire à l'acétate d'éthyle et sécher sur sulfate de sodium anhydre. Purifier par chromatographie sur colonne de silice en utilisant comme éluant un mélange de cyclohexane et d'acétate d'éthyle (10 : 1 V/V) pour obtenir le 1-cyano 4-(*tert*-butyldiméthylsilyloxyéthyl)-benzène.

### Etape B

Dissoudre 10 g du composé obtenu à l'Etape A dans 200 ml de tétrahydrofurane et refroidir entre -60°C et -70°C. Additionner à 0°C 90 ml d'une solution 0,57 M de cyclopropyllithium dans l'éther éthylique, et maintenir sous agitation pendant 2 heures. Hydrolyser à -60°C par addition de 10 ml de méthanol et de 10 ml d'une solution aqueuse de sulfate d'ammonium à 35 %. Extraire ensuite par l'acétate d'éthyle et sécher sur sulfate de sodium anhydre. Purifier par chromatographie sur colonne de silice en éluant par un gradient d'acétate d'éthyle dans le cyclohexane (2,5 à 20 %).

On obtient ainsi le cyclopropyl (4-*tert*-butyldiméthylsilyloxyéthyl phényl) cétone.
Rendement : 77,4 %
Spectre de résonance magnétique nucléaire du proton (solvant CDCl₃) :
   0,04 ppm, s, 6H ; 0,84 ppm, s, 9H ; 0,90-1,10 ppm, m, 2H ; 1,15-1,30 ppm, m, 2H ; 2,60-2,70 ppm, m, 1H ; 2,85 ppm, t, 2H ; 3,82 ppm, t, 2H ; 7,29 ppm, d, 2H ; 7,93 ppm, d, 2H.

### Etape C

Procéder comme il est indiqué dans la Préparation VIII, Etape B en utilisant comme cétone, le composé obtenu à l'étape précédente pour obtenir la N-[α-cyclopropyl 4-(*tert*-butyldiméthylsilyloxyéthyl) benzyl] N-propylamine.
Rendement : 87 %
Spectre de résonance magnétique nucléaire du proton (solvant CDCl₃) :
   0,05 ppm, s, 6H ; 0,20-1,20 ppm, m, 17H ; 1,20-1,60 ppm, m, 2H ; 2,40 ppm, t, 2H ; 2,79 ppm, m, 3H ; 3,79 ppm, t, 2H ; 7,10-7,30 ppm, m, 4H.

### PREPARATION XIV

### N-[cyclopropyl(2-méthylpyrid-4-yl) méthyl] N-propylamine(Composé 60)

### Etape A

A 7,3 g de 4-cyano-2-méthyl pyridine dans 100 ml de tetrahydrofurane anhydre, ajouter à -65°C 130 ml d'une solution de cyclopropyl lithium 0,7 M dans l'éther diéthylique. Après 4 heures d'agitation ajouter du méthanol et une solution de sulfate d'ammonium (6,3 g dans 20 ml d'eau). Après extraction à l'éther éthylique, laver la phase organique à l'eau, sécher et évaporer sous vide. Le résidu est purifié sur colonne de silice en utilisant comme éluant un mélange de chlorure de méthylène et de méthanol (98 : 2 V/V).

La cyclopropyl (2-méthylpyrid-4-yl) cétone est ainsi obtenue.
Rendement : 59 %
Spectre de résonance magnétique nucléaire du proton (solvant CDCl₃) :
   0,94-1,38 ppm, m, 4H ; 2,62 ppm, s, 3H ; 2,52-2,65 ppm, m, 1H ; 7,57 ppm, m, 2H ; 8,65 ppm, d, 1H.

### Etape B

Procéder selon la méthode décrite dans la préparation VII, en utilisant la cétone obtenue à l'étape précédente pour obtenir la N-[cyclopropyl (2-méthylpyrid-4-yl) méthyl] N-propylamine.

### PREPARATION XV

### N-[α-(méthoxyéthyl) benzyl] N-propylamine(Composé 61)

### Etape A

Agiter à environ 20°C une solution de 7,6 ml d'éther méthylique de chlorométhyle dans 20 ml de tétrachlorure de carbone avec 121 mg de chlorure de zinc anhydre et additionner en 40 minutes 11,5 ml de styrène en solution dans 20 ml de tétrachlorure de carbone. Agiter le milieu réactionnel pendant 1 heure et 30 minutes à température ambiante, puis additionner 10 ml d'eau et 10 ml d'hydroxyde de sodium 1 N. Laver la phase organique à l'eau saturée en chlorure de sodium, sécher sur sulfate de sodium anhydre et évaporer à sec.

On obtient ainsi l'α-chloro α-(méthoxyéthyl) toluène.
Rendement : 93 %

### Etape B

Mettre sous agitation pendant 15 heures à 60°C une solution contenant 15,5 g du composé obtenu à l'étape A dans 30 ml de diméthylformamide avec 34,4 ml de N-propylamine et 14 ml de triéthylamine, puis ajuster la température du milieu réactionnel à 80°C et laisser sous agitation pendant 3 heures.

Evaporer ensuite l'excès de N-propylamine sous pression réduite et diluer le milieu réactionnel dans 400 ml d'acétate d'éthyle. Laver la phase organique à l'eau puis épuiser avec trois fois 150 ml d'acide chlorhydrique 2 N. Les phases aqueuses acides sont alcalinisées à 0°C par l'hydroxyde de sodium à 30 %. Après extraction au chlorure de méthylène, lavage de l'extrait organique à l'eau, à l'eau saturée en chlorure de sodium et évaporation à sec, on obtient la N-[α-(méthoxyéthyl) benzyl] N-propylamine.
Huile.
Rendement : 50 %
Spectre de résonance magnétique nucléaire du proton (solvant CDCl₃) :
   0,82 ppm, t, 3H ; 1,30-1,60 ppm, m, 2H ; 1,78-2,05 ppm, m, 2H ; 2,36 ppm, m, 2H ; 3,20-3,35 ppm, m+s, 5H ; 3,71 ppm, t, 1H ; 7,26 ppm, s, 5H.

### PREPARATION XVI

### N-[α-(méthylthiométhyl) benzyl] N-propylamine(Composé 62)

### Etape A

Agiter une solution de 21,4 g de bromoacétophénone dans 90 ml d'éthanol à 0°C puis additionner lentement une solution de 5 g de méthiolate de sodium dans 25 ml d'eau. Agiter une heure à 0°C puis 2 heures à température ambiante et ensuite verser le milieu réactionnel sur 500 ml d'eau glacée et extraire par 2 fois 200 ml d'éther éthylique. Réunir les phases éthérées et laver à l'eau saturée en chlorure de sodium, sécher sur sulfate de sodium anhydre puis évaporer à sec. Le résidu est distillé pour obtenir la 2-(méthylthio) acétophénone.
Rendement : 74 %

### Etape B

Procéder comme il est décrit dans la préparation VII en utilisant comme cétone le composé obtenu à l'étape précédente. On obtient ainsi la N-[(α-méthylthiométhyl)benzyl] N-propylamine.
Huile.
Rendement : 58 %
Spectre de résonance magnétique nucléaire du proton (solvant CDCl₃) :
   0,87 ppm, m, 3H ; 1,40 ppm, m, 2H ; 2,05 ppm, s, 3H ; 2,12 ppm, m, 2H ; 2,70 ppm, m, 2H ; 3,72 ppm, m, 1H ; 7,26-7,37 ppm, m, 5H.

En procédant selon la préparation XVI ci-dessus on prépare également la N-[α-(méthylthiométhyl)-4-fluorobenzyl] N-propylamine **(Composé 63)**.

### PREPARATION DES COMPOSES DE FORMULE IIIa ET IIIb

### PREPARATION XVII

### N-(α-cyclopropyl-4-bromobenzyl) N-propyl thiourée(Composé 64)

### Etape A

### N'-benzoyl N-(α-cyclopropyl-4-bromobenzyl) N-propyl thiourée

Mettre en suspension 10,15 g de thiocyanate d'ammonium dans 60 ml d'acétone et refroidir à 0°C. Ajouter lentement 14,2 ml de chlorure de benzoyle en solution dans 15 ml d'acétone. Laisser sous agitation 15 minutes puis additionner 29 g de N-(α-cyclopropyl-4-bromobenzyl) N-propylamine ***(Composé 32)***. Laisser remonter à température ambiante et continuer l'agitation pendant 2 heures. Rajouter alors 20 ml d'eau, puis concentrer au maximum. Reprendre le résidu par un mélange d'eau et de chlorure de méthylène. Sécher sur sulfate de sodium anhydre puis concentrer et laisser reposer pour obtenir le produit attendu sous forme de cristaux jaunes.
Rendement : 87 %

### Etape B

A 38 g du composé obtenu à l'étape précédente en solution dans 550 ml de méthanol, ajouter 220 ml d'hydroxyde de sodium 1 N et porter à reflux pendant 24 heures. Evaporer le méthanol et extraire au chlorure de méthylène. Sécher la phase organique sur sulfate de sodium anhydre et évaporer sous vide. Purifier le résidu par chromatographie sur colonne de silice en utilisant comme éluant un mélange de cyclohexane et d'acétate d'éthyle (7 : 1 V/V).

On obtient la N-(α-cyclopropyl-4-bromobenzyl) N-propylthiourée sous forme de poudre blanche.
Spectre de résonance magnétique nucléaire du proton (solvant CDCl₃) :
   0,4-1,0 ppm, m, 7H ; 1,0-1,4 ppm, m, 1H ; 1,6-1,8 ppm, m, 2H ; 2,9-3,2 ppm, m, 3H ; 5,8 ppm, m, 2H ; 7,2-7,5 ppm, m, 4H.

### Autres composés(Composés 65 à 95)

Les dérivés de thiourée indiqués dans le Tableau IV sont obtenus selon le procédé décrit pour la N-(α-cyclopropyl-4-bromobenzyl) N-propylthiourée en utilisant les amines adéquates, dont la préparation est indiquée précédemment (Préparation des composés de formule VII), ou en utilisant des amines qui sont disponibles dans le commerce.

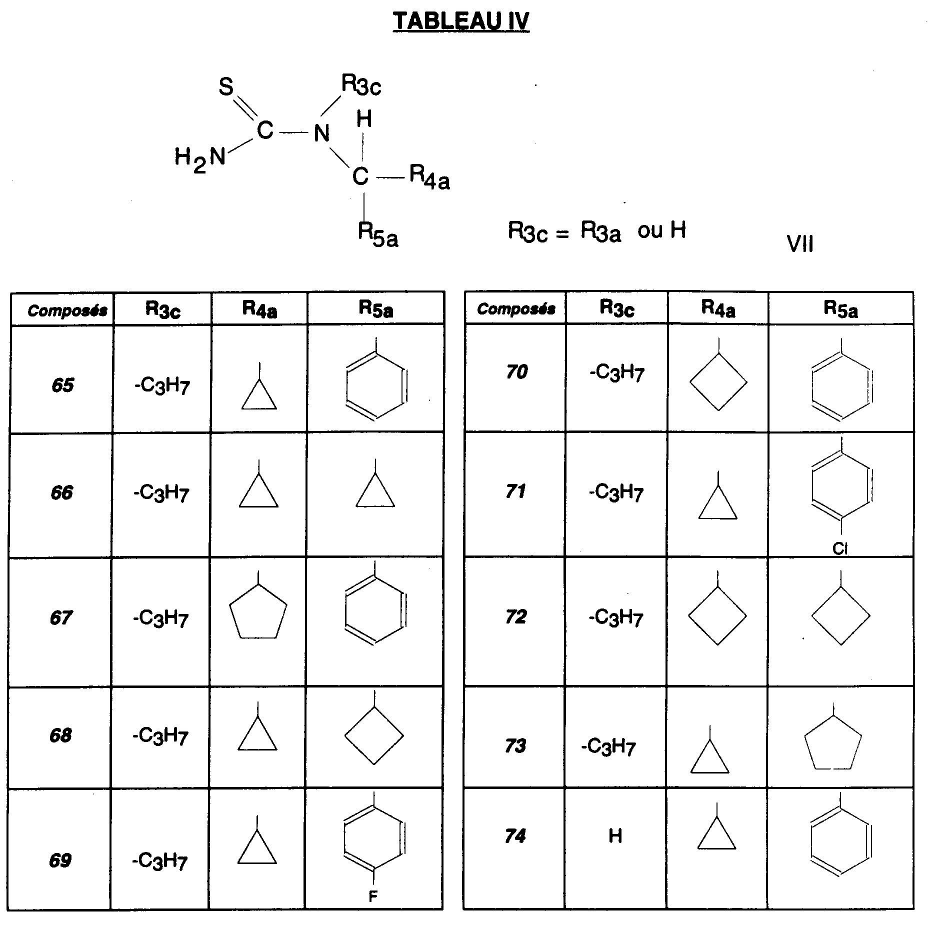

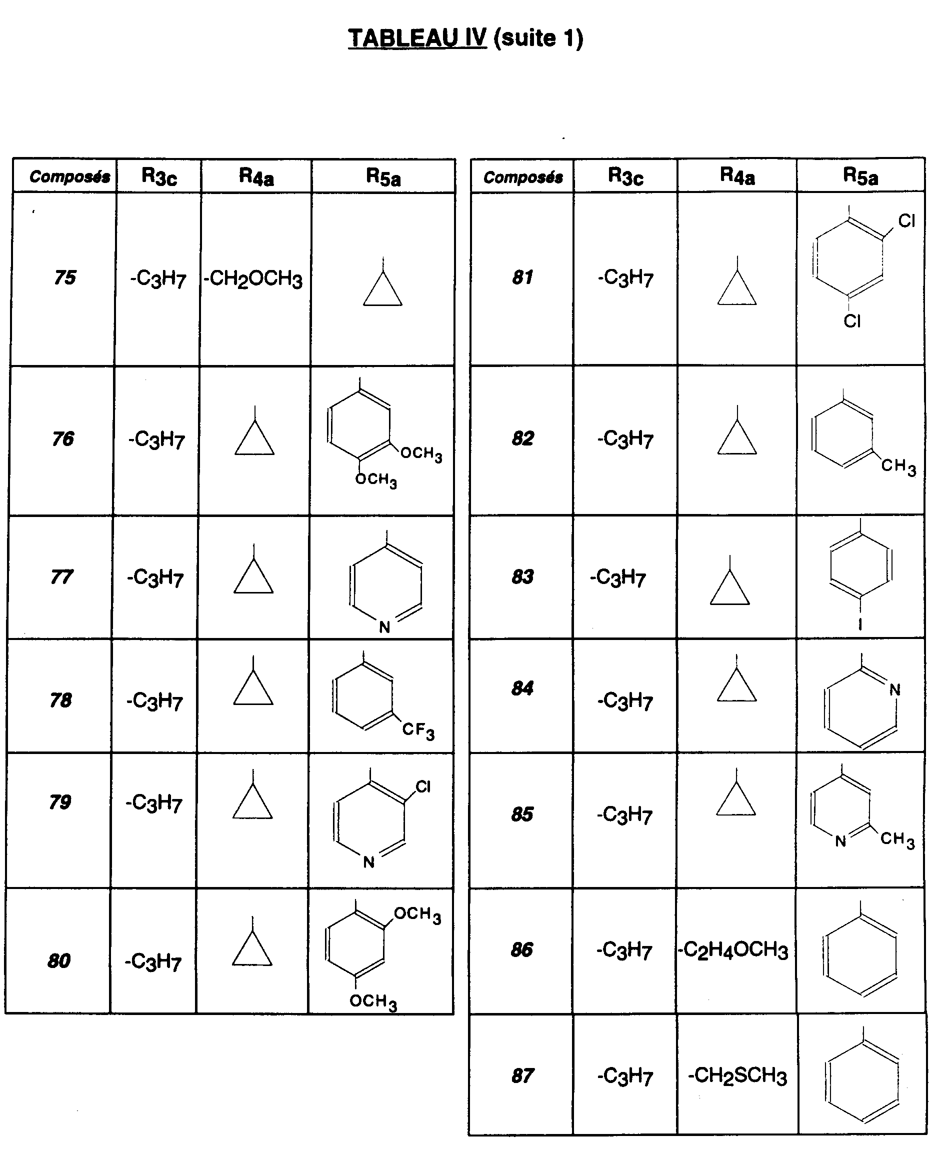

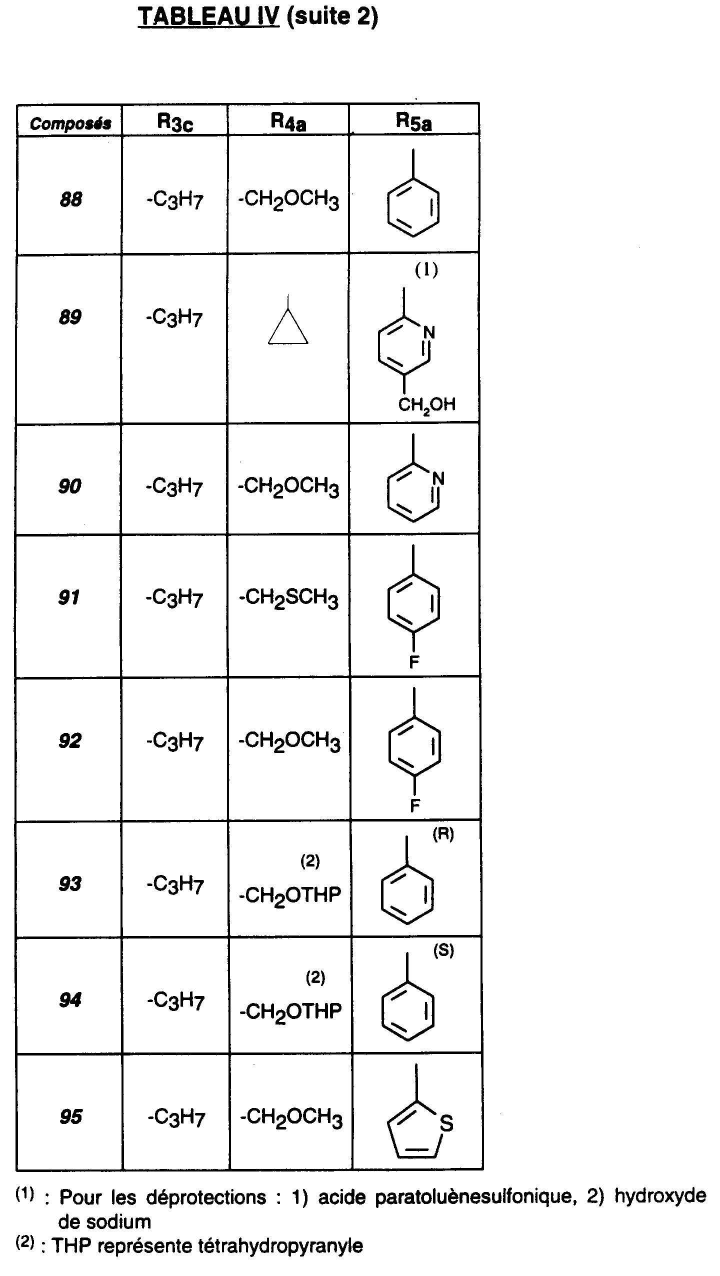

### PREPARATION XVIII

### N-[α(-tetrahydropyran-2-yloxyméthyl) benzyl] N-propyl thiourée (Composé 96)

### Etape A

### N'-benzoyl N-(α-hydroxyméthyl benzyl) N-propyl thiourée

Procéder comme il est décrit dans la Préparation XVII, Etape A, en utilisant le 2-phényl 2-(N-propylamino) éthanol ***(Composé 55)*** pour obtenir le composé attendu sous forme de cristaux jaunes. Pont de fusion : 82°C Rendement : 75 %

### Etape B

### N'-benzoyl N-[α-(tetrahydropyran-2-yloxy méthyl) benzyl] N-propyl thiourée

Ajouter sous agitation à 0°C une solution contenant 59,13 g de la N'-benzoyl thiourée obtenue à l'étape précédente dans 800 ml de chlorure de méthylène et 78 ml de 2,3-dihydropyrane, 1,64 g d'acide paratoluène sulfonique. Laisser le milieu réactionnel sous agitation pendant 2 heures de 0°C à 30°C. Laver ensuite deux fois par 500 ml d'une solution de chlorure de sodium et de bicarbonate de sodium, puis par une solution aqueuse saturée en chlorure de sodium.

Evaporer à sec et utiliser le produit ainsi obtenu à l'étape suivante.

### Etape C

Ajouter à une solution de 73 g de N'-benzoylthiourée protégée obtenue à l'étape précédente dans 1 litre d'éthanol, 500 ml d'hydroxyde de sodium 1 N. Porter le milieu réactionnel à 80°C pendant 16 heures puis évaporer l'éthanol sous pression réduite. Additionner à la phase aqueuse alcaline ainsi obtenue de l'eau saturée en chlorure de sodium et extraire par du chlorure de méthylène. Laver à l'eau saturée en chlorure de sodium, sécher sur sulfate de sodium anhydre et évaporer à sec. Purifier le résidu par chromatographie sur colonne de silice en utilisant comme éluant un mélange de cyclohexane et d'acétate d'éthyle (7 : 1 V/V puis 2 : 1 V/V).

Répéter la N'-débenzoylation de la thiourée si nécessaire selon le procédé indiqué ci-dessus.

On obtient ainsi la N-[α-(tetrahydropyran-2-yloxy méthyl) benzyl] N-propyl thiourée sous forme d'huile jaune.
Rendement : 29 %

### PREPARATION XIX

### N-[α-cyclopropyl 4-(hydroxyéthyl) benzyl] N-propyl thiourée(Composé 97)

### Etape A

### N'-benzoyl N-[α-cyclopropyl 4-(tert-butyldiméthylsilyloxyéthyl) benzyl] N-propyl thiourée

Ce composé a été préparé selon le procédé décrit dans la Préparation XVII, Etape A, en utilisant comme amine la N-[α-cyclopropyl 4-(*tert*-butyldiméthylsilyloxyéthyl) benzyl] N-propylamine ***(Composé 59)***.

A la fin de la réaction, le milieu réactionnel est évaporé à sec, repris par l'eau et la phase organique est extraite par l'acétate d'éthyle. Pour la purification, il est procédé à une chromatographie sur silice en utilisant comme solvant un mélange de cyclohexane et d'acétate d'éthyle (7 : 1 V/V). Rendement : 75 %

### Etape B

Dissoudre 8,8 g du composé obtenu à l'étape précédente dans 120 ml de méthanol. Additionner 4,5 ml d'hydroxyde de sodium à 30 %, puis chauffer à reflux pendant 8 heures. Evaporer le méthanol, additionner de l'eau et extraire par du chlorure de méthylène. Purifier sur colonne de silice en éluant avec du chlorure de méthylène. On obtient ainsi les composés suivants :
- N-[α-cyclopropyl-4-(*tert*-butyldiméthylsilyloxyéthyl) benzyl] N-propyl thiourée : 10,3 %
- N-[α-cyclopropyl-4-(hydroxyéthyl) benzyl] N-propyl thiourée : 17,3 %
- N'-benzoyl N-[α-cyclopropyl-4-(*tert*-butyldiméthylsilyloxyéthyl) benzyl] N-propyl thiourée : 34,2 %
- N'-benzoyl N-[α-cyclopropyl-4-(hydroxyéthyl) benzyl] N-propyl thiourée : 36,9 %

Dissoudre 0,4 g de N-[α-cyclopropyl-4-(*tert*-butyldiméthyl silyloxyéthyl) benzyl] N-propyl thiourée dans 10 ml de tetrahydrofurane. Additionner 1 ml d'une solution 1 M de fluorure de tétrabutylammonium dans le tetrahydrofurane.

Laisser sous agitation environ 3 heures à température ambiante. Additionner d'eau saturée en chlorure de sodium et extraire au chlorure de méthylène. Sécher sur sulfate de sodium anhydre et évaporer à sec. Purifier par chromatographie sur colonne de silice en utilisant comme éluant un mélange de chlorure de méthylène et de méthanol (95 : 5 V/V), pour obtenir la N-[α-cyclopropyl 4-(hydroxyéthyl) benzyl] N-propyl thiourée.
Spectre de résonance magnétique nucléaire du proton (solvant CDCl₃) :
   0,5-0,9 ppm, m, 7H ; 1,10-1,40 ppm, m, 1H ; 1,40-1,70 ppm, m, 2H ; 2,81 ppm, t, 2H ; 2,8-3,4 ppm, m, 2H ; 3,80 ppm, t, 2H ; 5,70-6,1 ppm, m, 3H ; 7,00-7,50 ppm, dd, 4H.

### PREPARATION XX

### N-[cyclopropyl (2-hydroxypyrid-5-yl) méthyl] N-propyl thiourée (Composé 98)

### Etape A

### N'-benzoyl N-[cyclopropyl (2-hydroxypyrid-5-yl) méthyl ] N-propyl thiourée

Ce composé a été préparé à partir de 0,21 g de thiocyanate d'ammonium, 0,32 ml de chlorure de benzoyle et 0,72 g du ***Composé 58*****,** selon le procédé décrit dans la préparation XVII, Etape A. Rendement : 81 %

### Etape B

Procéder comme il est décrit dans la préparation XVII, Etape B en utilisant la N'-benzoyl thiourée obtenue à l'étape précédente, pour obtenir le produit attendu.
Rendement : 40 %
Spectre de résonance magnétique nucléaire du proton (solvant CDCl₃) : 0,3-1,3 ppm, m, 8H ; 1,40-1,90 ppm, m, 2H ; 2,8-3,4 ppm, m, 2H ; 4,70 ppm, s, 2H ; 5,8-6,3 ppm, m, 3H ; 7,22 ppm, dd, 1H ; 7,75 ppm, d, 1H ; 8,64 ppm, d, 1H.

### PREPARATION XXI

### N-(cyclopentylpyrid-4-ylméthyl) N-propyl thiourée(Composé 99)

### Etape A

### N'-benzoyl N-(cyclopentylpyrid-4-yl méthyl) N-propyl thiourée

Préparer ce composé à partir de la N-(cyclopentyl pyrid-4yl méthyl) N-propylamine ***(Composé 37)*** selon le procédé décrit dans la préparation XVII, Etape A, puis hydrolyser en milieu acide pour l'Etape B.

A 1,18 g du composé obtenu à l'étape A, ajouter 6 ml d'acide chlorhydrique à 32 %. Porter le milieu réactionnel pendant une heure à 80°C puis refroidir et rajouter de l'eau. Extraire au chlorure de méthylène et éliminer la phase organique. Alcaliniser la phase aqueuse avec du carbonate de sodium et extraire au chlorure de méthylène. Sécher la phase organique et évaporer sous vide. Purifier le résidu par chromatographie sur colonne de silice en utilisant comme éluant un mélange de chlorure de méthylène et de méthanol (98 : 2 V/V).
Huile.
Rendement : 98 %.

### EXEMPLES

### Exemple 1

### 4-(2,4-dichlorophényl)-5-méthyl 2-[N-(α-cyclopropyl-4-carboxy benzyl) N-propylamino] thiazole

### Etape A

### 4-(2,4-dichlorophényl)-5-méthyl-2-[N-(α-cyclopropyl-4-bromobenzyl) N-propyl amino] thiazole

Dissoudre 14 g de 2-bromo-1-(2,4-dichlorophényl) propan-1-one ***(Composé 1)***, 17,1 g de N-(α-cyclopropyl-4-bromo benzyl) N-propyl thiourée ***(Composé 64)*** et 7,6 ml de triéthylamine dans 200 ml d'éthanol et chauffer à 65°C. La réaction est suivie par CCM. A la fin de la réaction, éliminer l'éthanol, hydrolyser avec de l'eau et extraire avec du chlorure de méthylène. Laver et sécher les phases organiques. Purifier sur colonne de silice en utilisant comme éluant un mélange de cyclohexane et d'acétate d'éthyle (20 : 1 V/V).
Rendement : 92 %
Spectre de résonance magnétique nucléaire du proton (solvant CDCl₃) :
   0,3-1,0 ppm, m, 7H ; 1,2-1,8 ppm, m, 3H ; 2,15 ppm, s, 3H ; 3,20 ppm, m, 2H ; 4,67 ppm, d, 1H ; 7,1-7,7 ppm, m, 7H.

### Etape B

A 1,59 g du composé obtenu à l'étape précédente en solution dans 40 ml d'éther éthylique, ajouter goutte à goutte et à -70°C, 1,8 ml de *tert*-butyllithium 1,7 N dans le pentane. L'addition terminée, introduire à -70°C de l'anhydride carbonique en laissant progressivement remonter la température à l'ambiante. Après hydrolyse du milieu réactionnel à 0°C par du chlorure d'ammonium, extraire à l'acétate d'éthyle. La phase organique est lavée avec de l'eau puis avec une solution saturée en chlorure de sodium, séchée sur sulfate de sodium anhydre puis concentrée sous vide. Le résidu est purifié sur colonne de silice en utilisant comme éluant un mélange de chlorure de méthylène et de méthanol (85 : 15 V/V) pour obtenir le 4-(2,4-dichlorophényl) 5-méthyl 2-[N-(α-cyclopropyl 4-carboxy benzyl) N-propylamino] thiazole (cristaux blancs).
Rendement : 80 %
Point de fusion : 86°C

Le spectre de résonance magnétique nucléaire du proton est indiqué dans le Tableau V.

### Exemple 2

### 4-(2,4-dichlorophényl)-5-méthyl-2-{N-[α-cyclopropyl-4-(méthoxycarbonyl) benzyl] N-propylamino} thiazole

Ajouter à 390 mg du composé de l'Exemple 1 en solution dans 100 ml méthanol anhydre, 200 mg de chlorure de thionyle, puis, porter à 40 °C sous argon pendant 16 heures. Après évaporation sous vide, purifier le résidu sur colonne de silice en utilisant comme éluant un mélange de cyclohexane et d'acétate d'éthyle (10 : 1 V/V). On obtient ainsi le 4-(2,4-dichlorophényl) 5-méthyl 2-{N-[α-cyclopropyl 4-(méthoxy carbonyl) benzyl] N-propylamino} thiazole.
Rendement : 79 %
Point de fusion : Gomme

Le spectre de résonance magnétique nucléaire du proton est indiqué dans le Tableau V.

### Exemple 3

### 4-(2,4-dichlorophényl)-5-méthyl-2-{N-[α-cyclopropyl-4-(éthoxy carbonyl) benzyl] N-propylamino} thiazole

Ce composé a été préparé selon le prcédé décrit dans l'exemple 2 mais en utilisant 100 ml d'éthanol anhydre au lieu du méthanol. Point de fusion : Gomme

Le spectre de résonance magnétique nucléaire du proton est indiqué dans le Tableau V.

### Exemple 4

### 4-(2,4-dichlorophényl)-5-méthyl-2-{N-[α-cyclopropyl-4-(hydroxy méthyl) benzyl] N-propylamino} thiazole

A 403 mg du composé de l'Exemple 1 dans 15 ml de tetrahydrofurane anhydre, additionner à - 7°C 2 ml d'une solution 1 N d'hydrure de bore dans le tetrahydrofurane, puis, laisser sous agitation une nuit. Ajouter ensuite 10 ml d'eau et 2 ml de méthanol en présence de carbonate de potassium. Evaporer à sec sous vide le mélange réactionnel.

Reprendre dans 20 ml d'éther éthylique, laver à l'eau, et à l'eau saturée en chlorure de sodium. Sécher sur sulfate de sodium anhydre puis concentrer sous vide.

Purifier sur colonne de silice en utilisant comme éluant un mélange de cyclohexane et d'acétate d'éthyle (2 : 1 V/V) pour obtenir le produit attendu. Rendement : 94 % Point de fusion : Gomme

Le spectre de résonance magnétique nucléaire du proton est indiqué dans le Tableau V.

### Exemple 5

### 4-(2,4-dichlorophényl)-5-méthyl-2-{N-[α-cyclopropyl-4-(acétoxy méthyl) benzyl] N-propylamino} thiazole

A 930 g du composé de l'Exemple 4 en solution dans 50 ml de chlorure de méthylène, ajouter en présence de 0,3 ml de triéthylamine, 0,6 ml d'anhydride acétique et du 4-diméthylaminopyridine comme catalyseur. Après 1 heure sous agitation à température ambiante, rajouter un fort excès de méthanol puis évaporer sous vide le milieu réactionnel.

Le résidu est repris par l'eau saturée en chlorure de sodium, puis évaporé sous vide.

Purifier sur colonne de silice en utilisant comme éluant un mélange de cyclohexane et d'acétate d'éthyle (7 : 1 V/V) pour obtenir le produit attendu sous forme d'huile.

Le spectre de résonance magnétique nucléaire du proton est indiqué dans le Tableau V.

Le chlorhydrate de 4-(2,4-dichlorophényl) 5-méthyl 2-{N-[α-cyclopropyl 4-(acétoxy méthyl) benzyl] N-propylamino} thiazole est obtenu avec une solution d'acide chlorhydrique 0,1 N dans l'isopropanol (cristaux blancs). Point de fusion : 54°C

### Exemple 6

### 4-(2,4-dichlorophényl)-5-méthyl-2-{N-[α-cyclopropyl-4-(iodo méthyl) benzyl] N-propylamino} thiazole

A 1,36 g de triphénylphosphine dans 50 ml de chlorure de méthylène, ajouter à température ambiante 0,35 g d'imidazole, 1,32 g d'iode puis, 1,6 g du produit de l'Exemple 4.

Après 3 heures d'agitation à température ambiante, le milieu réactionnel est lavé avec de l'eau, séché sur sulfate de sodium anhydre puis concentré sous vide. Le résidu est repris par de l'éther éthylique. Le précipité d'oxyde de triphénylphosphine est éliminé par filtration.

Le filtrat est purifié sous forme de résidu huileux sur colonne de silice en utilisant comme éluant un mélange de cyclohexane et d'acétate d'éthyle (20 : 1 V/V), pour obtenir le produit attendu.
Rendement : 76 %
Point de fusion : Gomme

Le spectre de résonance magnétique nucléaire du proton est indiqué dans le Tableau V.

### Exemple 7

### 4-(2,4-dichlorophényl)-5-méthyl-2-{N-[α-cyclopropyl-4-(benzyloxy méthyl) benzyl] N-propylamino} thiazole

A 635 mg du composé de l'Exemple 4 en solution dans 80 ml de tetrahydrofurane, ajouter 43 mg d'hydrure de sodium puis 308 mg de bromure de benzyle et 665 mg d'iodure de tetrabutylammonium.

Après 48 heures à température ambiante, extraire au chlorure de méthylène, laver à l'eau saturée en chlorure de sodium, sécher la phase organique sur sulfate de sodium anhydre et évaporer sous vide.

Purifier sur colonne de silice en utilisant comme éluant un mélange d'acétate d'éthyle et de cyclohexane (1 : 20 V/V) pour obtenir le composé attendu.
Rendement : 99 %
Point de fusion : Gomme

Le spectre de résonance magnétique nucléaire du proton du 4-(2,4-dichlorophényl)-5-méthyl-2-{N-[α-cyclopropyl-4-(benzyloxyméthyl) benzyl] N-propyl amino} thiazole est indiqué dans le Tableau V.

### Exemple 8

### 4-(2,4-dichlorophényl)-5-méthyl-2-{N-[α-cyclopropyl-4-(1-hydroxy éth-1-yl) benzyl] N-propylamino} thiazole

A 1,8 g du dérivé bromé décrit à l'Etape A de l'Exemple 1 en solution dans 40 ml d'éther éthylique anhydre refroidi à -70°C, additionner goutte à goutte 4,7 ml d'une solution 1,5 M de *tert*-butyllithium dans le pentane, on laisse remonter la température à -50°C puis très lentement on ajoute à -70°C 0,4 ml d'acétaldéhyde.

Laisser une heure à -70°C puis laisser revenir la température à 0°C. Après hydrolyse avec une solution d'acide chlorhydrique 1 N, laver la phase éthérée à l'eau saturée en chlorure de sodium, sécher sur sulfate de sodium anhydre puis purifier sur colonne de silice en utilisant comme éluant un mélange de cyclohexane et d'acétate d'éthyle (3 : 1 V/V).

On obtient ainsi le produit attendu sous forme d'huile.
Rendement : 66 %

Le spectre de résonance magnétique nucléaire du proton est indiqué dans le Tableau V.

Le chlorhydrate de 4-(2,4-dichlorophényl)-5-méthyl-2-{N-[α-cyclopropyl-4-(1-hydroxyéth-1-yl) benzyl] N-propylamino} thiazole est obtenu par addition d'acide chlorhydrique en solution dans de l'isopropanol.
Point de fusion : 85°C

### Exemple 9

### 4-(2,4-dichlorophényl)-5-méthyl-2-{N-[α-cyclopropyl-4-(2-hydroxy propan-2-yl) benzyl] N-propylamino} thiazole

Ce composé a été obtenu selon le procédé décrit dans l'Exemple 8 mais en utilisant l'acétone au lieu de I'acétaldéhyde.
Point de fusion : Gomme

Le spectre de résonance magnétique nucléaire du proton du 4-(2,4-dichlorophényl)-5-méthyl-2-{N-[α-cyclopropyl-4-(2-hydroxypropan-2-yl) benzyl] N-propylamino} thiazole est indiqué dans le Tableau V.

### Exemple 10

### 4-(2,4-dichlorophényl)-5-méthyl-2-{N-[α-cyclopropyl-4-(1-hydroxy propan-1-yl) benzyl] N-propylamino} thiazole

Ce composé a été obtenu selon le procédé décrit dans l'Exemple 8 en utilisant comme aldéhyde le propionaldéhyde. Point de fusion : Gomme

Le spectre de résonance magnétique nucléaire du proton est indiqué dans le Tableau V.

### Exemple 11

### 4-(2,4-dichlorophényl)-5-méthyl-2-{N-[α-cyclopropyl-4-(sulfoxy méthyl) benzyl] N-propylamino} thiazole, sel de sodium

A 670 mg du composé de l'Exemple 4 en solution dans 7 ml de diméthylformamide anhydre, ajouter 0,14 ml de pyridine distillée puis, à 0°C et goutte à goutte 0,12 ml de chlorhydrine sulfurique dans 0,6 ml de chlorure de méthylène.

Après 3 heures d'agitation à température ambiante, le milieu réactionnel est hydrolysé puis extrait par du chlorure de méthylène, séché sur sulfate de sodium anhydre et évaporer à sec.

Au résidu repris par 5 ml de chlorure de méthylène, sont ajoutés 1,1 ml d'une solution de méthylate de sodium 1,32 M. Le pH est ramené à 1 par addition de résine carboxylique.

Après filtration, la phase organique donne un précipité par addition d'éther isopropylique.

On obtient ainsi le sel attendu.
Rendement : 60 %
Point de fusion : 100°C-104°C

Le spectre de résonance magnétique nucléaire du proton est indiqué dans le Tableau V.

### Exemple 12

### 4-(2,4-dichlorophényl)-5-méthyl-2-{N-{α-cyclopropyl-4-{3-[(1-méthylpipérazin-4-yl) méthyl] benzoyloxy méthyl} benzyl} N-propylamino} thiazole

### Stade A

### 4-(2,4-dichlorophényl)-5-méthyl-2-{N-{α-cyclopropyl-4-[3-(chlorométhyl) benzoyloxy méthyl] benzyl} N-propylamino} thiazole

A 1,6 g du composé de l'Exemple 4 en solution dans 10 ml de pyridine anhydre, ajouter à 0°C goutte à goutte 0,6 ml de chlorure de 3-chlorométhylbenzoyle. Après 2 heures à 0°C, ajouter du méthanol pour hydrolyser l'excès de chlorure d'acide et diluer la phase organique par du chlorure de méthylène. Laver à l'acide chlorhydrique 2 N, à l'eau saturée en bicarbonate de sodium, puis à l'eau saturée en chlorure de sodium et sécher sur sulfate de sodium anhydre. Evaporer le solvant organique et purifier sur colonne de silice en utilisant comme solvant un mélange de cyclohexane et d'acétate d'éthyle (20 : 1 V/V) pour obtenir le produit attendu. Rendement :81 %

### Etape B

Ajouter à 0,82 g du produit obtenu à l'étape précédente en solution dans 5 ml de diméthylformamide, 0,46 g de carbonate de potassium et 0,74 ml de 1-méthyl pipérazine. Porter le milieu réactionnel à 70°C pendant 1 heure. Hydrolyser puis extraire à l'acétate d'éthyle.

Laver à l'eau saturée en chlorure de sodium, sécher sur sulfate de sodium anhydre et purifier le résidu obtenu sur colonne de silice en utilisant comme éluant un mélange de chlorure de méthylène et de méthanol (97 : 3 V/V).

On obtient ainsi le produit attendu sous forme d'huile.
Rendement : 50 %

Le spectre de résonance magnétique nucléaire du proton est indiqué dans le Tableau V.

Le chlorhydrate correspondant a été obtenu après addition d'une quantité adéquate d'acide chlorhydrique en solution dans l'isopropanol. Point de fusion : 166°C-169°C

### Exemple 13

### 4-(2,4-dichlorophényl)-5-méthyl-2-[N-(1-cyclopropyl-2-méthoxyéth-1-yl) N-propylamino] thiazole

Procéder comme il est décrit dans l'Exemple 1, Etape A, en utilisant comme thiourée la N-(1-cyclopropyl-2-méthoxyéth-1-yl) N-propyl thiourée ***(Composé 75)***, pour obtenir le composé attendu (gomme).
Rendement : 48 %

Le spectre de résonance magnétique nucléaire du proton de ce composé est indiqué dans le Tableau V.

Le chlorhydrate correspondant a été obtenu après addition d'une quantité adéquate d'acide chlorhydrique en solution dans l'isopropanol et précipitation par l'éther isopropylique. Point de fusion : 154°C

### Exemple 14

### 4-(2,4-dichlorophényl)-5-méthyl-2-[N-(α-hydroxyméthylbenzyl) N-propylamino] thiazole

### Etape A

### 4-(2,4-dichlorophényl)-5-méthyl-2-{N-[α-(tetrahydropyran-2-yloxyméthyl) benzyl] N-propylamino} thiazole

Procéder comme il est décrit à l'Exemple 1, Etape A en utilisant comme thiourée la N-[α-(tetrahydropyran-2-yloxyméthyl) benzyl] N-propyl thiourée (***Composé 96***), pour obtenir le produit attendu (gomme jaune).
Rendement : 80 %
Spectre de résonance magnétique nucléaire du proton (solvant CDCl₃) :
   0,78 ppm, m, 3H ; 1,42-1,75 ppm, m, 8H ; 2,16 ppm, s, 3H ; 3,20 ppm, m, 2H ; 3,5 ppm, m, 1H ; 3,82 ppm, m, 1H ; 4,0 ppm, m, 1H ; 4,3 ppm, m, 1H ; 4,72 ppm, d, 1H ; 5,5 ppm, m, 1H ; 7,22-7,45 ppm, m, 8H.

### Etape B

Dissoudre 19,8 g du produit obtenu à l'étape précédente dans 400 ml d'un mélange d'acide acétique, de tetrahydrofurane et d'eau (4 : 2 : 1 V/V). Mettre sous agitation le mélange réactionnel pendant 20 heures à 50°C puis pendant 4 heures à 70°C. Evaporer sous pression réduite et reprendre le résidu par 200 ml d'hydroxyde de sodium 1 N. Agiter pendant 30 minutes, puis extraire par du chlorure de méthylène. Laver à l'eau la phase organique jusqu'à la neutralité puis à l'eau saturée en chlorure de sodium. Sécher sur sulfate de sodium anhydre et purifier le résidu sur colonne de silice en utilisant comme éluant un mélange de cyclohexane et d'acétate d'éthyle (7 : 1 puis 4 : 1 V/V). On obtient ainsi 4-(2,4-dichlorophényl)-5-méthyl-2-{N-(α-(hydroxyméthyl) benzyl] N-propylamino} thiazole. Son spectre de résonance magnétique nucléaire est indiqué dans le Tableau V.
Rendement : 80 %

Le chlorhydrate correspondant a été obtenu après addition d'une quantité adéquate d'acide chlorhydrique en solution dans isopropanol et précipitation par l'éther isopropylique. Point de fusion : 160°C

### Exemple 15

### 4-(2,4-dichlorophényl)-5-méthyl-2-{N-[α-(acétoxyméthyl) benzyl] N-propylamino} thiazole

Agiter à 0°C une solution contenant 920 mg du composé décrit dans l'Exemple 14 en solution dans 10 ml de pyridine et additionner 0,28 ml d'anhydride acétique. Agiter le milieu réactionnel pendant 14 heures à 20°C puis additionner de la glace et évaporer à sec. Reprendre le résidu par du toluène et évaporer à nouveau à sec. Dissoudre le résidu dans le chlorure de méthylène. Laver la phase organique à l'eau, à l'eau saturée en chlorure de sodium puis sécher sur sulfate de sodium anhydre et évaporer à sec. Purifier sur colonne de silice en utilisant comme éluant un mélange de cyclohexane et d'acétate d'éthyle (9 : 1 V/V) pour obtenir le produit attendu (gomme). Le spectre de résonance magnétique nucléaire du proton est indiqué dans le Tableau V. Rendement : 87 %

Le produit est ensuite salifié sous forme de chlorhydrate. Point de fusion : 54°C

### Exemple 16

### 4-(2,4-dichlorophényl)-5-méthyl-2-{N-[α-(méthyloxyméthyl) benzyl] N-propylamino} thiazole

Mettre sous agitation une solution de 1,26 g du produit de l'Exemple 14 dans 15 ml de diméthylformamide et additionner 0,144 g d'hydrure de sodium. Après 2 minutes, ajouter dans le milieu réactionnel 0,56 ml d'iodométhane. Après 2 heures d'agitation de 0°C à 20°C, additionner de la glace dans le milieu réactionnel puis extraire à l'acétate d'éthyle. Laver la phase organique à l'eau puis à l'eau saturée en chlorure de sodium, sécher sur sulfate de sodium anhydre et évaporer à sec. Purifier le résidu sur colonne chromatographique en utilisant comme éluant un mélange de cyclohexane et d'acétate d'éthyle (10 : 1 V/V).

On obtient ainsi le composé attendu.
Rendement : 93 %

Le 4-(2,4-dichlorophényl) 5-méthyl 2-{N-[α-(méthyloxyméthyl) benzyl] N-propylamino} thiazole est salifiable sous forme de chlorhydrate qui cristallise dans l'éther éthylique. Point de fusion : 154°C (chlorhydrate)

Le spectre de résonance magnétique nucléaire du proton de la base est indiqué dans le Tableau V.

### Exemple 17

### 4-(2,4-dichlorophényl)-5-méthyl-2-{N-[α-(éthoxyméthyl) benzyl] N-propylamino} thiazole

Pour obtenir ce composé, procéder comme il est décrit dans l'Exemple 16 mais en utilisant l'iodoéthane au lieu de l'iodométhane. Le spectre de résonance magnétique nucléaire du proton est indiqué dans le Tableau V.
Rendement : 90 %

Le chlorhydrate correspondant a été obtenu après addition d'une quantité adéquate d'une solution d'acide chlorhydrique dans l'isopropanol. Point de fusion : 132°C

### Exemple 18

### 4-(2,4-dichlorophényl)-5-méthyl-2-{N-[α-(hydroxyéthyloxyméthyl) benzyl] N-propylamino} thiazole

### Etape A

### 1-bromo 2-tetrahydropyran-2-yloxyéthane

A une solution de 3,7 ml de 2-bromoéthanol en solution dans 100 ml de chlorure de méthylène, est additionnée 12 ml de dihydropyrane et 20 mg d'acide paratoluène sulfonique. Après agitation pendant 3 heures à 20°C, le milieu réactionnel est lavé à l'eau puis à l'eau saturée en chlorure de sodium, et évaporé à sec. Le résidu est distillé à 90°C sous une pression de 0,5 mmbar.

On obtient 9,9 g de produit huileux, incolore.
Rendement : 91 %

### Etape B

### 4-(2,4-dichlorophényl) 5-méthyl 2-{N-[α-(tetrahydropyran-2-yloxy éthyloxyméthyl) benzyl] N-propylamino} thiazole

Porter à 0°C une solution contenant 1,04 g du produit de l'Exemple 14 en solution dans 10 ml de diméthylformamide puis additionner 150 mg d'hydrure de sodium en suspension à 80 % dans l'huile. Ajouter ensuite 1,05 g du produit obtenu à l'Etape A et laisser sous agitation pendant 3 heures environ. Additionner d'eau et extraire par l'acétate d'éthyle. Laver les extraits à l'eau et évaporer à sec.

### Etape C

Dissoudre le résidu obtenu à l'étape B dans un mélange d'acide acétique, de tetrahydrofurane et d'eau (4 : 2 : 1 V/V) et agiter pendant 3 heures à 70°C. Après évaporation à sec reprendre le résidu par 20 ml de méthanol et 20 ml d'hydroxyde de sodium 1 N et mettre sous agitation à 40°C pendant environ 10 minutes. Evaporer ensuite le méthanol et extraire la phase aqueuse alcaline au chlorure de méthylène. Laver l'extrait organique à l'eau puis à l'eau saturée en chlorure de sodium, sécher sur sulfate de sodium anhydre et évaporer à sec.

Purifier le résidu sur colonne de silice en utilisant comme éluant un mélange de cyclohexane et d'acétate d'éthyle (4 : 1 V/V). On obtient ainsi le produit attendu salifiable sous forme de chlorhydrate. Rendement : 57 %
Point de fusion : 64°C (chlorhydrate).

Le spectre de résonance magnétique nucléaire du proton du 4-(2,4-dichlorophényl) 5-méthyl-2-{N-[α(-hydroxyéthyloxyméthyl) benzyl] N-propylamino} thiazole est indiqué dans le Tableau V.

### Exemple 19

### 4-(2,4-dichlorophényl)-5-méthyl-2-{N-[α-(méthoxyéthyloxyméthyl) benzyl] N-propylamino} thiazole

Ce composé a été préparé à partir du composé décrit dans l'exemple 18 et en utilisant le procédé décrit dans l'Exemple 16. Le spectre de résonance magnétique nucléaire du proton est indiqué dans le Tableau V.
Rendement : 70 %

Le chlorhydrate correspondant a été obtenu après addition d'une quantité adéquate d'acide chlorhydrique en solution dans l'isopropanol.
Point de fusion : 45°C

### Exemple 20

### 4-(2,4-diméthylpyrid-5-yl)-5-méthyl-2-[N-(dicyclopropyl méthyl) N-propylamino] thiazole

Ce composé a été préparé selon le procédé décrit dans l'Exemple 1, Etape A, en utilisant comme cétone la 2-bromo 1-(2,4-diméthyl pyrid-5-yl) propan-1-one ***(Composé 27)*** et comme thiourée la N-(dicyclopropyl méthyl) N-propyl thiourée ***(Composé 66)***.
Rendement : 80 %

Le spectre de résonance magnétique nucléaire du proton est indiqué dans le Tableau V.

### Exemple 21-22

Selon le procédé décrit dans l'Exemple 20 ont été obtenus les composés des Exemples 21 et 22 en utilisant les dérivés de bromocétones et de thiourées adéquats. Leurs caractéristiques spectrales sont indiquées dans le Tableau V.

### Exemple 23

### 4-(2,4-dihydroxyméthylphényl)-5-méthyl-2-[N-(α-cyclopropyl benzyl) N-propylamino] thiazole

### Etape A

### 4-(2,4-dicarboxyphényl)-5-méthyl-2-[N-(α-cyclopropylbenzyl) N-propylamino] thiazole

A 1,1 g de 4-(2,4-dibromophényl) 5-méthyl-2-[N-(α-cyclopropyl benzyl) N-propylamino] thiazole (préparés à partir de la 2-bromo-1-(2,4-dibromo phényl) propan-1-one ***(Composé 14)*** et de la N-(α-cyclopropylbenzyl) N-propyl thiourée ***(Composé 65)***, selon le procédé décrit dans l'Exemple 1, Etape A), en solution dans 30 ml d'éther éthylique anhydre, ajouter à -70°C 5 ml d'une solution 1,7 M de *tert*-butyllithium dans le pentane on laisse remonter la température à 50°C puis on soumet à l'action de gaz carbonique en laissant remonter la température à 20°C.

Après hydrolyse du mélange réactionnel à 0°C par de l'acide chlorhydrique 1 N, extraire à l'acétate d'éthyle, laver à l'eau, sécher sur sulfate de sodium anhydre et évaporer à sec.

On obtient ainsi le produit attendu.
Rendement : 99 %

### Etape B

Dissoudre 1 g du composé obtenu à l'étape A dans 20 ml de tetrahydrofurane anhydre et additionner à - 7°C 5 ml d'une solution 1 M d'hydrure de bore dans le tetrahydrofurane, et laisser sous agitation une nuit. Ajouter ensuite 0,5 g de carbonate de potassium et 10 ml d'un mélange de méthanol et d'eau (1 : 1 V/V). Porter 2 heures à 60°C puis à froid, évaporer sous vide. Reprendre le résidu à l'eau, extraire à l'éther éthylique, laver à l'eau puis à l'eau saturée en chlorure de sodium et sécher sur sulfate de sodium anhydre. Evaporer à sec et reprendre le résidu dans le pentane. On obtient ainsi le produit attendu.

Le spectre de résonance magnétique nucléaire du proton est indiqué dans le Tableau V.
Rendement : 40 %

### Exemple 24

### 4-[2-chloro-4-(1-hydroxyéth-1-yl)phényl]-5-méthyl-2-[N-(dicyclo propylméthyl) N-propylamino] thiazole

Ce composé a été préparé à partir du 4-(4-bromo-2-chlorophényl) 5-méthyl-2-[N-(dicyclopropylméthyl) N-propylamino] thiazole, en appliquant le procédé de préparation décrit dans l'Exemple 8.

Le spectre de résonance magnétique nucléaire du proton est indiqué dans le Tableau V.
Rendement : 83 %

Le 4-(4-bromo-2-chlorophényl)-5-méthyl-2-[N-(dicyclopropylméthyl) N-propylamino] thiazole a été obtenu à partir du ***Composé 16*** et du ***Composé 66*** selon le procédé décrit dans l'Exemple 1, Etape A.

Le chlorhydrate de 4-[2-chloro-4-(1-hydroxyéth-1-yl) phényl]-5-méthyl 2-[N-(dicyclopropylméthyl) N-propylamino] thiazole a été préparé après addition d'une quantité adéquate d'acide chlorhydrique en solution dans l'isopropanol.
Point de fusion : 93°C

### Exemple 25

### 4-[4-chloro-2-(1-hydroxyéth-1-yl) phényl]-5-méthyl-2-[N-(dicyclo propyl méthyl) N-propylamino] thiazole

Ce composé a été préparé selon le procédé décrit dans l'Exemple 24 à partir du 4-(2-bromo-4-chlorophényl)-5-méthyl-2-[N-(dicyclopropylméthyl) N-propylamino] thiazole.

Le spectre de résonance magnétique nucléaire du proton est indiqué dans le Tableau V.
Rendement : 83 %

Le chlorhydrate a été obtenu après addition d'une quantité adéquate d'acide chlorhydrique en solution dans l'isopropanol.
Pont de fusion : 123°C

### Exemple 26

### 4-(2,4-dichlorophényl)-5-méthyl-2-[N-(α-cyclopropylbenzyl) N-(tetrahydropyran-2-yloxyéthyl) amino] thiazole

Préparer le 4-(2,4-dichlorophényl)-5-méthyl-2-[N-(α-cyclopropyl benzyl) amino] thiazole à partir de la 2-bromo 1-(2,4-dichlorophényl) propan-1-one ***(Composé 1)*** et de la N-(α-cyclopropylbenzyl) thiourée ***(Composé 74)*** selon le procédé décrit dans l'Exemple 1, Etape A. Dissoudre 4 g de ce composé dans 60 ml de diméthylformamide anhydre et refroidir à 5°C, puis ajouter 493 mg d'hydrure de sodium par petites portions. Après une heure d'agitation à température ambiante, ajouter goutte à goutte 3,14 g de bromure de 1-(2-tetrahydropyran-2-yloxy) éthane en solution dans 30 ml de diméthylformamide. Laisser sous agitation pendant 3 heures. Additionner de l'eau et extraire par l'acétate d'éthyle, laver à l'eau, sécher sur sulfate de sodium anhydre et évaporer à sec. Purifier le résidu sur colonne de silice en utilisant comme éluant un mélange de cyclohexane et d'acétate d'éthyle (1 : 1 V/V). On obtient ainsi le produit attendu.

Le spectre de résonance magnétique nucléaire du proton est indiqué dans le Tableau V.
Rendement : 99 %

Le chlorhydrate correspondant a été préparé après addition d'une quantité adéquate d'acide chlorhydrique en solution dans l'isopropanol.
Point de fusion : 109°C

### Exemple 27

### 4-(2,4-dichlorophényl)-5-méthyl-2-[N-(α-cyclopropylbenzyl) N-(2-hydroxyéthy-1-yl) amino] thiazole

Dissoudre 4 g du produit de l'Exemple 26 dans 80 ml d'un mélange d'acide acétique, de tetrahydrofurane et d'eau (4 : 2 : 1 V/V) et chauffer à 40°C pendant 18 heures puis évaporer à sec. Purifier le résidu sur colonne de silice en utilisant comme éluant un mélange de cyclohexane et d'acétate d'éthyle pour obtenir le produit attendu.
Rendement : 90 %
Point de fusion : 128°C

### Exemples 28 à 31

Selon le procédé décrit dans l'Exemple 1, Etape A ont été obtenus les composés des Exemples 28 et 31 en utilisant les dérivés de bromo acétone et de thiourée adéquats. Leurs caractéristiques spectrales sont indiquées dans le Tableau V.

### Exemple 32

### 4-(2,4-dichlorophényl)-5-méthyl-2-{N-[α-(cyclopropylméthoxy méthyl) benzyl] N-propylamino} thiazole

A une solution de 4,64 mg du composé de l'Exemple 14 en solution dans 7 ml de diméthylformamide, additionner sous agitation à 0°C 32 mg d'hydrure de sodium puis 0,19 ml de bromure de cyclopropylméthyle. Agiter le milieu réactionnel pendant 16 heures à température ambiante. Verser ensuite sur de la glace, extraire à l'acétate d'éthyle, laver à l'eau puis à l'eau saturée en chlorure de sodium, sécher sur sulfate de sodium anhydre et évaporer à sec. Purifier le résidu par chromatographie sur colonne de silice en utilisant comme éluant un mélange de cyclohexane et d'acétate d'éthyle. On obtient ainsi le produit attendu .

Le spectre de résonance magnétique nucléaire du proton est indiqué dans le Tableau V.
Rendement : 50 %

Le chlorhydrate correspondant a été préparé après addition d'une quantité adéquate d'acide chlorhydrique en solution dans l'isopropanol.
Point de fusion : 58°C

### Exemple 33

### 4-(2,4-dichlorophényl)-5-méthyl-2-{N-[α-(cyclopropyl)-4-(méthoxyéthyl)benzyl]-N-propylamino}thiazole

A 0,64 g de 2-{N-[α-cyclopropyl-4-(hydroxyéthyl)benzyl]-N-propyl-amino}thiazole (Exemple 28) dans 15 ml de diméthylformamide anhydre, on ajoute, à 0°C, 0,042 g d'hydrure de sodium et on agite la suspension pendant 20 minutes. On ajoute alors 0,09 ml d'iodure de méthyle et laisse le mélange réactionnel pendant 3 heures à température ambiante. Celui-ci est alors versé sur de la glace pilée et on extrait, par trois reprises, avec de l'acétate d'éthyle. La phase organique est successivement lavée à l'eau, séchée sur sulfate de sodium puis concentrée sous vide. Le résidu obtenu est chromatographié sur gel de silice, éluant : cyclohexane-acétate d'éthyle (10/1 - v/v). La concentration des fractions de produit pur fournit 0,5 g du produit attendu qui est cristallisé sous forme de chlorhydrate.
Rendement 75 %.
Les caractéristiques spectrales de ce produit sont indiquées dans le Tableau V ci-après.

### Exemples 34-58

Selon le procédé décrit dans l'Exemple 1, Etape A ont été obtenus les composés des Exemples 34 à 58 en utilisant les dérivés de bromocétone et de thiourée adéquats. Leurs caractéristiques spectrales sont indiquées dans le Tableau V.

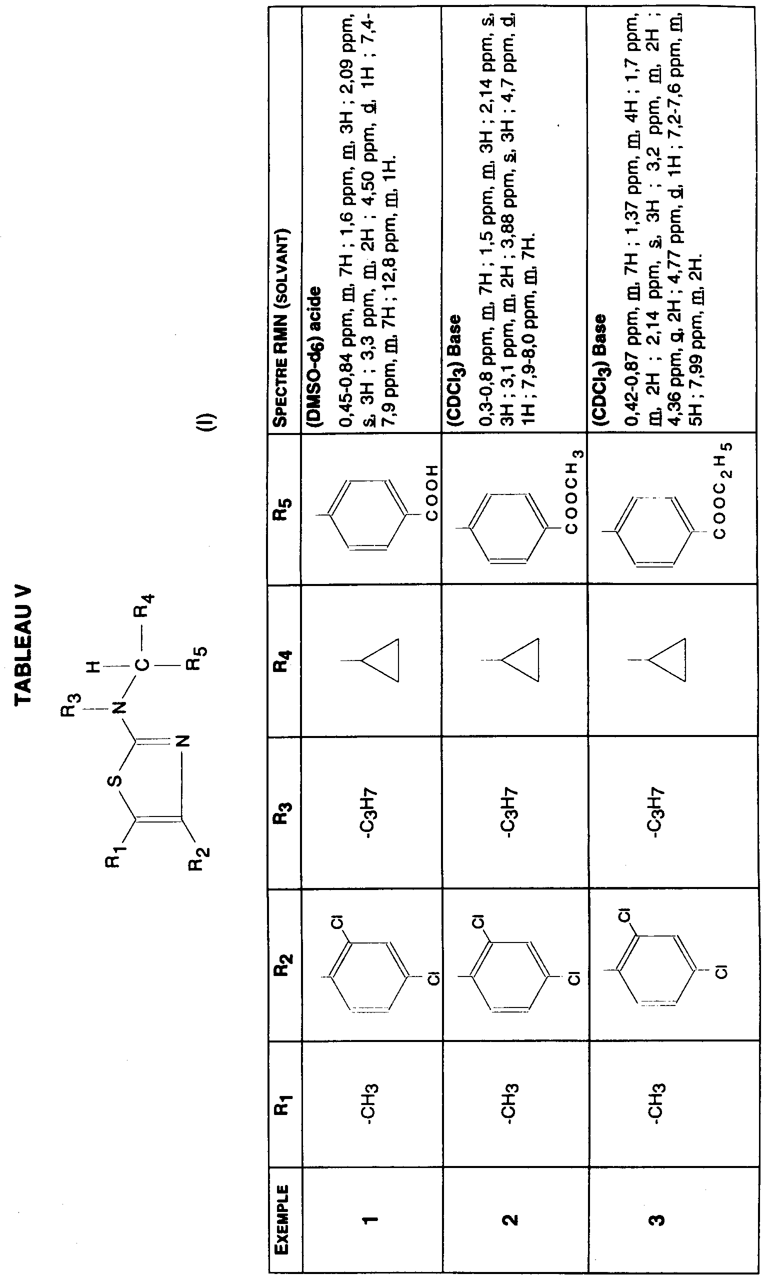

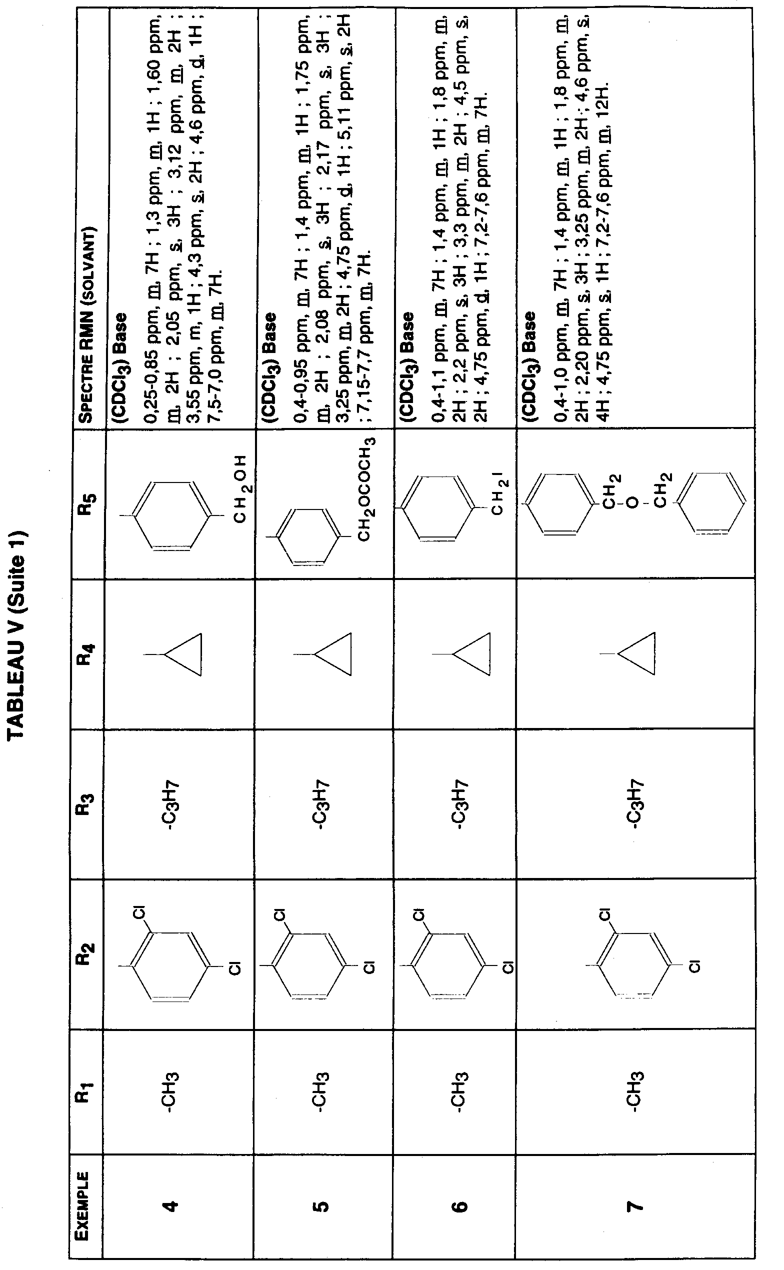

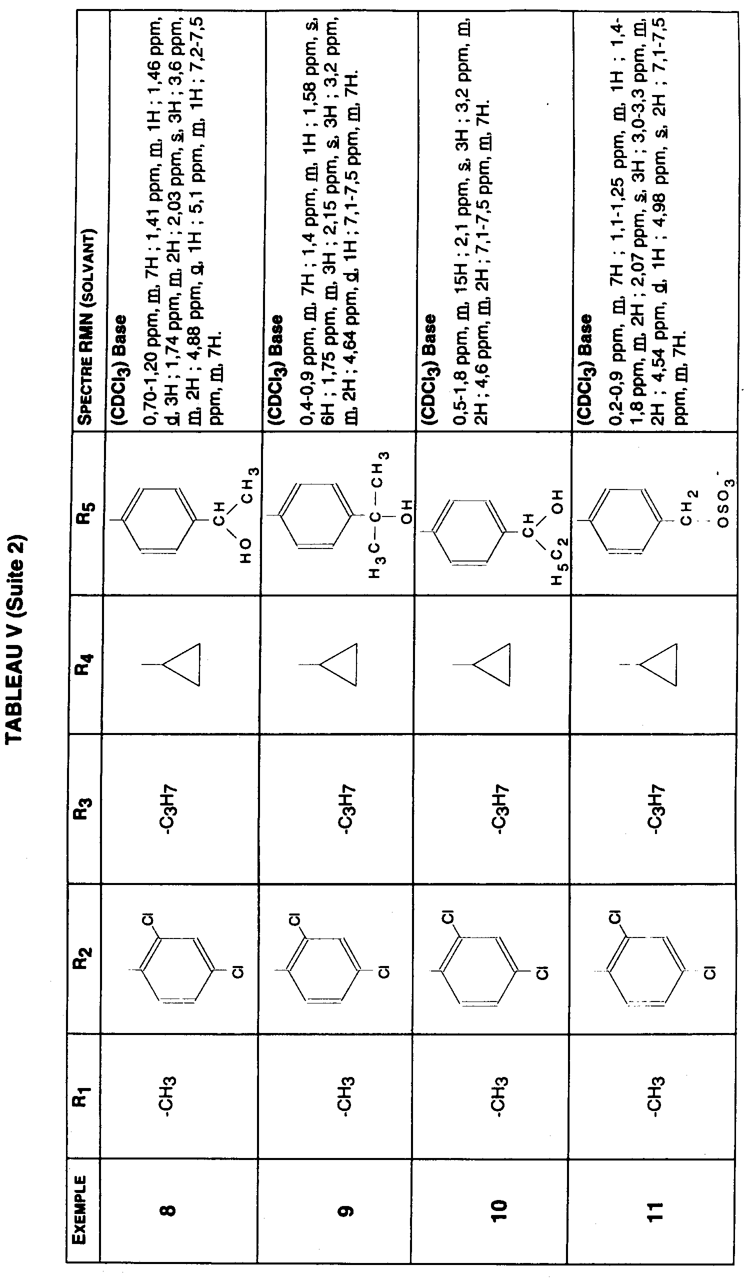

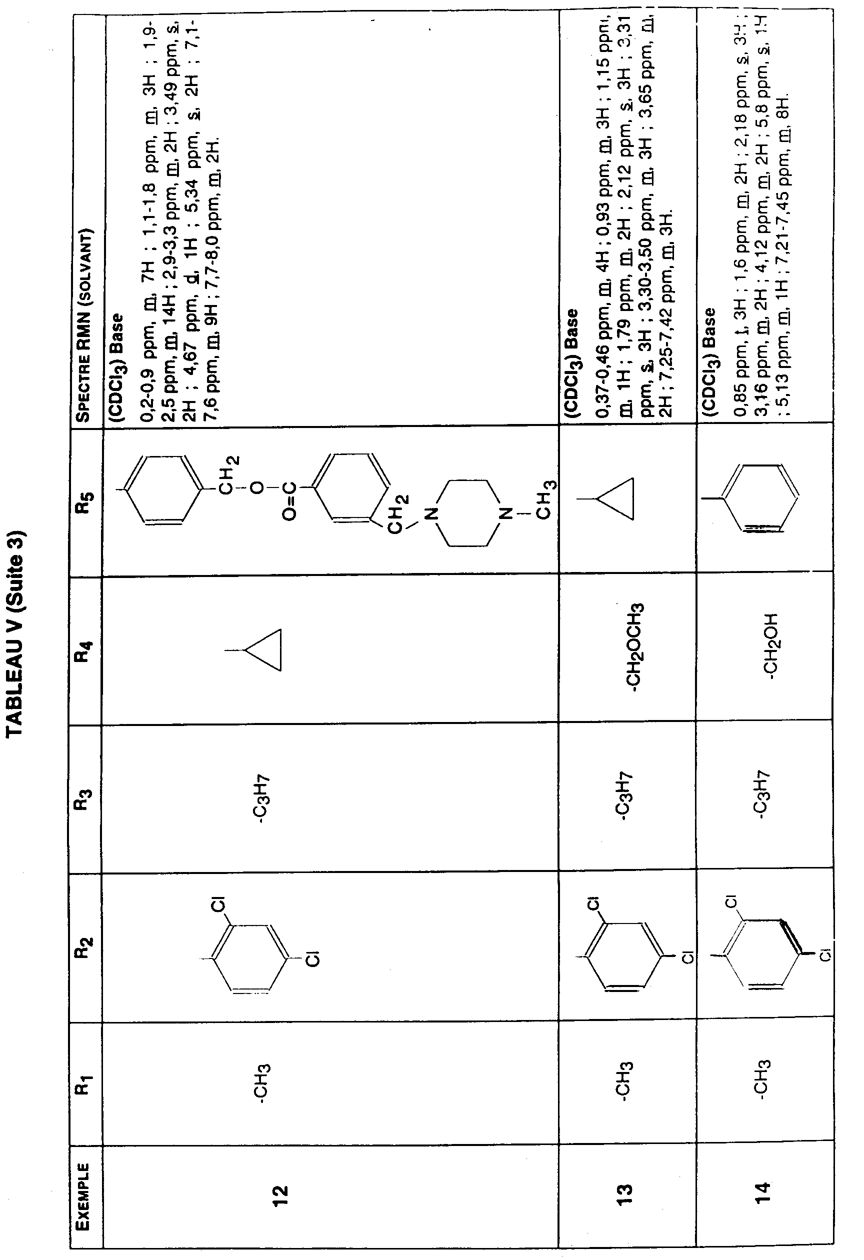

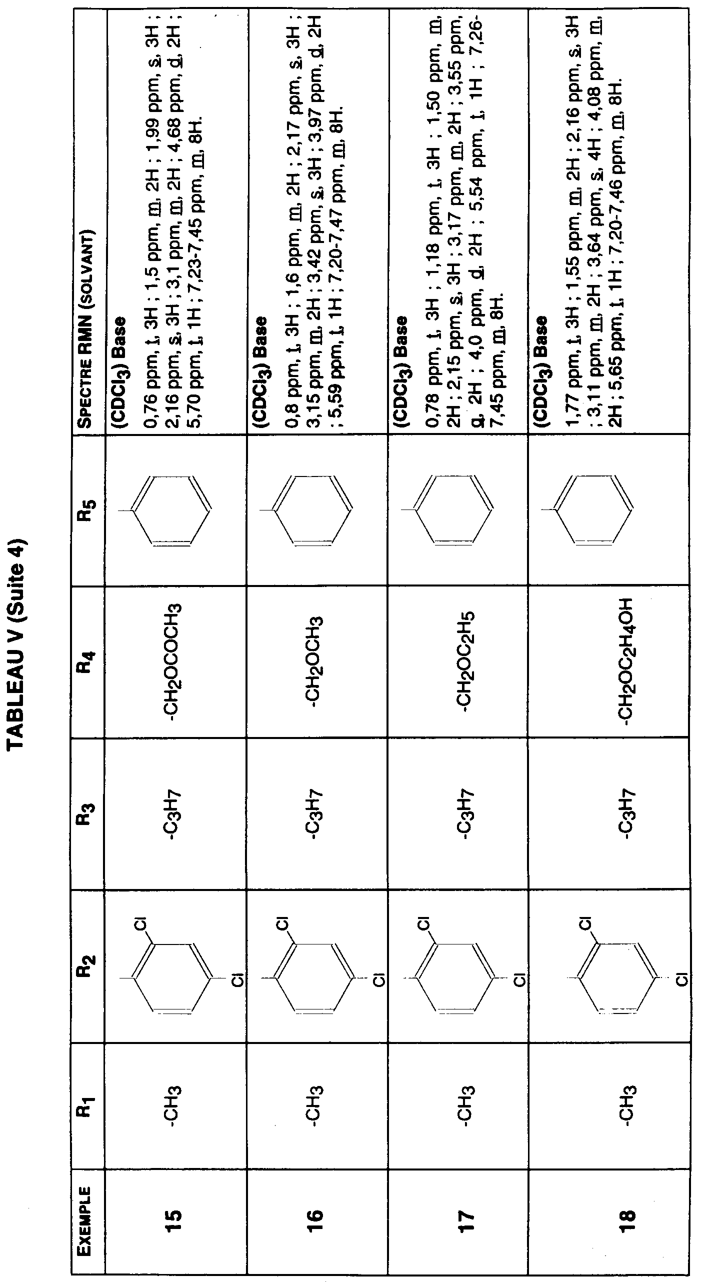

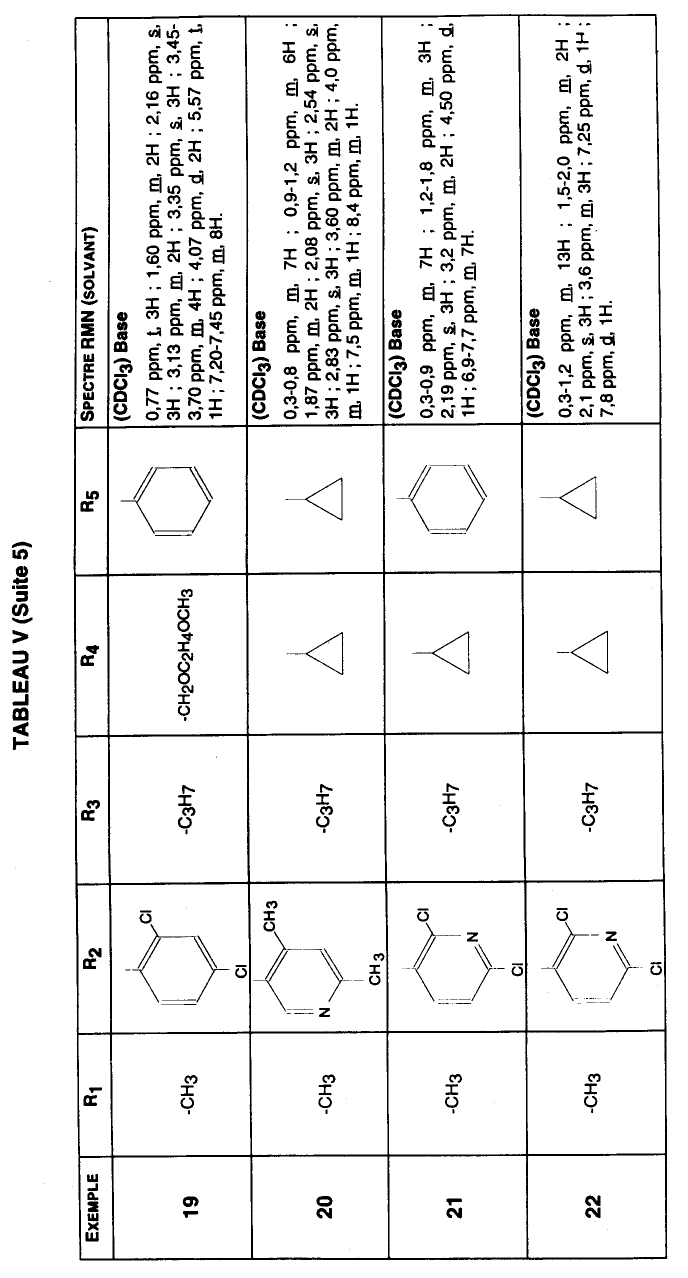

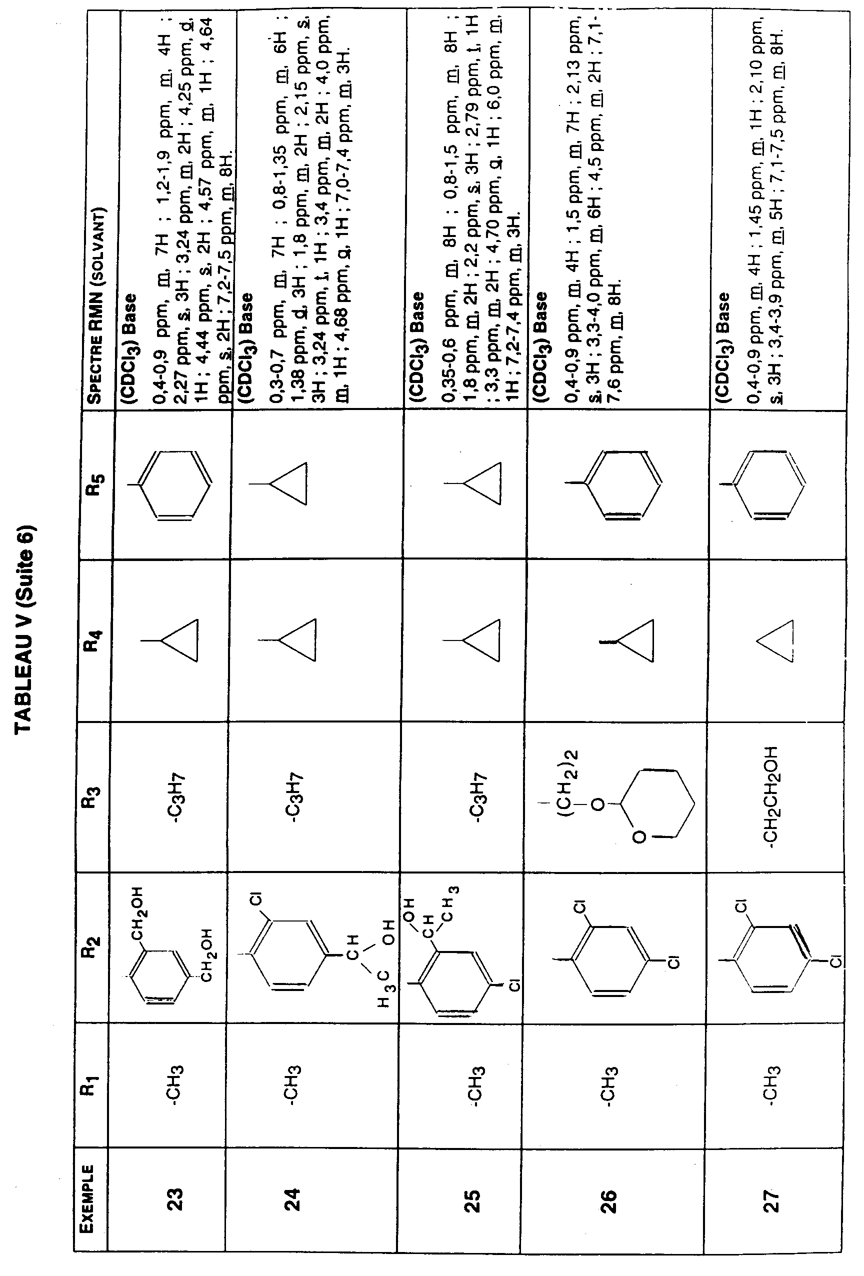

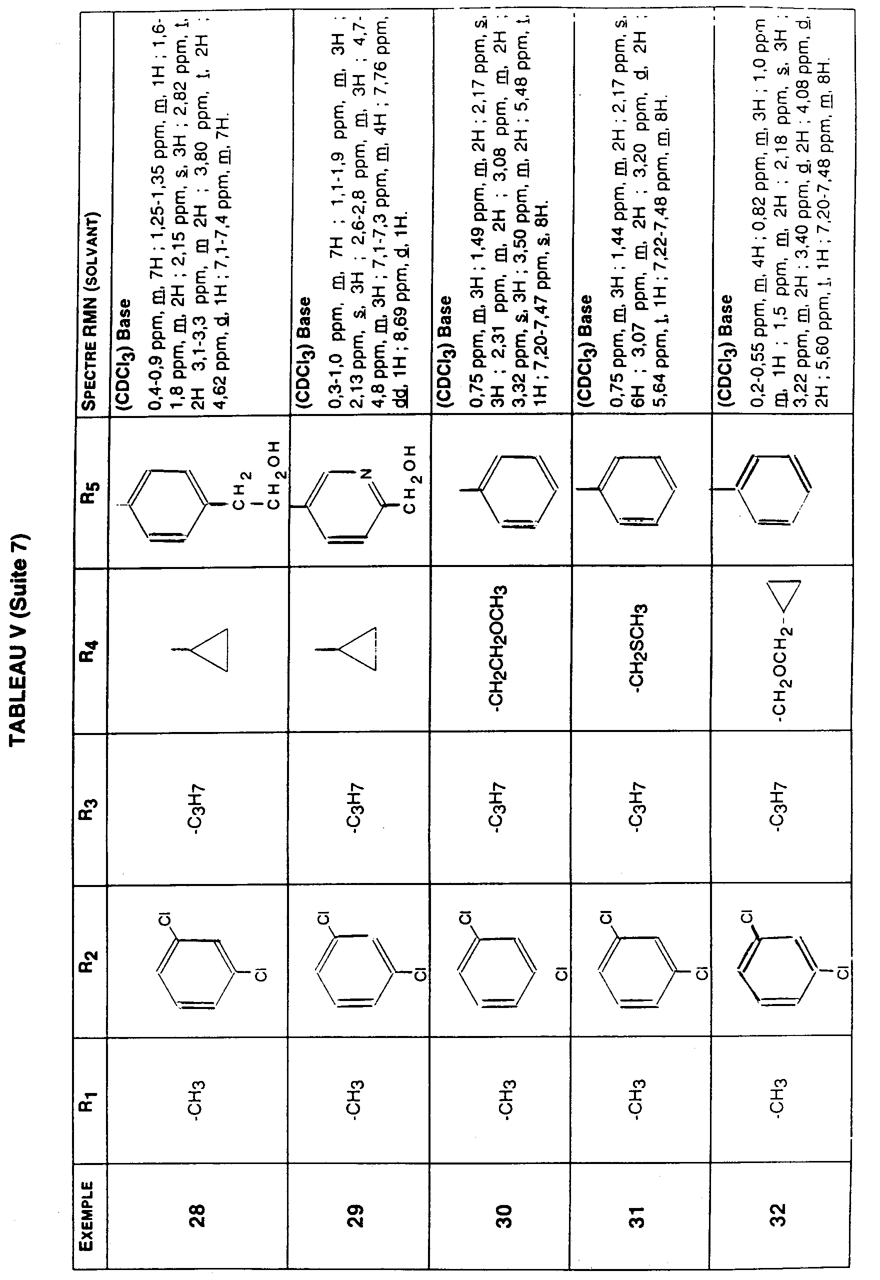

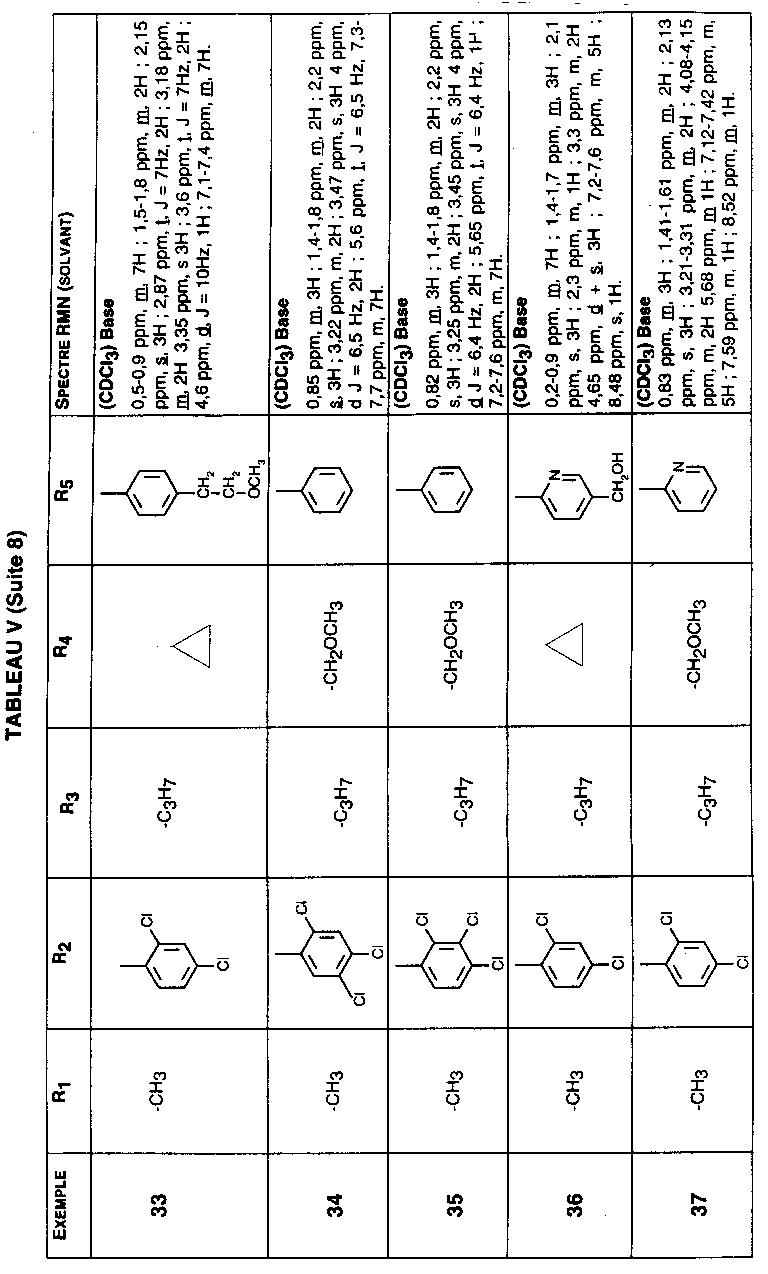

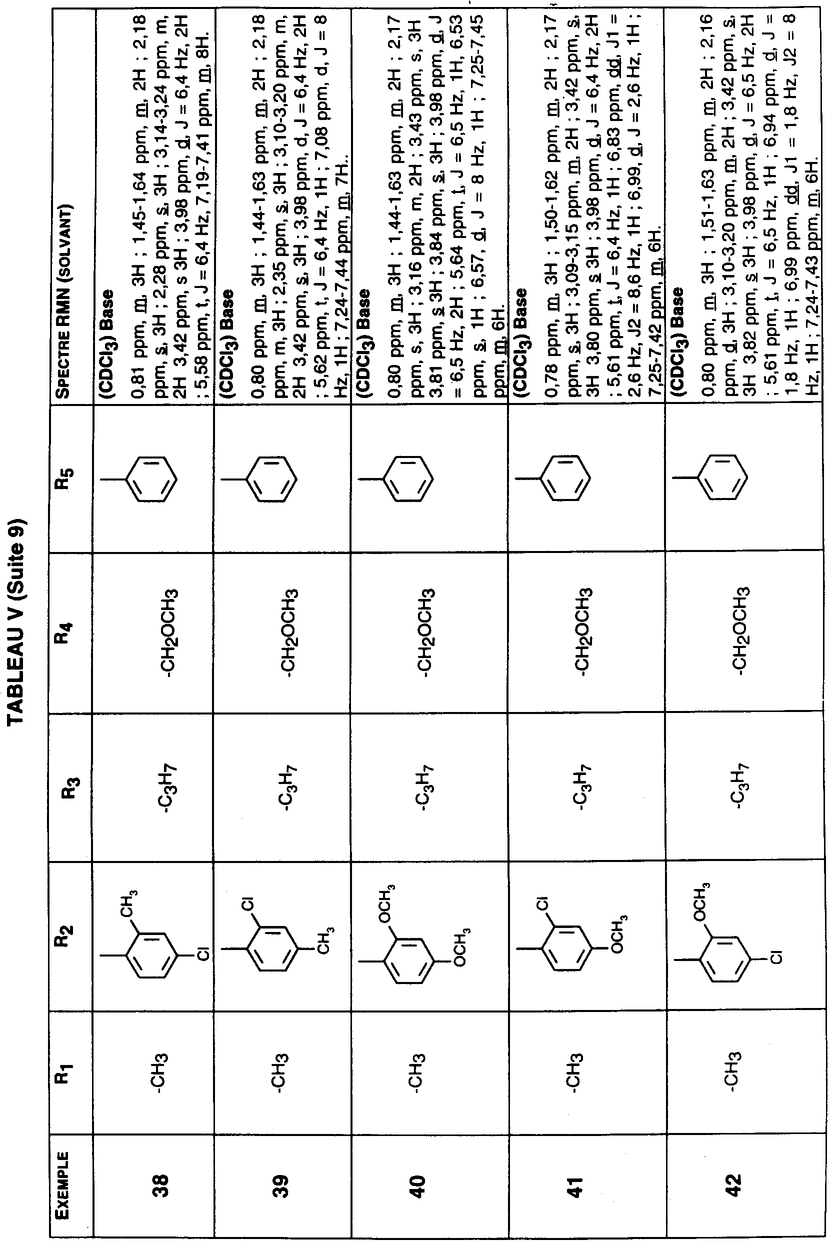

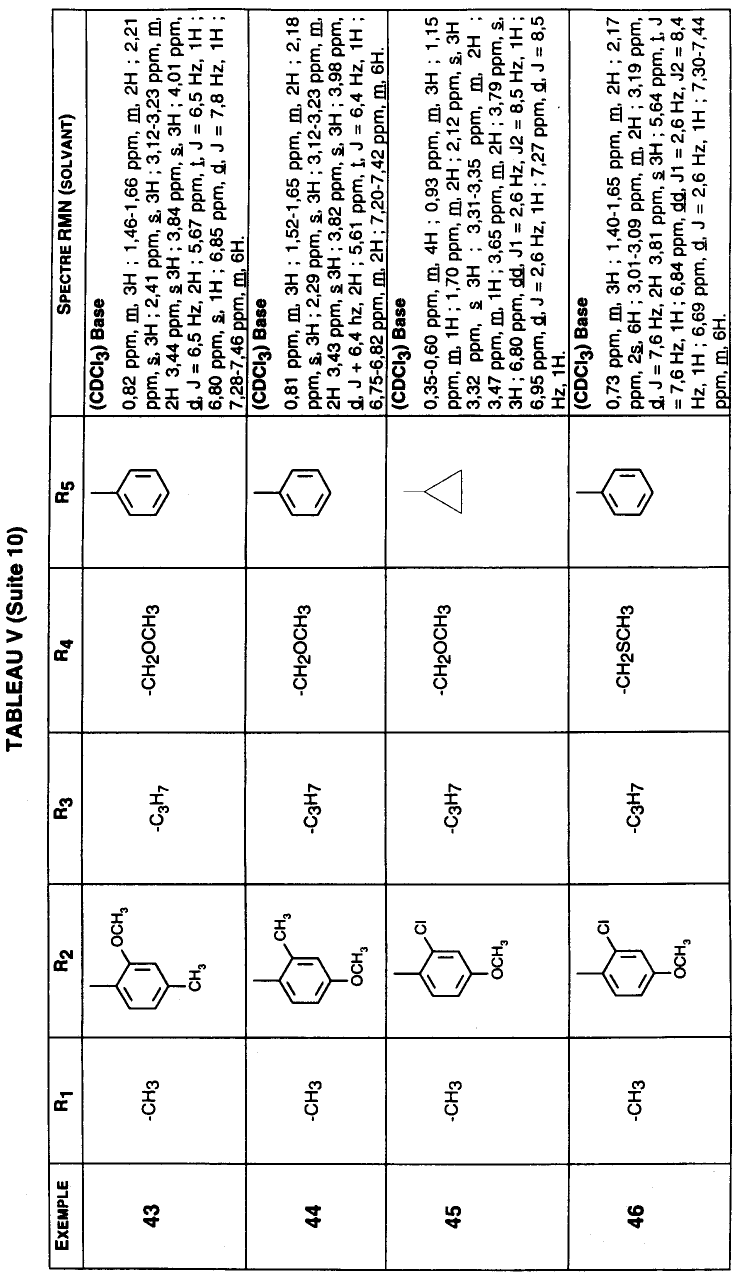

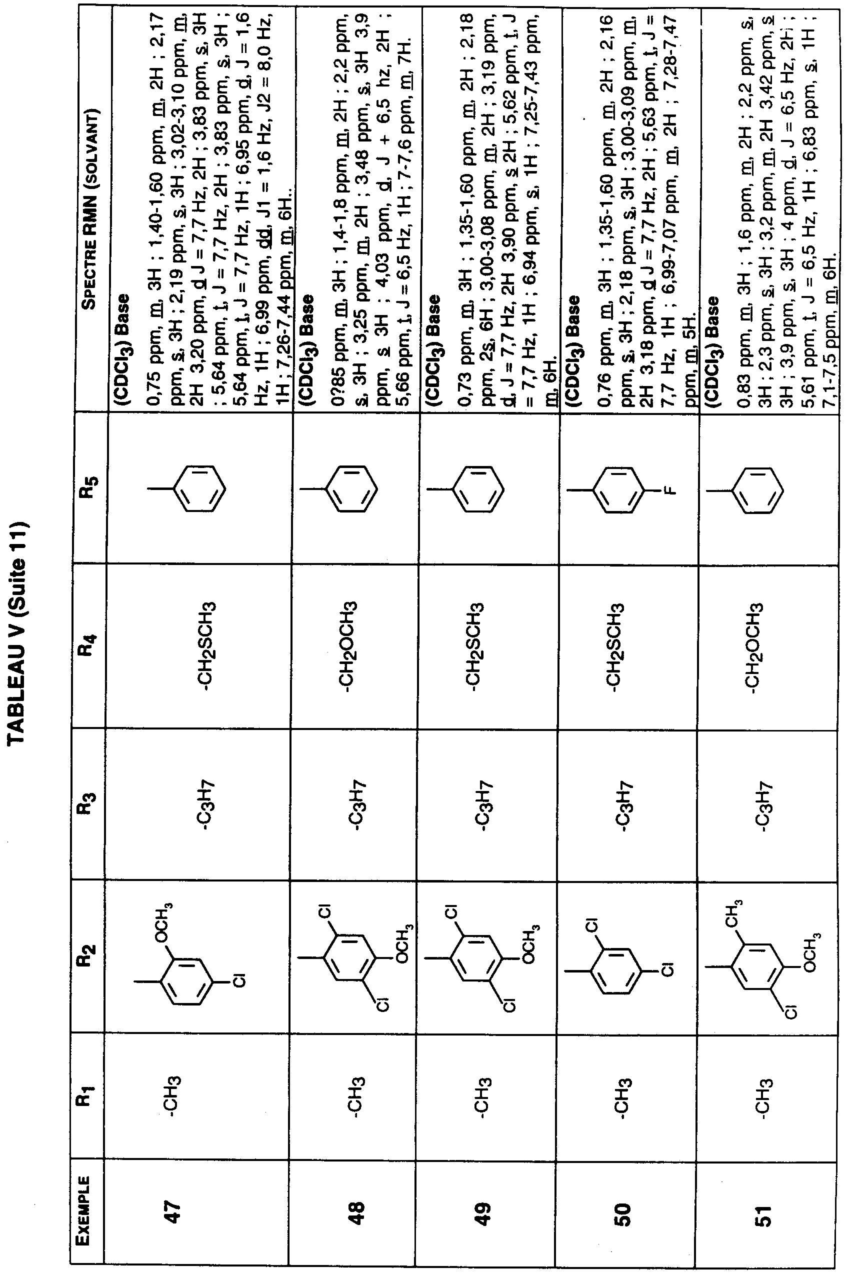

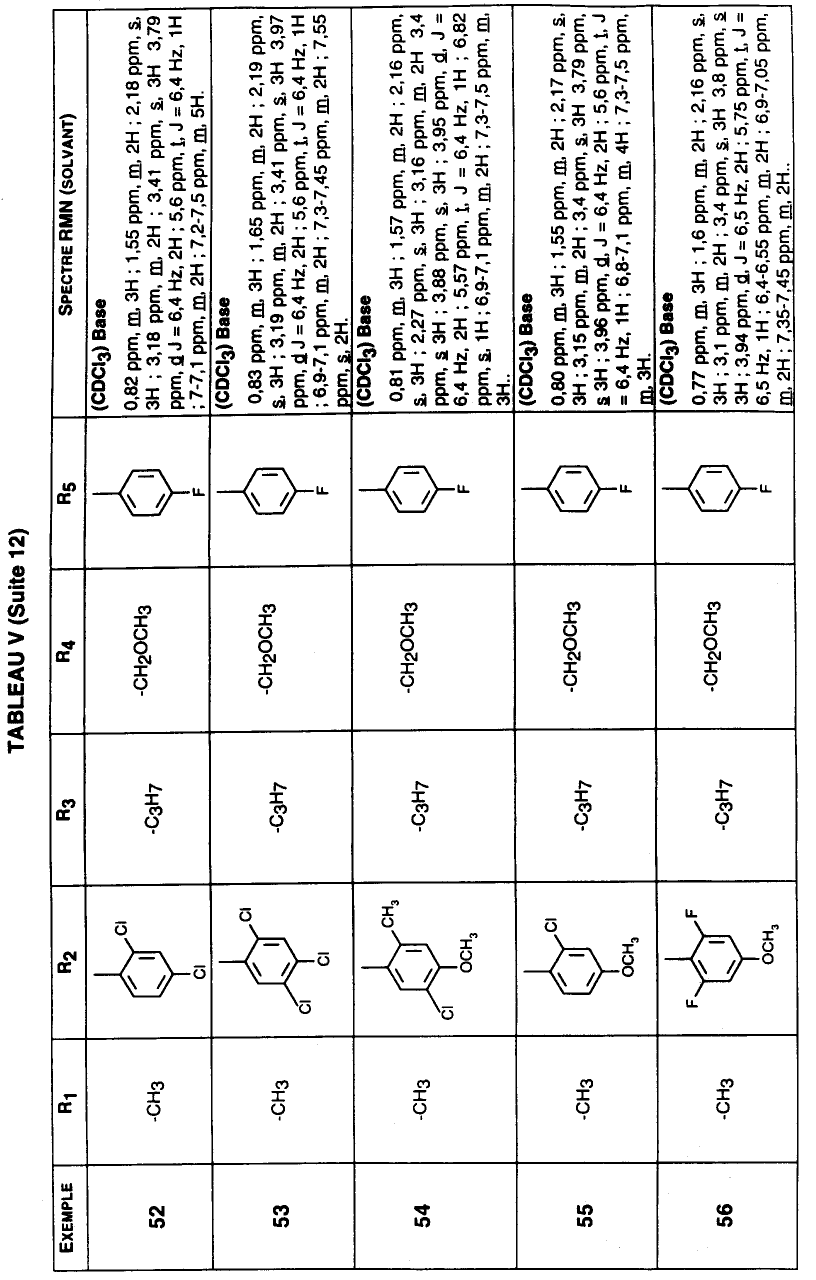

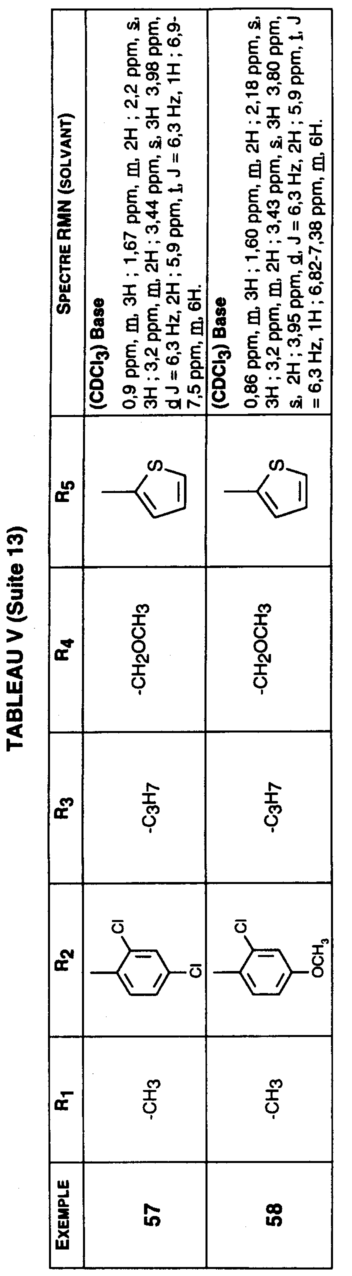

Les Exemples 59, 60 et 61, 62 décrits dans le Tableau VI ci-après ont été respectivement obtenus à partir des phénylglycines optiquement pures (R) et (S) selon l'Exemple 16.

Leurs pouvoirs rotatoires ont été mesurés à 20°C dans l'éthanol.

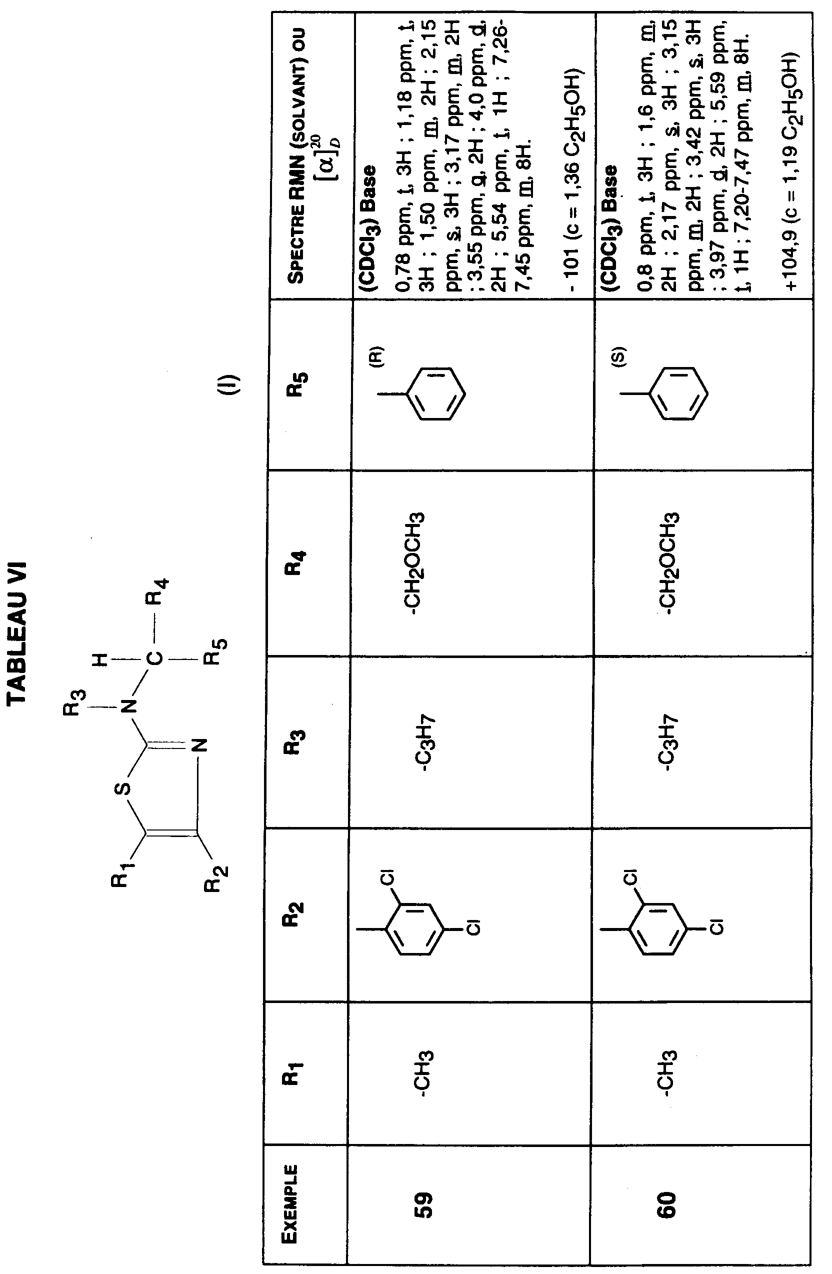

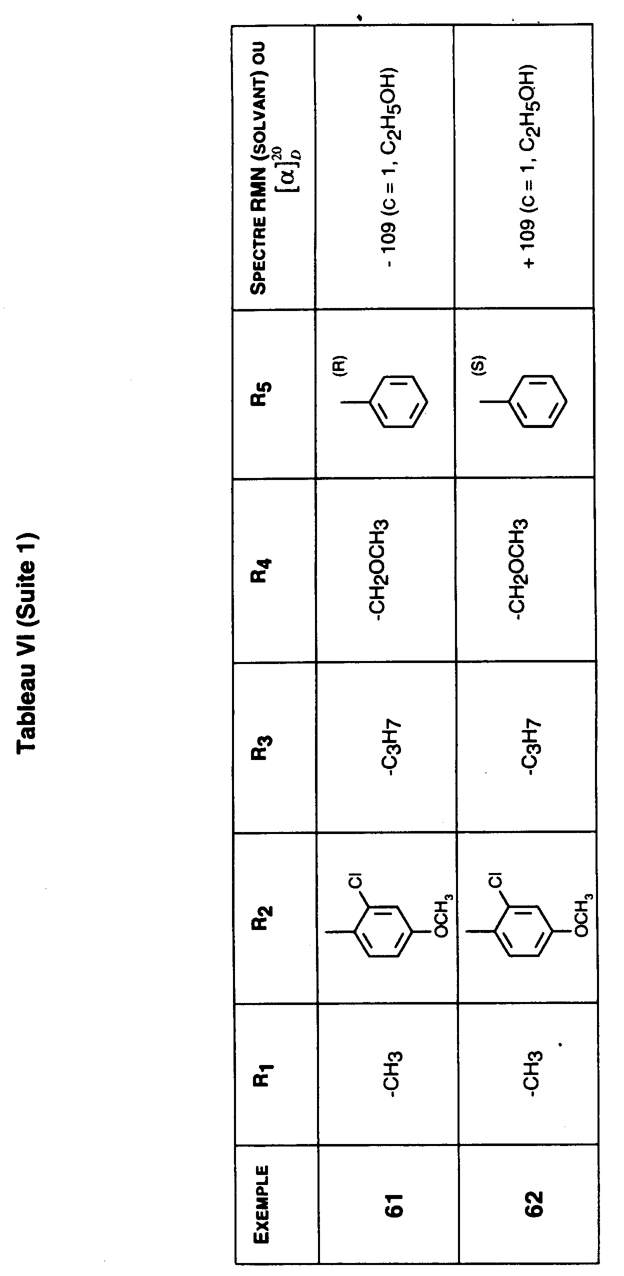

### PREPARATION PHARMACEUTIQUE

### Exemple 63

### Gélules dosées à 20 mg de chlorhydrate de 4-(2,4-dichloro phényl)-5-méthyl-2-[N-(α-éthoxyméthylbenzyl) N-propylamino] thiazole.

| | |
|---|---|
| Chlorhydrate de 4-(2,4-dichlorophényl)-5-méthyl-2-[N-(α-éthoxyméthyl benzyl) N-propylamino] thiazole | 20 mg |
| Amidon de maïs | 15 mg |
| Lactose | 25 mg |
| Talc | 5 mg |
| Pour une gélule N°3 | |

## Revendications

1. Composés de formule I : 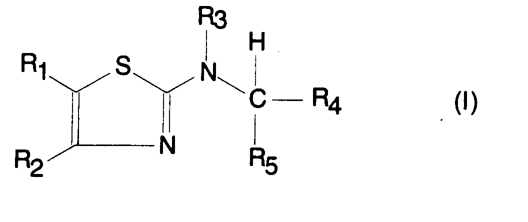 dans laquelle,
- R₁ représente un atome d'hydrogène ou un radical alkyle de 1 à 5 atomes de carbone,
- R₂ représente un radical de formule (A) : 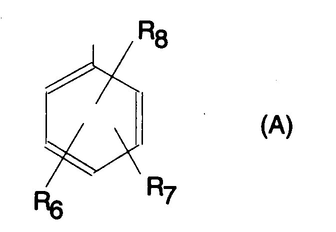 (dans laquelle R₆ représente un radical hydroxyalkyle de 1 à 5 atomes de carbone, et R₇ et R₈, identiques ou différents, représentent chacun un atome d'hydrogène, un atome d'halogène ou un radical hydroxyalkyle de 1 à 5 atomes de carbone, un trifluorométhyle, un radical alcoxy de 1 à 5 atomes de carbone ou un radical alkyle de 1 à 5 atomes de carbone),
un radical de formule (B) : 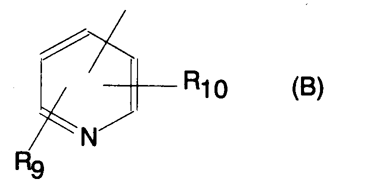 (dans laquelle R₉ et R₁₀ identiques ou différents, représentent chacun un atome d'hydrogène, un atome d'halogène, un radical alkyle de 1 à 5 atomes de carbone ou un radical alcoxy de 1 à 5 atomes de carbone),
ou un radical de formule (C) : 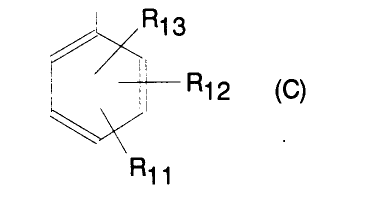 (dans laquelle R₁₁, R₁₂, R₁₃ identiques ou différents, représentent chacun un atome d'hydrogène, un atome d'halogène, un trifluorométhyle, un radical alcoxy de 1 à 5 atomes de carbone ou un radical alkyle de 1 à 5 atomes de carbone),
- R₃ représente un radical alkyle de 1 à 5 atomes de carbone, un radical hydroxyalkyle de 1 à 5 atomes de carbone, un radical tetrahydropyran-2-yloxyalkyle dont le radical alkyle comporte de 1 à 5 atomes de carbone, un radical alcoxyalkyle de 2 à 10 atomes de carbone ou un radical acyloxyalkyle de 3 à 11 atomes de carbone,
- R₄ représente un radical cycloalkyle de 3 à 6 atomes de carbone, un radical hydroxyalkyle de 1 à 5 atomes de carbone, un radical alcoxyalkyle de 2 à 10 atomes de carbone, un radical cycloalkyloxyalkyle de 4 à 11 atomes de carbone, un radical hydroxyalkyloxyalkyle de 2 à 10 atomes de carbone, un radical alcoxyalkyloxyalkyle de 3 à 12 atomes de carbone, un radical acyloxyalkyle de 3 à 11 atomes de carbone ou un radical alkylthioalkyle de 2 à 10 atomes de carbone, et,
- R₅ représente un radical cycloalkyle de 3 à 6 atomes de carbone, un radical phényle, un radical thiényle ou un radical pyridyle (éventuellement substitués par un ou plusieurs atomes d'halogène, par des radicaux alcoxy de 1 à 5 atomes de carbone, des radicaux alkyle de 1 à 5 atomes de carbone ou des radicaux trifluorométhyle), ou un radical de formule (D) : 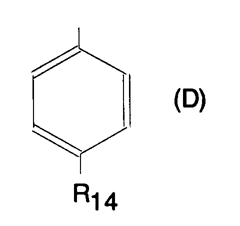 (dans lesquelles R₁₄ représente un radical carboxy, un radical carboxyalkyle de 2 à 6 atomes de carbone, un radical alcoxycarbonyle de 2 à 6 atomes de carbone, un radical acyloxyalkyle de 3 à 11 atomes de carbone, un radical alcoxyalkyle de 2 à 10 atomes de carbone, un radical aralcoxyalkyle de 8 à 16 atomes de carbone (éventuellement substitué sur le cycle aromatique par un ou plusieurs atomes d'halogène, des radicaux alcoxy de 1 à 3 atomes de carbone au par des radicaux trifluorométhyle), un radical alkyle mono-halogéné de 1 à 5 atomes de carbone, un radical hydroxyalkyle linéaire ou ramifié de 1 à 5 atomes de carbone, un radical de formule (E) : 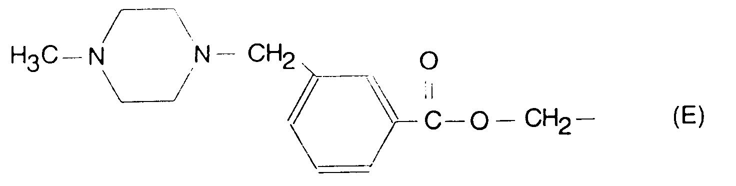 ou un radical sulfoxyalkyle de 1 à 5 atomes de carbone),
un radical 3-hydroxyalkylpyrid-6-yle ou un radical 2-hydroxyalkylpyrid-5-yle (dont les radicaux alkyle comportent de 1 à 5 atomes de carbone),
à condition toutefois que lorsque R₃ représente un radical alkyle de 1 à 5 atomes de carbone, R₄ représente un radical cycloalkyle et R₅ représente soit un radical cycloalkyle soit un radical phényle, un radical thiényle ou un radical pyridyle (éventuellement substitués par un ou plusieurs atomes d'halogène, par des radicaux alcoxy de 1 à 5 atomes de carbone, des radicaux alkyle de 1 à 5 atomes de carbone ou des radicaux trifluorométhyle), R₂ ne représente pas un radical de formule (C),
leurs stéréoisomères et leurs sels d'addition.

2. Composés de formule I selon la revendication 1, répondant à la formule I' : 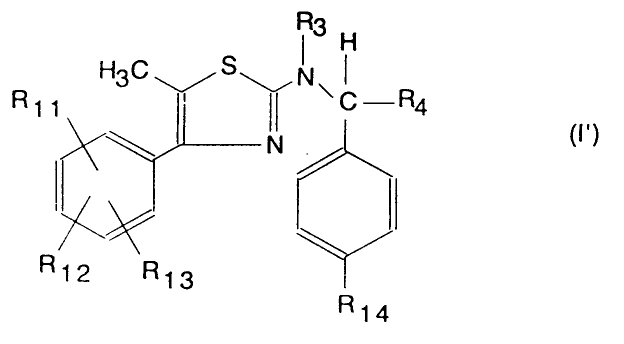 dans laquelle,
- R₁₁ représente un atome d'halogène et R₁₂ et R₁₃ ont la même signification que pour la formule I, selon la revendication 1,
- R₃ représente un radical alkyle de 1 à 5 atomes de carbone,
- R₄ représente un radical cycloalkyle de 3 à 6 atomes de carbone,
et
- R₁₄ a la même signification que pour la formule I, selon la revendication 1,
leurs stéréoisomères et leurs sels d'addition.

3. Composés de formule I, selon la revendication 1, répondant à la formule I'' : 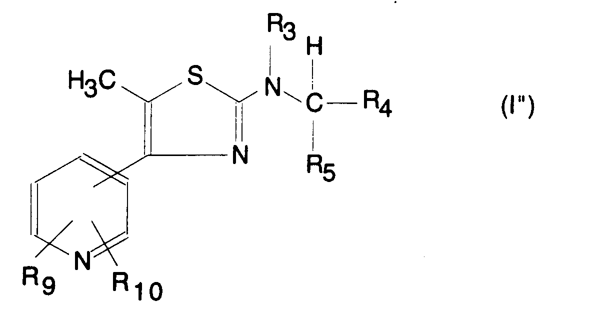 dans laquelle,
- R₃, R₄ et R₅ ,R₉ et R₁₀ ont la signification donnée pour la formule I, selon la revendication 1,
leurs stéréoisomères et leurs sels d'addition.

4. Composés de formule I, selon la revendication 1, répondant à la formule I''' : 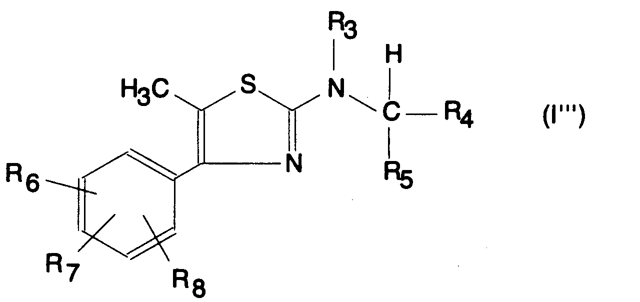 dans laquelle,
- R₇ représente un atome d'halogène ou un alcoxy en C₁-C₅, R₆ et R₈ ont la même signification que pour la formule I, selon la revendication 1,
- R₃ représente un radical alkyle de 1 à 5 atomes de carbone,
et,
- R₄ et R₅ ont la signification donnée pour la formule I, selon la revendication 1, leurs stéréoisomères et leurs sels d'addition.

5. Composés de formule I, selon la revendication 1 dans laquelle :
- R₁ représente un atome d'hydrogène ou un radical alkyle de 1 à 5 atomes de carbone,
- R₂ représente un radical de formule (A) ou un radical de formule (B),
- R₃ représente un radical alkyle de 1 à 5 atomes de carbone,
- R₄ représente un radical cycloalkyle de 3 à 6 atomes de carbone,
et
- R₅ représente un radical cycloalkyle de 3 à 6 atomes de carbone, un radical phényle, un radical thiényle ou un radical pyridyle (éventuellement substitués par un ou plusieurs atomes d'halogène, par des radicaux alcoxy de 1 à 5 atomes de carbone, des radicaux alkyle de 1 à 5 atomes de carbone ou des radicaux trifluorométhyle),
leurs stéréoisomères et leurs sels d'addition.

6. Composés de formule I, selon la revendication 1, dans laquelle :
- R₁ représente un atome d'hydrogène ou un radical alkyle de 1 à 5 atomes de carbone,
- R₂ représente un radical de formule (C),
- R₃ représente un radical alkyle de 1 à 5 atomes de carbone,
- R₄ représente un radical cycloalkyle de 3 à 6 atomes de carbone,
et,
- R₅ représente un radical de formule (D), un radical 3-hydroxyalkylpyrid-6-yle ou un radical 2-hydroxyalkylpyrid-5-yle,
leurs stéréoisomères et leurs sels d'addition.

7. Composés de formule I, selon la revendication 1, dans laquelle,
- R₁ représente un atome d'hydrogène ou un radical alkyle de 1 à 5 atomes de carbone,
- R₂ représente un radical de formule (C),
- R₃ représente un radical alkyle de 1 à 5 atomes de carbone,
- R₄ un radical hydroxyalkyle de 1 à 5 atomes de carbone, un radical alcoxyalkyle de 2 à 10 atomes de carbone, un radical cycloalkyloxyalkyle de 4 à 11 atomes de carbone, un radical hydroxyalkyloxyalkyle de 2 à 10 atomes de carbone, un radical alcoxyalkyloxyalkyle de 3 à 12 atomes de carbone, un radical acyloxyalkyle de 3 à 11 atomes de carbone ou un radical alkylthioalkyle de 2 à 10 atomes de carbone,
et,
- R₅ représente un radical cycloalkyle de 3 à 6 atomes de carbone, un radical phényle, un radical thiényle ou un radical pyridyle (éventuellement substitués par un ou plusieurs atomes d'halogène, par des radicaux alcoxy de 1 à 5 atomes de carbone, des radicaux alkyle de 1 à 5 atomes de carbone ou des radicaux trifluorométhyle),
leurs stéréoisomères et leurs sels d'addition.

8. Composés de formule I, selon la revendication 1, dans laquelle,
- R₁ représente un atome d'hydrogène ou un radical alkyle de 1 à 5 atomes de carbone,
- R₂ représente un radical de formule (C),
- R₃ représente un radical hydroxyalkyle de 1 à 5 atomes de carbone, un radical tetrahydropyran-2-yloxyalkyle dont le radical alkyle comporte de 1 à 5 atomes de carbone, un radical alcoxyalkyle de 2 à 10 atomes de carbone ou un radical acyloxyalkyle de 3 à 11 atomes de carbone,
- R₄ représente un radical cycloalkyle de 3 à 6 atomes de carbone
et,
- R₅ représente un radical cycloalkyle de 3 à 6 atomes de carbone, un radical phényle, un radical thiényle ou un radical pyridyle (éventuellement substitués par un ou plusieurs atomes d'halogène, par des radicaux alcoxy de 1 à 5 atomes de carbone, des radicaux alkyle de 1 à 5 atomes de carbone ou des radicaux trifluorométhyle),
leurs stéréoisomères et leurs sels d'addition.

9. Procédé de préparation des composés de formule I, selon la revendication 1, caractérisé en ce que l'on fait réagir un dérivé carbonylé alpha-halogéné, de préférence alpha-bromé, de formule II : 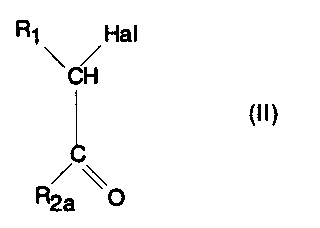 dans laquelle,
- R₁ a la même signification que pour la formule I, selon la revendication 1,
- R₂ₐ représente un radical de formule (Aₐ) : 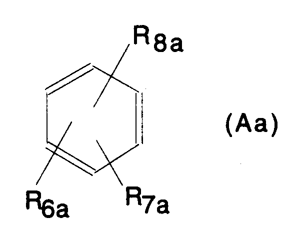 (dans laquelle R₆ₐ représente un atome de brome ou d'iode, et R₇ₐ et R₈ₐ, identiques ou différents, représentent chacun un atome d'hydrogène ou un atome d'halogène), un radical de formule (B) ou un radical de formule (C), les radicaux (B) et (C) ayant la même signification que pour la formule I, selon la revendication 1,
et,
- Hal représente un atome d'halogène, de préférence de brome,
soit
avec une thiourée de formule IIIₐ : 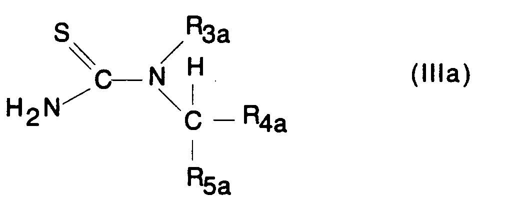 dans laquelle,
- R₃ₐ représente un radical alkyle de 1 à 5 atomes de carbone,
- R₄ₐ représente un radical cycloalkyle de 3 à 6 atomes de carbone, ou un radical de formule (F) :
- Alk - O - ProtecA (F)
(dans laquelle Alk représente un radical alkyle de 1 à 5 atomes de carbone et ProtecA, un groupe protecteur éliminable par hydrolyse acide), un radical alcoxyalkyle de 2 à 10 atomes de carbone ou un radical alkylthioalkyle de 2 à 10 atomes de carbone,
- R₅ₐ représente un radical cycloalkyle de 3 à 6 atomes de carbone, un radical phényle ou un radical pyridyle (éventuellement substitués par un ou plusieurs atomes d'halogène, par des radicaux alcoxy de 1 à 5 atomes de carbone, des radicaux alkyle de 1 à 5 atomes de carbone ou par des radicaux trifluorométhyle), un radical 3-hydroxyalkylpyrid-6-yle, un radical 2-hydroxy alkylpyrid-5-yle ou un radical de formule (D), selon la revendication 1, dans laquelle R₁₄ représente un radical hydroxyalkyle de 2 à 5 atomes de carbone,
pour former des composés de formule Ia : 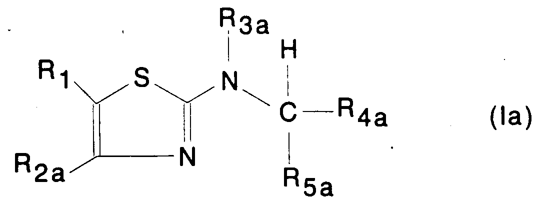 dans laquelle,
- R₁ a la même signification que pour la formule I, selon la revendication 1,
- R₂ₐ a la signification donnée pour la formule II,
et
- R₃ₐ, R₄ₐ et R₅ₐ ont la signification donnée pour la formule IIIa,
soit
avec une thiourée de formule III_{b} : 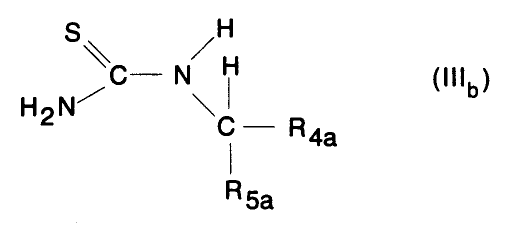 dans laquelle,
- R₄ₐ et R₅ₐ ont les mêmes significations que pour la formule IIIₐ. pour former les composés de formule IV : 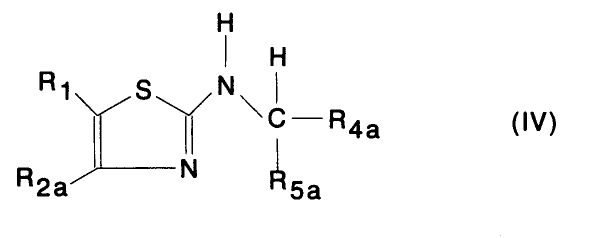 dans laquelle,
- R₁ a la signification donnée pour la formule I, selon la revendication 1,
- R₂ₐ a la signification donnée pour la formule II,
et
- R₄ₐ et R₅ₐ, les significations données pour la formule IIIₐ,
que l'on fait réagir avec un halogénure de formule V :
Hal-R_{3b} (V)
dans laquelle,
- Hal représente un atome d'halogène
et,
- R_{3b} représente un radical alkyle de 1 à 5 atomes de carbone ou un radical de formule (F),
pour former les composés de formule Ib : 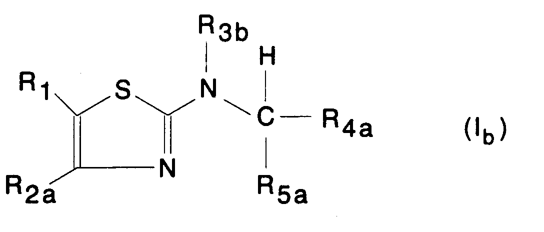 dans laquelle,
- R₁ a la signification donnée pour la formule I, selon la revendication 1,
- R₂ₐ a la signification donnée pour la formule II,
- R₄ₐ et R₅ₐ, les significations données pour la formule IIIₐ et,
- R_{3b} a la signification donnée pour la formule V,
et ensuite, on réalise l'une des étapes a) à d) suivantes :
(a) l'on soumet les composés de formules la et Ib dans lesquelles R₂ₐ représente un radical de formule (Aₐ) :
* soit à l'action du *tert*-butyllithium et d'anhydride carbonique et ensuite à une réduction pour obtenir les composés de formule I, selon la revendication 1, dans laquelle R₂ représente un radical de formule (A), dont le substituant R₆ et éventuellement les substituants R₇ et/ou R₈ représentent un radical hydroxyméthyle,
* soit à l'action du *tert*-butyllithium et d'un aldéhyde aliphatique (C₂-C₅) pour obtenir les composés de formule I, selon la revendication 1, dans laquelle R₂ représente un radical de formule (A) dont le substituant R₆ et éventuellement les substituants R₇ et/ou R₈ représentent un radical hydroxyalkyle linéaire ou ramifié de 2 à 5 atomes de carbone,
ou,
(b)
* soit
l'on soumet les composés de formule Iₐ et I_{b} dans lesquelles R₅ₐ représente un radical 4-bromophényle :
ou,
à l'action du *tert*-butyllithium et d'anhydride carbonique pour obtenir les composés de formule I, selon la revendication 1, dans laquelle R₅ représente un radical 4-carboxyphényle, à partir desquels sont ensuite obtenus :
- par estérification, les composés de formule I, selon la revendication 1, dans laquelle R₅ représente un radical 4-(alcoxycarbonyl) phényle,
- par réduction, les composés de formule I, selon la revendication 1, dans laquelle R₅ représente un radical 4-(hydroxyméthyl) phényle,
- par réduction et ensuite estérification, les composés de formule I, selon la revendication 1, dans laquelle R₅ représente un radical 4-(acyloxyméthyl) phényle,
- par réduction et ensuite action d'un halogénure d'alkyle ou d'un halogénure d'aralkyle, les composés de formule I, selon la revendication 1, dans laquelle R₅ représente un radical 4-(alcoxyméthyl) phényle ou 4-(aralcoxyméthyl) phényle,
- par réduction et ensuite par action de chlorhydrine sulfurique, les composés de formule I, selon la revendication 1, dans laquelle R₅ représente un radical 4-(sulfoxyméthyl) phényle,
- par réduction, action du chlorure de 3-chlorométhylbenzoyle puis de la N-méthylpipérazine, les composés de formule I, selon la revendication 1, dans laquelle R₅ représente un radical de formule (D) et R₁₄ est un radical de formule (E),
- par réduction puis action d'un halogène, les composés de formule I, selon la revendication 1, dans laquelle R₅ représente un radical 4-(halogénométhyl) phényle,
ou,
à l'action du tert-butyllithium et à l'action
- d'un aldéhyde aliphatique, pour obtenir les composés de formule I, selon la revendication 1, dans laquelle R₅ représente un radical 4-(*sec*-hydroxyalkyl) phényle,
- d'une cétone aliphatique, pour obtenir les composés de formule I, selon la revendication 1, dans laquelle R₅ représente un radical 4-(*tert*-hydroxyalkyl) phényle,
* soit
l'on soumet les composés de formule Iₐ et I_{b} dans lesquelles R₅ₐ représente un radical de formule (D) dans laquelle R₁₄ représente un radical hydroxyalkyle de 2 à 5 atomes de carbone :
- à une estérification pour obtenir les composés de formule I, selon la revendication 1, dans laquelle R₅ représente un radical 4-(acyloxyalkyl) phényle,
- à une alkylation pour obtenir les composés de formule I, selon la revendication 1, dans laquelle R₅ représente un radical 4-(alcoxyalkyl) phényle ou 4-(aralcoxyalkyl) phényle,
- à l'action de chlorhydrine sulfurique pour obtenir les composés de formule I, selon la revendication 1, dans laquelle R₅ représente un radical 4-(sulfoxyalkyl) phényle,
- à l'action d'un halogène pour obtenir les composés de formule I, selon la revendication 1, dans laquelle R₅ représente un radical 4-(halogénoalkyl) phényle,
(c) ou,
l'on soumet les composés de formule Iₐ et I_{b} dans lesquelles R₄ₐ représente un radical de formule (F) à une déprotection, pour obtenir les composés de formule I, selon la revendication 1, dans laquelle R₄ représente un radical hydroxyalkyle, à partir desquels sont ensuite obtenus :
- par estérification, les composés de formule I, selon la revendication 1, dans laquelle R₄ représente un radical acyloxyalkyle,
- par alkylation, les composés de formule I, selon la revendication 1, dans laquelle R₄ représente un radical alcoxyalkyle ou cycloalkyloxyalkyle,
- par alkylation avec des alcools aliphatiques halogénés protégés puis déprotection, les composés de formule I, selon la revendication 1, dans laquelle R₄ représente un radical hydroxyalkyloxyalkyle,
- par alkylation avec des alcools aliphatiques halogénés protégés, puis déprotection et ensuite alkylation, les composés de formule I, selon la revendication 1, dans laquelle R₄ représente un radical alcoxyalkyloxyalkyle,
(d) ou,
l'on soumet les produits de formule I_{b} dans laquelle R_{3b} représente un radical de formule (F) à une déprotection par hydrolyse acide, pour obtenir les composés de formule I, selon la revendication 1, dans laquelle R₃ représente un radical hydroxyalkyle, à partir desquels sont obtenus ensuite par alkylation ou par estérification, les composés de formule I, selon la revendication 1, dans laquelle R₃ représente respectivement un radical alcoxyalkyle, ou un radical acyloxyalkyle,
et le cas échéant. les composés de formule I, selon la revendication 1,
sont ensuite séparés en leurs stéréoisomères possibles et/ou salifiés, pour former les sels correspondants.

10. Utilisation des composés de formule I, selon l'une quelconque des revendications 1 à 8, pour la préparation d'un médicament utilisable dans le traitement des maladies nécessitant une modulation de l'action du facteur de libération de l'hormone corticotrope (CRF).

11. Composition pharmaceutique contenant comme principe actif au moins un composé selon l'une quelconque des revendications 1 à 8, sous forme de base ou sous forme de sel avec un acide minéral ou organique pharmaceutiquement acceptable, en association ou en mélange avec un excipient inerte, non toxique, pharmaceutiquement acceptable.

## Claims

1. Compounds of formula I: 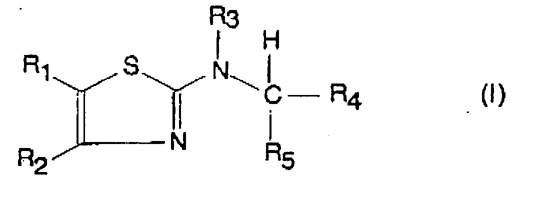 wherein
- R₁ denotes a hydrogen atom or a C₁₋₅-alkyl radical,
- R₂ denotes a radical of formula (A): 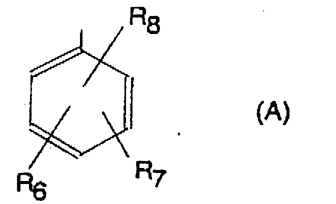 (wherein R₆ denotes a C₁₋₅-hydroxyalkyl radical and R₇ and R₈, which may be identical or different, each denote a hydrogen atom, a halogen atom or a C₁₋₅-hydroxyalkyl radical, a trifluoromethyl, a C₁₋₅-alkoxy radical or a C₁₋₅-alkyl radical),
a radical of formula (B): 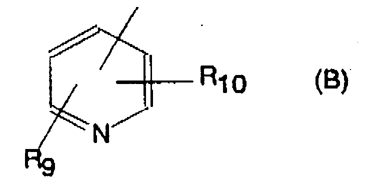 (wherein R₉ and R₁₀, which may be identical or different, each denote a hydrogen atom, a halogen atom, a C₁₋₅-alkyl radical or a C₁₋₅-alkoxy radical),
or a radical of formula (C): 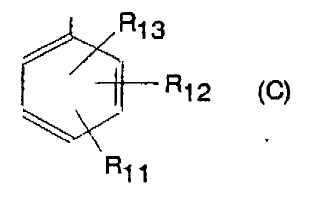 (wherein R₁₁, R₁₂ and R₁₃, which may be identical or different, each denote a hydrogen atom, a halogen atom, a trifluoromethyl, a C₁₋₅-alkoxy radical or a C₁₋₅-alkyl radical),
- R₃ denotes a C₁₋₅-alkyl radical, a C₁₋₅-hydroxyalkyl radical, a tetrahydropyran-2-yloxyalkyl radical wherein the alkyl moiety comprises 1 to 5 carbon atoms, a C₂₋₁₀-alkoxyalkyl radical or a C₃₋₁₁-acyloxyalkyl radical,
- R₄ denotes a C₃₋₆-cycloalkyl radical, a C₁₋₅-hydroxyalkyl radical, a C₂₋₁₀-alkoxyalkyl radical, a C₄₋₁₁-cycloalkyloxyalkyl radical, a C₂₋₁₀-hydroxyalkyloxyalkyl, a C₃₋₁₂-alkoxyalkyloxyalkyl radical, a C₃₋₁₁-acyloxyalkyl radical or a C₂₋₁₀-alkylthioalkyl radical,
and
- R₅ denotes a C₃₋₆-cycloalkyl radical, a phenyl radical, a thienyl radical or a pyridyl radical (optionally substituted by one or more halogen atoms, by C₁₋₅-alkoxy radicals, C₁₋₅-alkyl radicals or trifluoromethyl radicals), or a radical of formula (D): 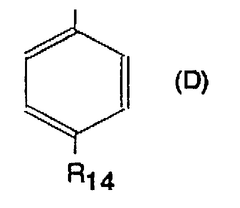 (wherein R₁₄ denotes a carboxy radical, a C₂₋₆-carboxyalkyl radical, a C₂₋₆-alkoxycarbonyl radical, a C₃₋₁₁-acyloxyalkyl radical, a C₂₋₁₀-alkoxyalkyl radical, a C₈₋₁₆-aralkoxyalkyl radical (optionally substituted on the aromatic ring by one or more halogen atoms, C₁₋₃-alkoxy radicals or trifluoromethyl radicals), a monohalogenated C₁₋₅-alkyl radical, a straight-chained or branched C₁₋₅-hydroxyalkyl radical, a radical of formula (E): 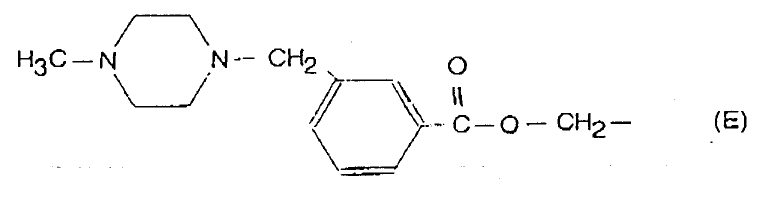 or a C₁₋₅-sulphoxyalkyl radical),
a 3-hydroxyalkylpyrid-6-yl radical or a 2-hydroxyalkylpyrid-5-yl radical (wherein the alkyl radicals contain 1 to 5 carbon atoms),
with the proviso, however, that, if R₃ denotes a C₁₋₅-alkyl radical, R₄ denotes a cycloalkyl radical and R₅ denotes either a cycloalkyl radical or a phenyl radical, a thienyl radical or a pyridyl radical (optionally substituted by one or more halogen atoms, by C₁₋₅-alkoxy radicals, C₁₋₅-alkyl radicals or trifluoromethyl radicals), R₂ does not represent a radical of formula (C),
the stereoisomers thereof and the addition salts thereof.

2. Compounds of formula I according to claim 1, corresponding to formula I': 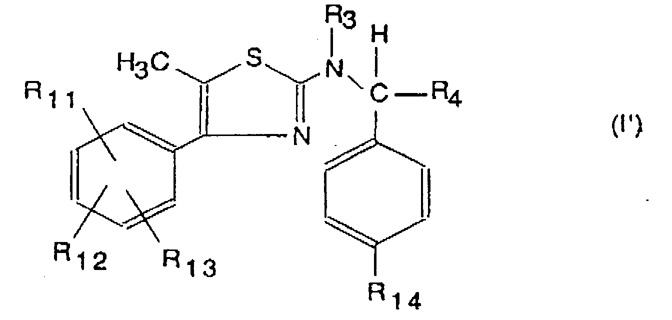 wherein
- R₁₁ denotes a halogen atom and R₁₂ and R₁₃ have the same meanings as in formula I according to claim 1,
- R₃ denotes a C₁₋₅-alkyl radical,
- R₄ denotes a C₃₋₆-cycloalkyl radical and
- R₁₄ has the same meanings as in formula I according to claim 1,
the stereoisomers thereof and the addition salts thereof.

3. Compounds of formula I according to claim 1 corresponding to the formula I'': 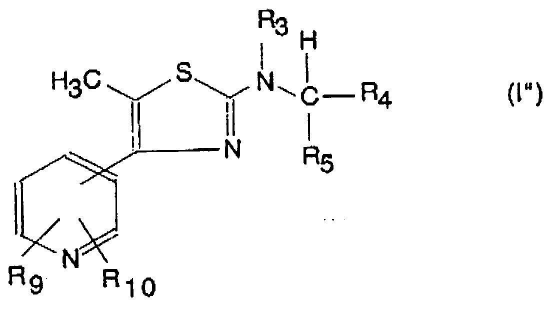 wherein
- R₃, R₄ and R₅, R₉ and R₁₀ are defined as for formula I according to claim 1,
the stereoisomers thereof and the addition salts thereof.

4. Compounds of formula I according to claim 1, corresponding to the formula I''': 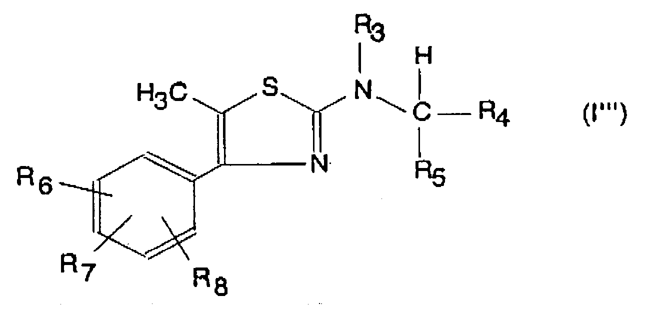 wherein
- R₇ denotes a halogen atom or a C₁₋₅-alkoxy,
- R₆ and R₈ are defined as for formula I according to claim 1,
- R₃ denotes a C₁₋₅-alkyl radical, and
- R₄ and R₅ have the meanings given for formula I according to claim 1,
the stereoisomers thereof and the addition salts thereof.

5. Compounds of formula I according to claim 1 wherein:
- R₁ denotes a hydrogen atom or a C₁₋₅-alkyl radical,
- R₂ denotes a radical of formula (A) or a radical of formula (B),
- R₃ denotes a C₁₋₅-alkyl radical,
- R₄ denotes a C₃₋₆-cycloalkyl radical, and
- R₅ denotes a C₃₋₆-cycloalkyl radical, a phenyl radical, a thienyl radical or a pyridyl radical (optionally substituted by one or more halogen atoms, by C₁₋₅-alkoxy radicals, C₁₋₅-alkyl radicals or trifluoromethyl radicals),
the stereoisomers thereof and the addition salts thereof.

6. Compounds of formula I according to claim 1, wherein:
- R₁ denotes a hydrogen atom or a C₁₋₅-alkyl radical,
- R₂ denotes a radical of formula (C),
- R₃ denotes a C₁₋₅-alkyl radical,
- R₄ denotes a C₃₋₆-cycloalkyl radical, and
- R₅ denotes a radical of formula (D), a 3-hydroxyalkylpyrid-6-yl radical or a 2-hydroxyalkylpyrid-5-yl radical,
the stereoisomers thereof and the addition salts thereof.

7. Compounds of formula I according to claim 1, wherein:
- R₁ denotes a hydrogen atom or a C₁₋₅-alkyl radical,
- R₂ denotes a radical of formula (C),
- R₃ denotes a C₁₋₅-alkyl radical,
- R₄ denotes a C₁₋₅-hydroxyalkyl radical, a C₂₋₁₀-alkoxyalkyl radical, a C₄₋₁₁-cycloalkyloxyalkyl radical, a C₂₋₁₀-hydroxyalkyloxyalkyl radical, a C₃₋₁₂-alkoxyalkyloxyalkyl radical, a C₃₋₁₁-acyloxyalkyl radical or a C₂₋₁₀-alkylthioalkyl radical,
and
- R₅ denotes a C₃₋₆-cycloalkyl radical, a phenyl radical, a thienyl radical or a pyridyl radical (optionally substituted by one or more halogen atoms, by C₁₋₅-alkoxy radicals, C₁₋₅-alkyl radicals or trifluoromethyl radicals),
the stereoisomere thereof and the addition salts thereof.

8. Compounds of formula I according to claim 1, wherein
- R₁ denotes a hydrogen atom or a C₁₋₅-alkyl radical,
- R₂ denotes a radical of formula (C),
- R₃ denotes a C₁₋₅-hydroxyalkyl radical, a tetrahydropyran-2-yloxyalkyl radical wherein the alkyl radical contains 1 to 5 carbon atoms, a C₂₋₁₀-alkoxyalkyl radical or a C₃₋₁₁-acyloxyalkyl radical,
- R₄ denotes a C₃₋₆-cycloalkyl radical
and
- R₅ denotes a C₃₋₆-cycloalkyl radical, a phenyl radical, a thienyl radical or a pyridyl radical (optionally substituted by one or more halogen atoms, by C₁₋₅-alkoxy radicals, C₁₋₅-alkyl radicals or trifluoromethyl radicals),
the stereoisomers thereof and the addition salts thereof.

9. Process for preparing compounds of formula I according to claim 1, characterised in that an alpha-halogenated, preferably alpha-brominated, carbonyl derivative of formula II: 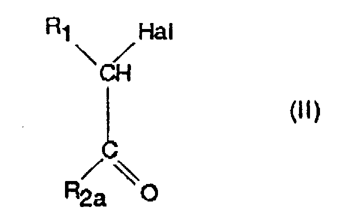 wherein
- R₁ is as defined for formula I, according to claim 1,
- R₂ₐ denotes a radical of formula (Aₐ): 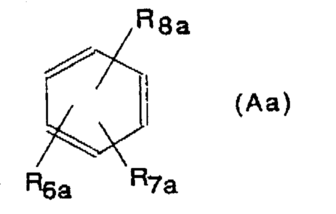 (wherein R₆ₐ denotes a bromine or iodine atom and R₇ₐ and R₈ₐ, which may be identical or different, each denote a hydrogen atom or a halogen atom), a radical of formula (B) or a radical of formula (C), radicals (B) and (C) having the same meaning as in formula I according to claim 1,
and
- Hal denotes a halogen atom, preferably bromine, is reacted
either
with a thiourea of formula IIIₐ: 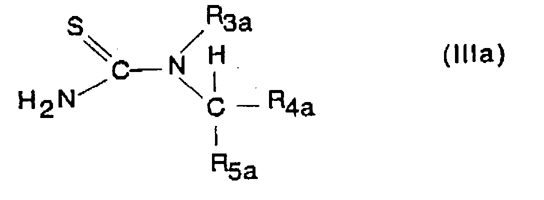 wherein
- R₃ₐ denotes a C₁₋₅-alkyl radical,
- R₄ₐ denotes a C₃₋₆-cycloalkyl radical, or a radical of formula (F):
- Alk - O - ProtecA (F)
(wherein Alk denotes a C₁₋₅-alkyl radical and ProtecA denotes a protecting group which can be removed by acidic hydrolysis), a C₂₋₁₀-alkoxyalkyl radical or a C₂₋₁₀-alkylthioalkyl radical,
- R₅ₐ denotes a C₃₋₆-cycloalkyl radical, a phenyl radical or a pyridyl radical (optionally substituted by one or more halogen atoms, by C₁₋₅-alkoxy radicals, C₁₋₅-alkyl radicals or trifluoromethyl radicals), a 3-hydroxyalkylpyrid-6-yl radical, a 2-hydroxyalkylpyrid-5-yl radical or a radical of formula (D) according to claim 1, wherein R₁₄ denotes a C₂₋₅-hydroxyalkyl radical,
to form compounds of formula Ia. 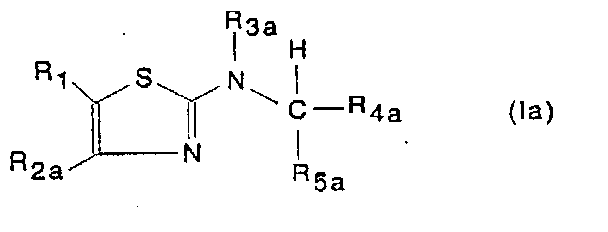
wherein
- R₁ is as defined for formula I according to claim 1,
- R₂ₐ is as defined for formula II,
and
- R₃ₐ, R₄ₐ and R₅ₐ are as defined for formula IIIa,
or
with a thiourea of formula III_{b}: 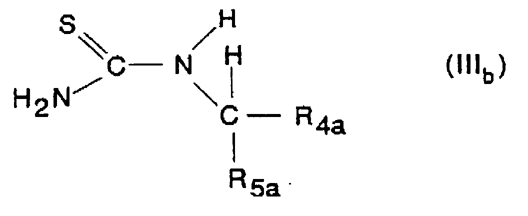 wherein
- R₄ₐ and R₅ₐ have the same meanings as for formula IIIₐ,
to form compounds of formula IV: 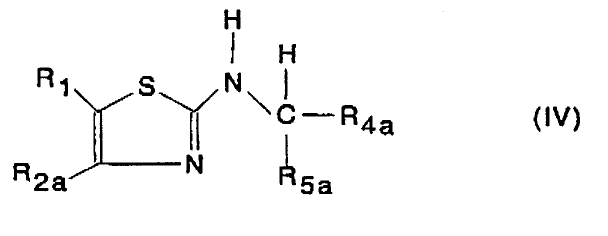 wherein
- R₁ is as defined for formula I according to claim 1,
- R₂ₐ is as defined for formula II
and
- R₄ₐ and R₅ₐ are as defined for formula IIIₐ,
and this compound of formula IV is reacted with a halide of formula V:
Hal-R_{3b} (V)
wherein
- Hal denotes a halogen atom
and
- R_{3b} denotes a C₁₋₅-alkyl radical or a radical of formula (F),
to form compounds of formula Ib: 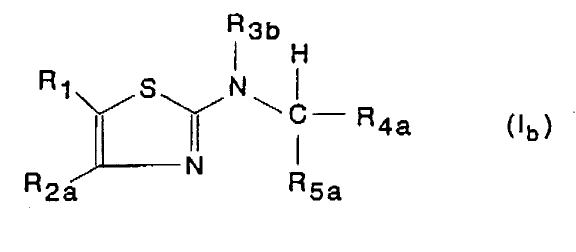 wherein
- R₁ is as defined for formula I according to claim 1,
- R₂ₐ is as defined for formula II,
- R₄ₐ and R₅ₐ are as defined for formula IIIₐ
and
- R_{3b} is as defined for formula V,
and subsequently one of the following steps a) to d) is carried out:
(a) the compounds of formulae Ia and Ib wherein R₂ₐ denotes a radical of formula (Aₐ) are subjected:
* either to the action of *tert*-butyl lithium and carbon dioxide and then to reduction in order to obtain the compounds of formula I according to claim 1 wherein R₂ denotes a radical of formula (A), in which the substituent R₆ and optionally substituents R₇ and/or R₈ denote a hydroxymethyl radical,
* or to the action of *tert*-butyl lithium and an aliphatic aldehyde (C₂₋₅) in order to obtain the compounds of formula I according to claim 1 wherein R₂ denotes a radical of formula (A) in which the substituent R₆ and optionally substituents R₇ and/or R₈ denote a straight-chained or branched hydroxyalkyl radical having 2 carbon atoms,
or
(b)
* either the compounds of formula Iₐ and I_{b} wherein R₅ₐ denotes a 4-bromophenyl radical are subjected;
either
to the action of *tert*-butyl lithium and carbon dioxide in order to obtain the compounds of formula I according to claim 1, wherein R₅ denotes a 4-carboxyphenyl radical, from which the following are subsequently obtained:
- by esterification: the compounds of formula I according to claim 1 wherein R₅ denotes a 4-(alkoxycarbonyl)phenyl radical,
- by reduction: the compounds of formula I according to claim 1 wherein R₅ denotes a 4-(hydroxymethyl)phenyl radical,
- by reduction and then esterification: the compounds of formula I according to claim 1, wherein R₅ denotes a 4-(acyloxymethyl)phenyl radical,
- by reduction followed by the action of an alkyl halide or an aralkyl halide: the compounds of formula I according to claim 1 in which R₅ denotes a 4-(alkoxymethyl)phenyl or 4-(aralkoxymethyl)phenyl radical,
- by reduction and subsequently reaction with chlorosulphonic acid: the compounds of formula I according to claim 1 wherein R₅ denotes a 4-(sulphoxymethyl)phenyl radical,
- by reduction, reaction with 3-chloromethylbenzoyl chloride and then N-methylpiperazine: the compounds of formula I according to claim 1 wherein R₅ denotes a radical of formula (D) and R₁₄ is a radical of formula (E),
- by reduction followed by reaction with a halogen: the compounds of formula I according to claim 1 wherein R₅ denotes a 4-(halomethyl)phenyl radical,
or
to the action of tert-butyl lithium and to the action of
- an aliphatic aldehyde, in order to obtain compounds of formula I according to claim 1 wherein R₅ denotes a 4-(*sec*-hydroxyalkyl)phenyl radical,
- of an aliphatic ketone, to obtain compounds of formula I according to claim 1 wherein R₅ denotes a 4-(*tert*-hydroxyalkyl)phenyl radical,
* or
the compounds of formula Iₐ and I_{b} wherein R₅ₐ denotes a radical of formula (D) in which R₁₄ denotes a C₂₋₅-hydroxyalkyl radical are subjected:
- to esterification in order to obtain the compounds of formula I according to claim 1 wherein R₅ denotes a 4-(acyloxyalkyl)phenyl radical,
- to alkylation in order to obtain the compounds of formula I according to claim 1, wherein R₅ denotes a 4-(alkoxyalkyl)phenyl or 4-(aralkoxyalkyl)phenyl radical,
- to the action of chlorosulphonic acid in order to obtain compounds of formula I according to claim 1 wherein R₅ denotes a 4-(sulphoxyalkyl)phenyl radical,
- to the action of a halogen in order to obtain compounds of formula I according to claim 1 wherein R₅ denotes a 4-(haloalkyl)phenyl radical,
(c) or
the compounds of formula Iₐ and I_{b} wherein R₄ₐ denotes a radical of formula (F) are subjected to deprotection in order to obtain the compounds of formula I according to claim 1 wherein R₄ denotes a hydroxyalkyl radical, from which are then obtained:
- by esterification: the compounds of formula I according to claim 1 wherein R₄ denotes an acyloxyalkyl radical,
- by alkylation: the compounds of formula I according to claim 1 wherein R₄ denotes an alkoxyalkyl or cycloalkyloxyalkyl radical,
- by alkylation with protected halogenated aliphatic alcohols followed by deprotection: the compounds of formula I according to claim 1 wherein R₄ denotes a hydroxyalkyloxyalkyl radical,
- by alkylation with protected halogenated aliphatic alcohols followed by deprotection and then alkylation: the compounds of formula I according to claim 1 wherein R₄ denotes an alkoxyalkyloxyalkyl radical,
(d) or
the products of formula I_{b} wherein R_{3b} denotes a radical of formula (F) are subjected to deprotection by acid hydrolysis, in order to obtain the compounds of formula I according to claim 1 wherein R₃ denotes a hydroxyalkyl radical, from which are then obtained, by alkylation or esterification, the compounds of formula I according to claim 1 wherein R₃ denotes, respectively, an alkoxyalkyl radical or an acyloxyalkyl radical,
and if applicable, the compounds of formula I according to claim 1
are then separated into their possible stereoisomers and/or salified to form the corresponding salts.

10. Use of the compounds of formula I according to any one of claims 1 to 8 for preparing a medicament which may be used in the treatment of diseases requiring modification of the action of corticotropin releasing factor (CRF).

11. Pharmaceutical compositions containing as active principle at least one compound according to any one of claims 1 to 8 in the form of a base or in the form of a salt with a pharmaceutically acceptable organic or inorganic acid, combined or mixed with a non-toxic pharmaceutically acceptable inert excipient.

## Patentansprüche

1. Verbindungen der Formel I: 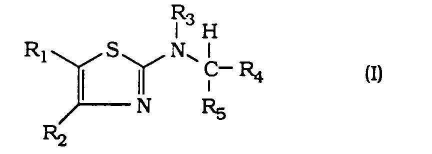 in der
- R₁ ein Wasserstoffatom oder eine Alkylgruppe mit 1 bis 5 Kohlenstoffatomen,
- R₂ eine Gruppe der Formel (A): 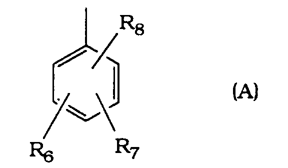 (in der R₆ eine Hydroxyalkylgruppe mit 1 bis 5 Kohlenstoffatomen und R₆ und R₇, die gleichartig oder verschieden sind, jeweils ein Wasserstoffatom, ein Halogenatom, eine Hydroxyalkylgruppe mit 1 bis 5 Kohlenstoffatomen, eine Trifluormethylgruppe, eine Alkoxygruppe mit 1 bis 5 Kohlenstoffatomen oder eine Alkylgruppe mit 1 bis 5 Kohlenstoffatomen darstellen),
eine Gruppe der Formel (B): 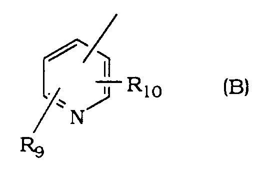 (in der R₉ und R₁₀, die gleichartig oder verschieden sind, jeweils ein Wasserstoffatom, ein Halogenatom, eine Alkylgruppe mit 1 bis 5 Kohlenstoffatomen oder eine Alkoxygruppe mit 1 bis 5 Kohlenstoffatomen darstellen),
oder eine Gruppe der Formel (C): 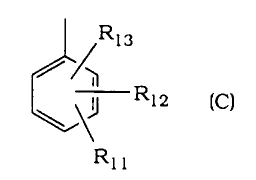 (in der R₁₁, R₁₂ und R₁₃, die gleichartig oder verschieden sind, jeweils ein Wasserstoffatom, ein Halogenatom, eine Trifluormethylgruppe, eine Alkoxygruppe mit 1 bis 5 Kohlenstoffatomen oder eine Alkylgruppe mit 1 bis 5 Kohlenstoffatomen darstellen),
- R₃ eine Alkylgruppe mit 1 bis 5 Kohlenstoffatomen, eine Hydroxyalkylgruppe mit 1 bis 5 Kohlenstoffatomen, eine Tetrahydropyran-2-yloxyalkylgruppe, deren Alkylrest 1 bis 5 Kohlenstoffatome aufweist, eine Alkoxyalkylgruppe mit 2 bis 10 Kohlenstoffatomen oder eine Acyloxyalkylgruppe mit 3 bis 11 Kohlenstoffatomen,
- R₄ eine Cycloalkylgruppe mit 3 bis 6 Kohlenstoffatomen, eine Hydroxyalkylgruppe mit 1 bis 5 Kohlenstoffatomen, eine Alkoxyalkylgruppe mit 2 bis 10 Kohlenstoffatomen, eine Cycloalkyloxyalkylgruppe mit 4 bis 11 Kohlenstoffatomen, eine Hydroxyalkyloxyalkylgruppe mit 2 bis 10 Kohlenstoffatomen, eine Alkoxyalkyloxyalkylgruppe mit 3 bis 12 Kohlenstoffatomen, eine Acyloxyalkylgruppe mit 3 bis 11 Kohlenstoffatomen oder eine Alkylthioalkylgruppe mit 2 bis 10 Kohlenstoffatomen und
- R₅ eine Cycloalkylgruppe mit 3 bis 6 Kohlenstoffatomen, eine Phenylgruppe, eine Thienylgruppe oder eine Pyridylgruppe (die gegebenenfalls mit einem oder mehreren Halogenatomen, Alkoxygruppen mit 1 bis 5 Kohlenstoffatomen, Alkylgruppen mit 1 bis 5 Kohlenstoffatomen oder Trifluormethylgruppen substituiert sind) oder eine Gruppe der Formel (D): 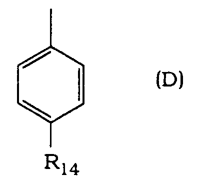 (in der R₁₄ eine Carboxygruppe, eine Carboxyalkylgruppe mit 2 bis 6 Kohlenstoffatomen, eine Alkoxycarbonylgruppe mit 2 bis 6 Kohlenstoffatomen, eine Acyloxyalkylgruppe mit 3 bis 11 Kohlenstoffatomen, eine Alkoxyalkylgruppe mit 2 bis 10 Kohlenstoffatomen, eine Aralkoxyalkylgruppe mit 8 bis 16 Kohlenstoffatomen (die gegebenenfalls am aromatischen Ring durch eines oder mehrere Halogenatome, Alkoxygruppen mit 1 bis 3 Kohlenstoffatomen oder Trifluormethylgruppen substituiert ist), eine mono-halogenierte Alkylgruppe mit 1 bis 5 Kohlenstoffatomen, eine geradkettige oder verzweigte Hydroxalkylgruppe mit 1 bis 5 Kohlenstoffatomen, eine Gruppe der Formel (E): 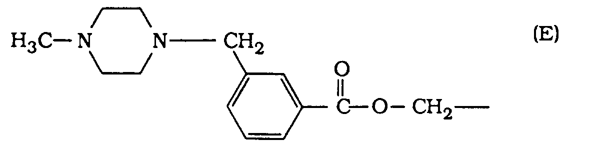 oder eine Sulfoxyalkylgruppe mit 1 bis 5 Kohlenstoffatomen darstellt), eine 3-Hydroxyalkylpyrid-6-yl-gruppe oder eine 2-Hydroxyalkylpyrid-5-yl-gruppe (deren Alkylgruppen 1 bis 5 Kohlenstoffatome aufweisen), mit der Maßgabe bedeuten, daß, wenn R₃ eine Alkylgruppe mit 1 bis 5 Kohlenstoffatomen darstellt, R₄ eine Cycloalkylgruppe und R₅ entweder eine Cycloalkylgruppe oder eine Phenylgruppe, eine Thienylgruppe oder eine Pyridylgruppe (die gegebenenfalls durch eines oder mehrere Halogenatome, Alkoxygruppen mit 1 bis 5 Kohlenstoffatomen, Alkylgruppen mit 1 bis 5 Kohlenstoffatomen oder Trifluormethylgruppen substituiert sind) bedeuten und R₂ keine Gruppe der Formel (C) darstellt,
deren Stereoisomere und Additionssalze.

2. Verbindungen der Formel (I) nach Anspruch 1 der Formel (I'): 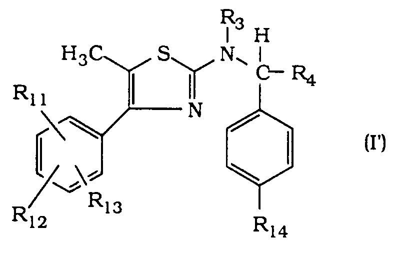 in der
- R₁₁ ein Halogenatom darstellt, R₁₂ und R₁₃ die bezüglich der Formel I in Anspruch 1 angegebenen Bedeutungen besitzen,
- R₃ eine Alkylgruppe mit 1 bis 5 Kohlenstoffatomen darstellt,
- R₄ eine Cycloalkylgruppe mit 3 bis 5 Kohlenstoffatomen darstellt und
- R₁₄ die in Anspruch 1 bezüglich der Formel I angegebenen Bedeutungen besitzt,
deren Stereoisomere und deren Additionssalze.

3. Verbindungen der Formel I nach Anspruch 1 der Formel I'': 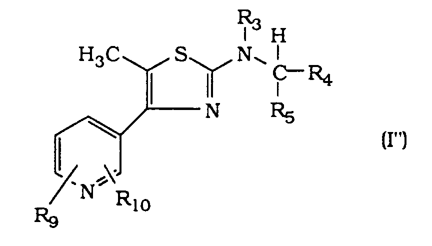 in der
- R₃, R₄ und R₅, R₉ und R₁₀ die bezüglich der Formel I in Anspruch 1 angegebenen Bedeutungen besitzen,
deren Stereoisomere und deren Additionssalze.

4. Verbindungen der Formel I nach Anspruch 1 der Formel I''': 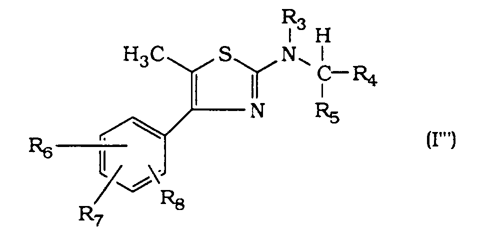 in der
- R₇ ein Halogenatom oder eine C₁-C₅-Alkoxygruppe darstellt, R₆ und R₈ die bezüglich der Formel I in Anspruch 1 angegebenen Bedeutungen besitzen,
- R₃ eine Alkylgruppe mit 1 bis 5 Kohlenstoffatomen darstellt und
- R₄ und R₅ die bezüglich der Formel I in Anspruch 1 angegebenen Bedeutungen besitzen,
deren Stereoisomere und deren Additionssalze.

5. Verbindungen der Formel I nach Anspruch 1, in der
- R₁ ein Wasserstoffatom oder eine Alkylgruppe mit 1 bis 5 Kohlenstoffatomen,
- R₂ eine Gruppe der Formel (A) oder eine Gruppe der Formel (B),
- R₃ eine Alkylgruppe mit 1 bis 5 Kohlenstoffatomen,
- R₄ eine Cycloalkylgruppe mit 3 bis 6 Kohlenstoffatomen und
- R₅ eine Cycloalkylgruppe mit 3 bis 6 Kohlenstoffatomen, eine Phenylgruppe, eine Thienylgruppe oder eine Pyridylgruppe (die gegebenenfalls durch eines oder mehrere Halogenatome, Alkoxygruppen mit 1 bis 5 Kohlenstoffatomen, Alkylgruppen mit 1 bis 5 Kohlenstoffatomen oder Trifluormethylgruppen substituiert sind) bedeuten,
deren Stereoisomere und deren Additionssalze.

6. Verbindungen der Formel I nach Anspruch 1, in der
- R₁ ein Wasserstoffatom oder eine Alkylgruppe mit 1 bis 5 Kohlenstoffatomen,
- R₂ eine Gruppe der Formel (C),
- R₃ eine Alkylgruppe mit 1 bis 5 Kohlenstoffatomen,
- R₄ eine Cycloalkylgruppe mit 3 bis 6 Kohlenstoffatomen und
- R₅ eine Gruppe der Formel (D), eine 3-Hydroxyalkylpyrid-6-yl-gruppe oder eine 2-Hydroxyalkylpyrid-5-yl-gruppe bedeuten,
deren Stereoisomere und deren Additionssalze.

7. Verbindungen der Formel I nach Anspruch 1, in der
- R₁ ein Wasserstoffatom oder eine Alkylgruppe mit 1 bis 5 Kohlenstoffatomen,
- R₂ eine Gruppe der Formel (C),
- R₃ eine Alkylgruppe mit 1 bis 5 Kohlenstoffatomen,
- R₄ eine Hydroxyalkylgruppe mit 1 bis 5 Kohlenstoffatomen, eine Alkoxyalkylgruppe mit 2 bis 10 Kohlenstoffatomen, eine Cycloalkyloxyalkylgruppe mit 4 bis 11 Kohlenstoffatomen, eine Hydroxyalkyloxyalkylgruppe mit 2 bis 10 Kohlenstoffatomen, eine Alkoxyalkyloxyalkylgruppe mit 3 bis 12 Kohlenstoffatomen, eine Acyloxyalkylgruppe mit 3 bis 11 Kohlenstoffatomen oder eine Alkylthioalkylgruppe mit 2 bis 10 Kohlenstoffatomen und
- R₅ eine Cycloalkylgruppe mit 3 bis 6 Kohlenstoffatomen, eine Phenylgruppe, eine Thienylgruppe oder eine Pyridylgruppe (die gegebenenfalls durch eines oder mehrere Halogenatome, Alkoxygruppen mit 1 bis 5 Kohlenstoffatomen, Alkylgruppen mit 1 bis 5 Kohlenstoffatomen oder Trifluormethylgruppen substituiert sind) bedeuten,
deren Stereoisomere und deren Additionssalze.

8. Verbindungen der Formel I nach Anspruch 1, in der
- R₁ ein Wasserstoffatom oder eine Alkylgruppe mit 1 bis 5 Kohlenstoffatomen,
- R₂ eine Gruppe der Formel (C),
- R₃ eine Hydroxyalkylgruppe mit 1 bis 5 Kohlenstoffatomen, eine Tetrahydropyran-2-yloxyalkylgruppe, deren Alkylrest 1 bis 5 Kohlenstoffatome aufweist, eine Alkoxyalkylgruppe mit 2 bis 10 Kohlenstoffatomen oder eine Acyloxyalkylgruppe mit 3 bis 11 Kohlenstoffatomen,
- R₄ eine Cycloalkylgruppe mit 3 bis 6 Kohlenstoffatomen und
- R₅ eine Cycloalkylgruppe mit 3 bis 6 Kohlenstoffatomen, eine Phenylgruppe, eine Thienylgruppe oder eine Pyridylgruppe (die gegebenenfalls durch eines oder mehrere Halogenatome, Alkoxygruppen mit 1 bis 5 Kohlenstoffatomen, Alkylgruppen mit 1 bis 5 Kohlenstoffatomen oder Trifluormethylgruppen substituiert sind) bedeuten,
deren Stereoisomere und deren Additionssalze.

9. Verfahren zur Herstellung der Verbindungen der Formel I nach Anspruch 1, **dadurch gekennzeichnet**, daß man ein α-halogeniertes, vorzugsweise α-bromiertes Carbonylderivat der Formel II: 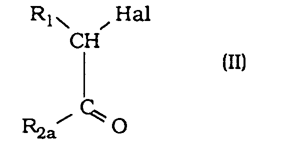 in der
- R₁ die bezüglich der Formel I in Anspruch 1 angegebenen Bedeutungen besitzt,
- R₂ₐ eine Gruppe der Formel (Aₐ): 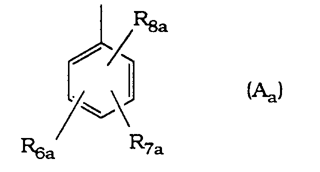 (in der R₆ₐ ein Bromatom oder ein Iodatom und R₇ₐ und R₈ₐ, die gleichartig oder verschieden sind, jeweils ein Wasserstoffatom oder ein Halogenatom darstellen), eine Gruppe der Formel (B) oder eine Gruppe der Formel (C) darstellt, wobei die Gruppen (B) und (C) die bezüglich der Formel I in Anspruch 1 angegebenen Bedeutungen besitzen und
- Hal ein Halogenatom, vorzugsweise ein Bromatom, bedeutet,
**entweder**
mit einem Thioharnstoff der Formel IIIₐ: 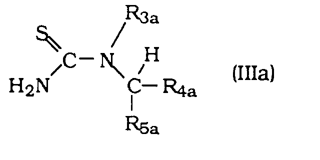 in der
- R₃ₐ eine Alkylgruppe mit 1 bis 5 Kohlenstoffatomen,
- R₄ₐ eine Cycloalkylgruppe mit 3 bis 6 Kohlenstoffatomen oder eine Gruppe der Formel (F):
- Alk - O - ProtecA (F)
(in der Alk eine Alkylgruppe mit 1 bis 5 Kohlenstoffatomen und ProtecA eine durch saure Hydrolyse abspaltbare Schutzgruppe darstellen), eine Alkoxyalkylgruppe mit 2 bis 10 Kohlenstoffatomen oder eine Alkylthioalkylgruppe mit 2 bis 10 Kohlenstoffatomen, und
- R₅ₐ eine Cycloalkylgruppe mit 3 bis 6 Kohlenstoffatomen, eine Phenylgruppe oder eine Pyridylgruppe (die gegebenenfalls durch eines oder mehrere Halogenatome, Alkoxygruppen mit 1 bis 5 Kohlenstoffatomen, Alkylgruppen mit 1 bis 5 Kohlenstoffatomen oder Trifluormethylgruppen substituiert sind), eine 3-Hydroxyalkylpyrid-6-yl-gruppe, eine 2-Hydroxyalkylpyrid-5-yl-gruppe oder eine Gruppe der Formel (D), wie sie in Anspruch 1 definiert ist, in der R₁₄ eine Hydroxyalkylgruppe mit 2 bis 5 Kohlenstoffatomen darstellt, bedeuten,
umsetzt zur Bildung der Verbindungen der Formel Iₐ: 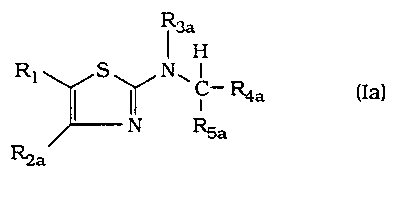 in der
- R₁ die bezüglich der Formel I in Anspruch 1 angegebenen Bedeutungen besitzt,
- R₂ₐ die bezüglich der Formel II angegebenen Bedeutungen besitzt und
- R₃ₐ, R₄ₐ und R₅ₐ die bezüglich der Formel IIIₐ angegebenen Bedeutungen besitzt,
**oder**
mit einem Thioharnstoff der Formel III_{b}: 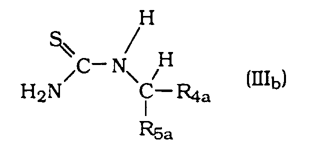 in der
- R₄ₐ und R₅ₐ die bezüglich der Formel IIIₐ angegebenen Bedeutungen besitzen, umsetzt zur Bildung der Verbindungen der Formel IV: 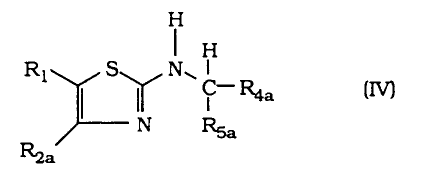 in der
- R₁ die bezüglich der Formel I in Anspruch 1 angegebenen Bedeutungen besitzt,
- R₂ₐ die bezüglich der Formel II angegebenen Bedeutungen besitzt und
- R₄ₐ und R₅ₐ die bezüglich der Formel IIIₐ angegebenen Bedeutungen besitzt, welche man mit einem Halogenid der Formel V:
Hal - R_{3b} (V)
in der
- Hal ein Halogenatom und
- R_{3b} eine Alkylgruppe mit 1 bis 5 Kohlenstoffatomen oder eine Gruppe der Formel (F) darstellen,
umsetzt zur Bildung der Verbindungen der Formel I_{b}: 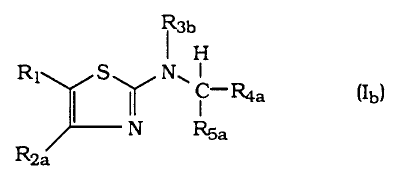 in der
- R₁ die bezüglich der Formel I in Anspruch 1 angegebenen Bedeutungen besitzt,
- R₂ₐ die bezüglich der Formel II angegebenen Bedeutungen besitzt,
- R₄ₐ und R₅ₐ die bezüglich der Formel IIIₐ angegebenen Bedeutungen besitzen
und
- R_{3b} die bezüglich der Formel V angegebenen Bedeutungen besitzt,
**und anschließend** eine der folgenden Stufen a) bis d) durchführt:
(a) man unterwirft die Verbindungen der Formeln Ia und Ib, worin R₂ₐ eine Gruppe der Formel (Aₐ) darstellt:
* entweder der Einwirkung von tert.-Butyllithium und Kohlendioxid und anschließend einer Reduktion zur Bildung der Verbindungen der Formel I nach Anspruch 1, worin R₂ eine Gruppe der Formel (A) darstellt, deren Substituent R₆ und gegebenenfalls deren Substituenten R₇ und/oder R₈ eine Hydroxymethylgruppe bedeuten,
* oder der Einwirkung von tert.-Butyllithium und eines aliphatischen (C₂-C₅)-Aldehyds zur Bildung der Verbindungen der Formel I nach Anspruch 1, in der R₂ eine Gruppe der Formel (A) darstellt, deren Substituent R₆ und gegebenenfalls deren Substituenten R₇ und/oder R₈ eine geradkettige oder verzweigte Hydroxyalkylgruppe mit 2 bis 5 Kohlenstoffatomen darstellt,
oder
(b)
* entweder
die Verbindungen der Formel Iₐ und I_{b}, in denen R₅ₐ eine 4-Bromphenylgruppe darstellt,
entweder der Einwirkung von tert.-Butyllithium und Kohlendioxid unterwirft zur Bildung der Verbindungen der Formel I nach Anspruch 1, in der R₅ eine 4-Carboxyphenylgruppe darstellt, ausgehend von denen man anschließend:
- durch Veresterung die Verbindungen der Formel I nach Anspruch 1 erhält, in der R₅ eine 4-(Alkoxycarbonyl)-phenylgruppe darstellt,
- durch Reduktion die Verbindungen der Formel I nach Anspruch 1 erhalt, in der R₅ eine 4-(Hydroxymethyl)-phenylgruppe darstellt,
- durch Reduktion und anschließende Veresterung die Verbindungen der Formel I nach Anspruch 1 erhält, in der R₅ eine 4-(Acyloxymethyl)-phenylgruppe darstellt,
- durch Reduktion und anschließende Einwirkung eines Alkylhalogenids oder eines Aralkylhalogenids die Verbindungen der Formel I nach Anspruch 1 erhält, in der R₅ eine 4-(Alkoxymethyl)-phenylgruppe oder eine 4-(Aralkoxymethyl)-phenylgruppe darstellt,
- durch Reduktion und anschließende Einwirkung von Chloroschwefelsäure die Verbindungen der Formel I nach Anspruch 1 erhält, in der R₅ eine 4-(Sulfoxymethyl)-phenylgruppe darstellt,
- durch Reduktion, der Einwirkung von 3-Chlormethylbenzoylchlorid und anschließend von N-Methylpiperazin die Verbindungen der Formel I nach Anspruch 1 erhält, in der R₅ eine Gruppe der Formel (D) und R₁₄ eine Gruppe der Formel (E) darstellen,
- durch Reduktion und dann durch Einwirkung eines Halogens die Verbindungen der Formel I nach Anspruch 1 erhält, in der R₅ eine 4-(Halogenomethyl)-phenylgruppe darstellt,
oder
der Einwirkung von tert.-Butyllithium und der Einwirkung
- eines aliphatischen Aldehyds unterwirft zur Bildung der Verbindungen der Formel I nach Anspruch 1, in der R₅ eine 4-(sec-Hydroxyalkyl)-phenylgruppe darstellt, oder
- eines aliphatischen Ketons unterwirft zur Bildung der Verbindungen der Formel I nach Anspruch 1, in der R₅ eine 4-(tert.-Hydroxyalkyl)-phenylgruppe darstellt,
* oder
man unterwirft die Verbindungen der Formel Iₐ und I_{b}, in denen R₅ₐ eine Gruppe der Formel (D) darstellt, in der R₁₄ eine Hydroxyalkylgruppe mit 2 bis 5 Kohlenstoffatomen bedeutet,
- einer Veresterung zur Bildung der Verbindungen der Formel I nach Anspruch 1, in der R₅ eine 4-(Acyloxyalkyl)-phenylgruppe darstellt,
- einer Alkylierung zur Bildung der Verbindungen der Formel I nach Anspruch 1, in der R₅ eine 4-(Alkoxyalkyl)-phenylgruppe oder eine 4-(Aralkoxyalkyl)-phenylgruppe darstellt,
- der Einwirkung von Chloroschwefelsäure zur Bildung der Verbindungen der Formel I nach Anspruch 1, in der R₅ eine 4-(Sulfoxyalkyl)-phenylgruppe darstellt,
- der Einwirkung eines Halogens zur Bildung der Verbindung der Formel I nach Anspruch 1, in der R₅ eine 4-(Halogenoalkyl)-phenylgruppe darstellt,
(c) oder
man unterwirft die Verbindungen der Formel Iₐ und I_{b}, in denen R₄ₐ eine Gruppe der Formel (F) darstellt, einer Behandlung zur Abspaltung der Schutzgruppe zur Bildung der Verbindungen der Formel I nach Anspruch 1, in der R₄ eine Hydroxyalkylgruppe darstellt, ausgehend von denen man anschließend:
- durch Veresterung die Verbindungen der Formel I nach Anspruch 1 erhält, in der R₄ eine Acyloxyalkylgruppe darstellt,
- durch Alkylierung die Verbindungen der Formel I nach Anspruch 1 erhält, in der R₄ eine Alkoxyalkylgruppe oder eine Cycloalkyloxyalkylgruppe darstellt,
- durch Alkylierung mit aliphatischen geschützten halogenierten Alkoholen und dann durch Abspaltung der Schutzgruppen die Verbindungen der Formel I nach Anspruch 1 erhält, in der R₄ eine Hydroxyalkyloxyalkylgruppe darstellt,
- durch Alkylierung mit geschützten halogenierten aliphatischen Alkoholen und dann durch Abspaltung der Schutzgruppen und anschließende Alkylierung die Verbindungen der Formel I nach Anspruch 1 erhält, in der R₄ eine Alkoxyalkyloxyalkylgruppe darstellt,
(d) oder
man unterwirft die Produkte der Formel I_{b}, in der R_{3b} eine Gruppe der Formel (F) darstellt, einer Behandlung zur Abspaltung der Schutzgruppe durch saure Hydrolyse zur Bildung der Verbindungen der Formel I nach Anspruch 1, in der R₃ eine Hydroxyalkylgruppe darstellt, ausgehend von welchen Verbindungen man anschließend durch Alkylierung oder durch Veresterung die Verbindungen der Formel I nach Anspruch 1 erhält, in der R₃ eine Alkoxyalkylgruppe bzw. eine Acyloxyalkylgruppe bedeutet,
**und gegebenenfalls die Verbindungen der Formel I nach Anspruch 1**
anschließend in ihre möglichen Stereoisomeren aufspaltet und/oder zur Bildung der entsprechenden Salze in ihre Salze überführt.

10. Verwendung der Verbindungen der Formel I nach irgendeinem der Ansprüche 1 bis 8 zur Herstellung eines Arzneimittels zur Behandlung von Krankheiten, die eine Modulierung der Wirkung des Faktors der Freisetzung des corticotropen Hormons (CRF) erforderlich machen.

11. Pharmazeutische Zubereitung enthaltend als Wirkstoff mindestens eine Verbindung nach irgendeinem der Ansprüche 1 bis 8 in Form der Base oder in Form des Salzes mit einer anorganischen oder organischen, pharmazeutisch annehmbaren Säure in Kombination oder in Mischung mit einem inerten, nichttoxischen, pharmazeutisch annehmbaren Trägermaterial oder Bindemittel.
